(19) Europäisches Patentamt
European Patent Office
Office européen des brevets

(11) Publication number: **0 293 532**
**A1**

(12) **EUROPEAN PATENT APPLICATION**

(21) Application number: **87309151.6**

(22) Date of filing: **15.10.87**

(51) Int. Cl.4: **C07D 499/68 , C07D 501/36 , A61K 31/43 , A61K 31/545 , //C07D241/08,C07D401/06**

The title of the invention has been amended (Guidelines for Examination in the EPO, A-III, 7.3).

(30) Priority: **04.06.87 GB 8713061**

(43) Date of publication of application: **07.12.88 Bulletin 88/49**

(84) Designated Contracting States: **BE CH DE FR GB IT LI NL**



(71) Applicant: **BEECHAM GROUP PLC**
**Beecham House Great West Road**
**Brentford Middlesex TW8 9BD(GB)**

(72) Inventor: **Southgate, Robert Beecham Pharmaceuticals**
**Brockham Park Betchworth**
**Surrey RH3 7AJ(GB)**
Inventor: **Harrington, Frank Peter Beecham Pharmaceuticals**
**Brockham Park Betchworth**
**Surrey RH3 7AJ(GB)**
Inventor: **O'Hanlon, Peter John Beecham Pharmaceuticals**
**Great Burgh Yew Tree Bottom Road Epsom**
**Surrey KT18 5XQ(GB)**

(74) Representative: **Lockwood, Barbara Ann et al**
**Beecham Pharmaceuticals Biosciences Research Centre Great Burgh Yew Tree Bottom Road**
**Epsom Surrey KT18 5XQ(GB)**


(54) **6- or 7-beta-[2-[4-(substituted)-2,3-dioxopiperazin-1-yl) carbonylamino]-(substituted)acetamido]-penicillin and cephalosporin derivatives.**


(57) Antibacterial agents have the formula (I) or are pharmaceutically acceptable salts or in vivo hydrolysable esters thereof:


EP 0 293 532 A1

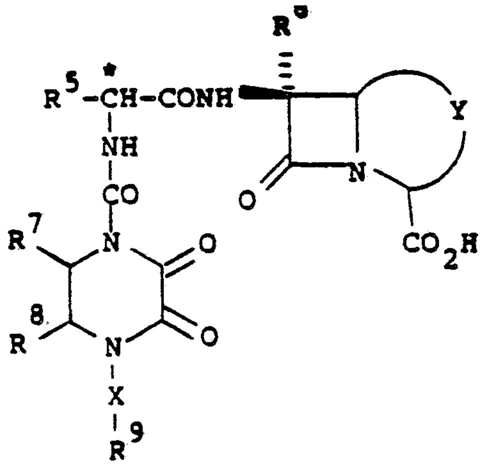

(I)

in which Y is

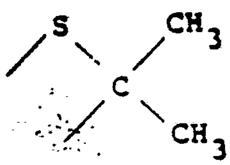

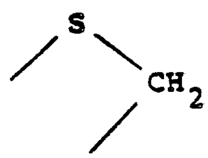

or

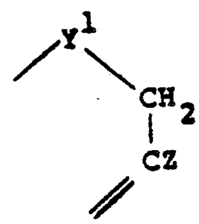

in which $Y^1$ is oxygen, sulphur or $-CH_2-$ and Z represents hydrogen, halogen, $C_{1-4}$ alkoxy, $-CH_2-Q$ or $-CH=CH-Q$ where Q represents hydrogen, halogen, hydroxy, mercapto, cyano, carboxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen;
$R^5$ represents phenyl, substituted phenyl, cyclohexenyl, cyclohexadienyl or an optionally substituted 5 or 6-membered heterocyclic ring containing up to three hetero atoms selected from oxygen, sulphur or nitrogen, $R^6$ is hydrogen, hydroxymethyl, formamido, or methoxy, $R^7$ and $R^8$ are the same or different and represent hydrogen, $C_{1-6}$ alkyl, substituted $C_{1-6}$ alkyl, halogen, amino, phenyl, substituted phenyl, hydroxy or $C_{1-6}$ alkoxy or $R^7$ and $R^8$ form the residue of an optionally substituted 5 or 6- membered carbocyclic ring or a 5 or 6-membered heterocyclic ring containing up to three hetero atoms selected from oxygen, sulphur or nitrogen, $R^9$ is

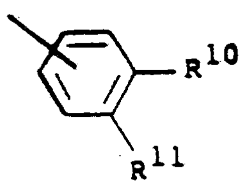

or

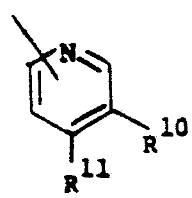

wherein $R^{10}$ and $R^{11}$ are the same or different and each represents hydroxy, or protected hydroxy and $XR^9$ is $-(CH_2)_nR^9$, $-NHCOR^9$, $-N=CHR^9$, $-NHCH_2R^9$, or $-COR^9$, where n is from 0 to 2; with the proviso that $XR^9$ does not represent $-N=CHR^9$ when $R^6$ represents hydrogen.
The use of the compounds is described together with intermediates for their preparation.

## NOVEL COMPOUNDS

This invention relates to a class of novel β-lactam compounds which have antibacterial activity and are of value in the treatment of infections in animals especially mammals, including man, caused by a wide range of micro-organisms, particularly Gram-negative organisms. The invention also relates to a process for the preparation of such compounds, intermediates for use in the preparation of the compounds and to pharmaceutical compositions containing the antibacterially active compounds.

UK Patent GB-2107307B discloses and claims β-lactam antibiotics which have an α-formamido substituent on the carbon atom adjacent to the carbonyl group of the β-lactam ring, and in particular compounds of formula (A)

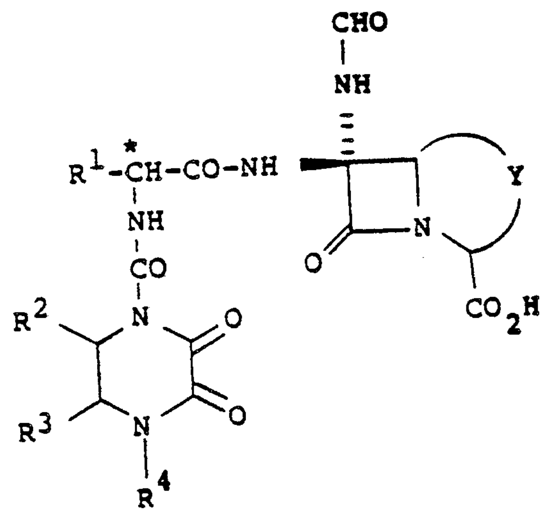

(A)

wherein Y is

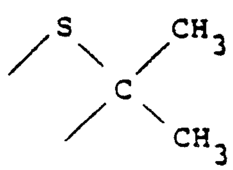

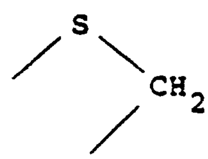

or

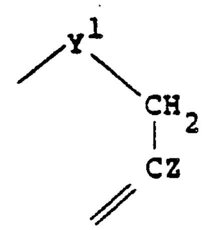

in which $Y^1$ is oxygen, sulphur or $-CH_2-$ and Z represents hydrogen, halogen or an organic group such as $C_{1-4}$ alkoxy, $-CH_2-Q$ or $-CH=CH-Q$ where Q represents hydrogen, halogen, hydroxy, mercapto, cyano, carboxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen;

$R^1$ is phenyl, substituted phenyl, cyclohexenyl, cyclohexadienyl, or a 5- or 6-membered heterocyclic ring containing up to 3 hetero-atoms selected from oxygen, sulphur or nitrogen, optionally substituted with hydroxy, amino, halogen, substituted amino or a $C_{1-6}$ alkoxy; $R^2$ and $R^3$ are the same or different and represent hydrogen, $C_{1-6}$ alkyl, substituted $C_{1-6}$ alkyl, halogen, amino, hydroxy or $C_{1-6}$ alkoxy or $R^2$ and $R^3$ together form the residue of a 5- or 6-membered heterocyclic ring: and $R^4$ represents hydrogen, $C_{1-6}$ alkyl, substituted alkyl, aryl or aralkyl: or a pharmaceutically acceptable salt or in vivo hydrolysable ester-thereof.

The present invention provides a compound of formula (I) or a pharmaceutically acceptable salt or in vivo hydrolysable ester thereof:

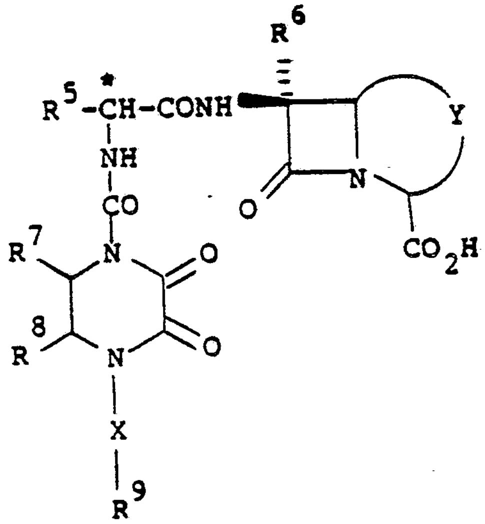

(I)

wherein Y is

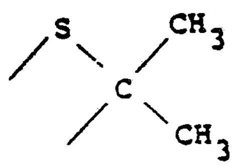

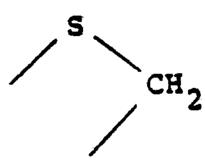

or

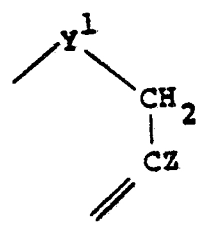

in which $Y^1$ is oxygen, sulphur or $-CH_2-$ and Z represents hydrogen, halogen, $C_{1-4}$ alkoxy, $-CH_2-Q$ or $-CH=CH-Q$ where Q represents hydrogen, halogen, hydroxy, mercapto, cyano, carboxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen;
$R^5$ represents phenyl, substituted phenyl, cyclohexenyl, cyclohexadienyl or a 5 or 6-membered heterocyclic ring containing up to three hetero atoms selected from oxygen, sulphur or nitrogen optionally substituted with hydroxy, amino, substituted amino, halogen or $C_{1-6}$ alkoxy, $R^6$ is hydrogen, hydroxymethyl, formamido, or methoxy, $R^7$ and $R^8$ are the same or different and represent hydrogen, $C_{1-6}$ alkyl, substituted $C_{1-6}$ alkyl, halogen, amino, phenyl, substituted phenyl, hydroxy or $C_{1-6}$ alkoxy or $R^7$ and $R^8$ form the residue of a 5 or 6-membered carbocyclic ring or a 5 or 6-membered heterocyclic ring containing up to three hetero atoms selected from oxygen, sulphur or nitrogen optionally substituted with hydroxy, amino, substituted amino, halogen or $C_{1-6}$ alkoxy; $R^9$ is

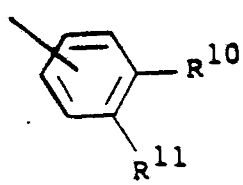

or

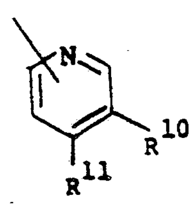

wherein $R^{10}$ and $R^{11}$ are the same or different and each represents hydroxy, or protected hydroxy and $XR^9$ is $-(CH_2)_nR^9$, $-NHCOR^9$, $-N=CHR^9$, $-NHCH_2R^9$, or $-COR^9$, where n is from 0 to 2; with the proviso that $XR^9$ does not represent $-N=CHR^9$ when $R^6$ represents hydrogen. Preferably $XR^9$ does not represent $-N=CHR^9$ when $R^6$ represents hydrogen or methoxy.

Suitable protected hydroxy groups include $C_{1-6}$ alkylcarbonyloxy such as acetoxy, $C_{1-6}$ alkoxycarbonyloxy such as t-butoxycarbonyloxy, substituted $C_{1-6}$ alkoxycarbonyloxy wherein the alkyl is substituted by up to three halogen groups, such as trichloroethoxycarbonyloxy, aralkyloxycarbonyloxy, substituted

aralkyloxycarbonyloxy such as p-nitrobenzyloxycarbonyloxy, tri $C_{1-6}$ alkylsilyloxy such as trimethylsilyloxy, or optionally substituted aralkyloxy such as benzyloxy.

When used herein the term 'aryl' includes phenyl and naphthyl optionally substituted with up to five, preferably up to three, groups selected from halogen, $C_{1-6}$ alkyl, phenyl, $C_{1-6}$ alkoxy, halo($C_{1-6}$) alkyl, hydroxy, amino, nitro, carboxy, $C_{1-6}$ alkoxycarbonyl, $C_{1-6}$ alkoxycarbonyl-($C_{1-6}$)-alkyl $C_{1-6}$ alkylcarbonyloxy, or $C_{1-6}$ alkylcarbonyl groups.

When used herein the term 'alkyl' includes straight and branched chain alkyl groups containing from 1 to 6 carbon atoms, such as methyl, ethyl, propyl and butyl. A particular alkyl group is methyl.

The term 'halogen' refers to fluorine, chlorine, bromine and iodine.

Suitable 'substituted amino' groups include $C_{1-6}$ alkylamino and di($C_{1-6}$)alkylamino.

Suitably the substituted phenyl group for $R^5$, $R^7$ or $R^8$ is a phenyl group substituted with up to three groups selected from $C_{1-6}$ alkyl, phenyl, halogen, $C_{1-6}$ alkoxy, amino, nitro, hydroxy, $C_{1-6}$ alkylamido, $C_{1-6}$ alkylcarbonyloxy, carboxy, $C_{1-6}$ alkoxycarbonyl, halo($C_{1-6}$)alkyl, oxo($C_{1-6}$)alkyl, $C_{1-6}$ alkylcarbonyl, aryloxy, aralkyloxy, arylcarbonyl, $C_{1-6}$ alkylamino di($C_{1-6}$)alkylamino or protected amino. Suitable amino protecting groups are those well known in the art which may be removed under conventional conditions without disruption of the remainder of the molecule.

Suitable amino protecting groups include benzyl optionally substituted in the phenyl ring by one or two substituents selected from $C_{1-4}$ alkyl, $C_{1-4}$ alkoxy, trifluoromethyl, halogen or nitro; $C_{1-4}$ alkoxycarbonyl, for example tert-butoxycarbonyl; halogen substituted $C_{1-4}$ alkoxycarbonyl, for example trichloroethoxycarbonyl, benzyloxycarbonyl optionally substituted as for benzyl above; allyloxycarbonyl; trityl; or enamines derived from reaction, with $\beta$-keto carboxylic acid esters for example with ethyl acetoacetate.

When $R^5$ represents a 5- or 6-membered heterocyclic ring, the ring is preferably heteroaromatic.

Suitable examples of a heteroaromatic group for $R^5$ include 2-thienyl, 3-thienyl, 2-furyl, 3-furyl and 2-amino-4-thiazolyl.

In formula (I) the group $R^5$ is preferably phenyl, 2-thienyl or 4-hydroxyphenyl.

The group $R^6$ is preferably formamido, methoxy or hydroxymethyl, most preferably formamido.

Suitable $C_{1-6}$ alkyl groups for the groups $R^7$ and $R^8$ include methyl, ethyl, n-and iso-propyl and n-, sec-, iso- and tert-butyl. Preferably $R^7$ and $R^8$ are hydrogen.

The bond joining the group $R^9$ to the group X may be attached to any one of the carbon atoms in the group $R^9$ which is not attached to $R^{10}$ or $R^{11}$.

Preferably the group $R^9$ may be represented by

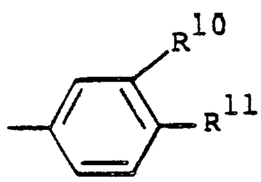 , 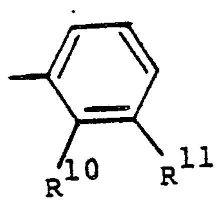 or 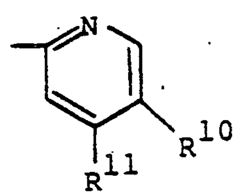

Suitable values for the groups $R^{10}$ and $R^{11}$ include hydroxy and acetoxy.

Particular values of the group $R^9$ include 3,4-dihydroxyphenyl, 3,4-diacetoxyphenyl, 2,3-dihydroxyphenyl and 4,5-dihydroxypyridin-2-yl.

Preferred values of the group $R^9$ include 3,4-dihydroxyphenyl and 4,5-dihydroxypyridin-2-yl.

It will be understood that the group $R^9$ may exist in certain instances in one or more tautomeric forms and that such tautomeric forms are included within the scope of this invention. For example, when $R^9$ is 4,5-dihydroxypyridin-2-yl, that group may also be represented by the 1,4-dihydro-5-hydroxy-4-oxopyridin-2-yl group of the formula:

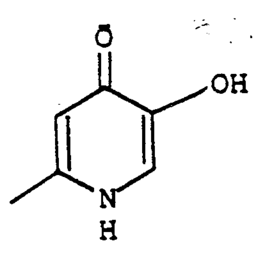

Preferably X is $(-CH_2-)_n-$. More preferably n=1 so that X is a methylene group.

Preferred values for Y are

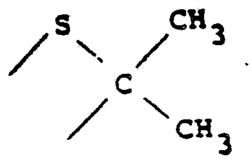

and

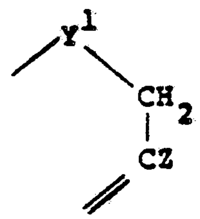

wherein $Y^1$ is sulphur.

Suitably Z is $C_{1-6}$ alkylcarbonyloxymethyl, preferably acetoxymethyl, or is a heterocyclylthiomethyl group of the formula $-CH_2-S-Het$ wherein 'Het' is a five or six membered heterocyclic ring containing from 1 to 4 atoms selected from N, O, and S unsubstituted or substituted with one or two groups selected from $C_{1-6}$ alkyl, $C_{1-6}$ alkoxy, hydroxy $C_{1-6}$ alkyl, $C_{2-6}$ alkenyl, $C_{1-6}$ alkoxy $C_{1-6}$ alkyl, carboxy $C_{1-6}$ alkyl, sulphonyl $C_{1-6}$ alkyl, carbamoyl $C_{1-6}$ alkyl, trifluoromethyl, hydroxy, halogen, oxo, amino $C_{1-6}$ alkyl, $C_{1-6}$ alkylamino $C_{1-6}$ alkyl, di ($C_{1-6}$ alkyl) amino $C_{1-6}$ alkyl, and carboxy $C_{1-6}$ alkyl or two substituents may be linked to form the residue of a heterocyclic or carbocyclic ring.

Examples of the group 'Het' include unsubstituted and substituted imidazolyl, triazolyl, tetrazolyl, thiazolyl, thiadiazolyl, thiatriazolyl, oxazolyl, triazinyl and oxadiazolyl.

Suitable groups 'Het' include unsubstituted and substituted 1,2,3-triazolyl; 1,2,4-triazolyl; tetrazolyl; oxazolyl; thiazolyl; 1,3,4-oxadiazolyl; 1, 3,4-thiadiazolyl, or 1,2,4-thiadiazolyl.

Preferably the heterocyclylthio group is 1-methyl -1H-tetrazol-5-ylthio, or 1,3,4-thiadiazol-2-ylthio.

The nitrogen containing heterocyclic group bonded via nitrogen is suitably a pyridinium group unsubstituted or substituted with one or two groups selected from $C_{1-6}$ alkyl, $C_{1-6}$ alkoxy, hydroxy $C_{1-6}$ alkyl, $C_{2-6}$ alkenyl, $C_{1-6}$ alkoxy $C_{1-6}$ alkyl, carboxy $C_{1-6}$ alkyl, sulphonyl $C_{1-6}$ alkyl, carbamoylmethyl, carbamoyl, trifluoromethyl, hydroxy, halogen, oxo, and amino $C_{1-6}$ alkyl.

The carbon atom marked * in formulae herein is asymmetric and thus compounds of formula (I) may exist as two optically active diastereoisomers. In general the isomer prepared from the D-side chain exhibits the highest antibacterial activity and accordingly the D compound or the DL mixtures are preferred, with the D compound being particularly preferred.

The compounds of formula (I) with the preferred D- side -chain can be separated from a mixture of both diastereoisomers by conventional methods, or be prepared from intermediates that bear a D- side chain.

Moreover where $R^6$ represents the formamido group, this can exist in two preferred conformations, those wherein the hydrogen atoms of the -NH-CHO are, cis- or trans-, of which the cis conformation normally predominates.

Since the $\beta$-lactam antibiotic compounds of the present invention are intended for use in pharmaceutical compositions it will readily be understood that they are each provided in substantially pure form, for example at least 50% pure, more suitably at least 75% pure and preferably at least 95% pure (% are on a wt/wt basis). Impure preparations of the compounds may be used for preparing the more pure forms used in the pharmaceutical compositions. Although the purity of intermediate compounds of the present invention is less critical it will readily be understood that the substantially pure form is preferred as for the $\beta$-lactam antibiotic compounds. Preferably, whenever possible, the compounds of the present invention are obtained in crystalline form.

Examples of suitable pharmaceutically acceptable in vivo hydrolysable ester groups include those which break down readily in the human body to leave the parent acid or its salt. Suitable ester groups of this type include those of part formula (i), (ii), (iii) and (iv):

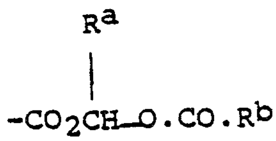

(i)

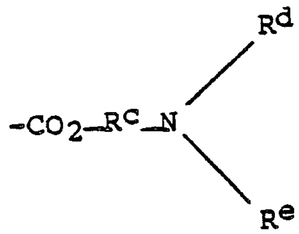

(ii)

$-CO_2CH_2-OR^f$ (iii)

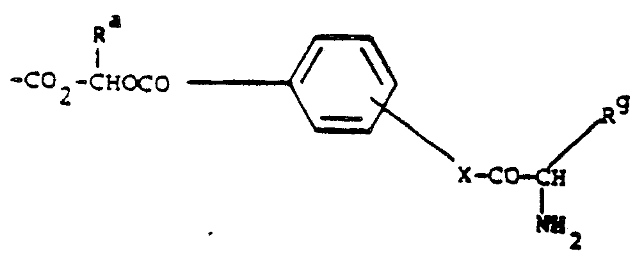

(iv)

wherein $R^a$ is hydrogen, $C_{1-6}$ alkyl, $C_{3-7}$ cycloalkyl, methyl, or phenyl, $R^b$ is $C_{1-6}$ alkyl, $C_{1-6}$ alkoxy, phenyl, benzyl, $C_{3-7}$ cycloalkyl, $C_{1-6}$ alkyl $C_{3-7}$ cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$ independently represent $C_{1-6}$ alkyl; $R^f$ represents $C_{1-6}$ alkyl; $R^g$ represents hydrogen or phenyl optionally substituted by up to three groups selected from halogen, $C_{1-6}$ alkyl, or $C_{1-6}$ alkoxy; and X is oxygen or NH.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1-yl, and (1-aminoethyl)carbonyloxymethyl; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

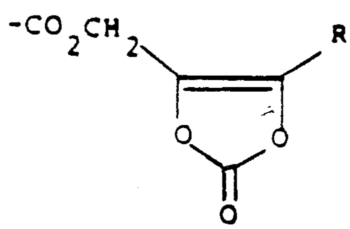

wherein R is hydrogen, $C_{1-6}$ alkyl or phenyl.

Suitable pharmaceutically acceptable salts of the carboxy group of the compound of formula (I) include

metal salts, eg aluminium, alkali metal salts such as sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine, cycloalkylamines such as dicyclohexylamine, or with procaine, dibenzylamine, N,N-dibenzylethylenediamine, 1-ephenamine, N-ethylpiperidine, N-benzyl-β-phenethylamine, dehydroabietylamine, N,N′-bisdehydro-abietylamine, ethylenediamine, or bases of the pyridine type such as pyridine, collidine or quinoline, or other amines which have been used to form salts with known penicillins and cephalosporins. Other useful salts include the lithium and silver salt.

Some of the compounds of this invention may be crystallised or recrystallised from solvents containing water. In such cases water of hydration may be formed. This invention includes within its scope stoichiometric hydrates as well as compounds containing variable amounts of water that may be produced by processes such as lyophilisation.

Specific compounds within this invention include the following and pharmaceutically acceptable salts and in vivo hydrolysable esters thereof:-

6β-[D-2[[4-(3,4-Dihydroxybenzyl)-2,3-dioxopiperazin-1-yl]carbonylamino]-2-phenylacetamido]penicillanic acid;

6β-[D-2[[4-(3,4-Dihydroxybenzyl)-2,3-dioxopiperazin-1-yl]carbonylamino]-2-phenylacetamido]-6α-formamidopenicillanic acid;

6β-[D-2-[[4-(3,4-Dihydroxybenzyl)-2,3-dioxopiperazin-1-yl]carbonylamino]-2-phenylacetamido]-6α-methoxypenicillanic acid;

6β-[D-2-[[4-(3,4-Dihydroxybenzyl)-2,3-dioxopiperazin-1-yl]carbonylamino]-2-(4-hydroxyphenyl)acetamido]-6α-formamidopenicillanic acid;

6β-[D-2-(3,4-Diacetoxyphenyl)-2-[[4-(3,4-dihydroxybenzyl)-2,3-dioxopiperazin-1-yl]carbonylamino] acetamido]-6α-formamidopenicillanic acid;

6β-[D-2-[[4-(4,5-Dihydroxypyridin-2-ylmethyl)2,3-dioxopiperazin-1-yl]carbonylamino]-2-phenylacetamido]-6α-formamidopenicillanic acid;

6β-[D-2-[[4-(3,4-dihydroxybenzyl)-2,3-dioxopiperazin-1-yl]carbonylamino]-2-phenylacetamido]-6α-(hydroxymethyl) penicillanic acid;

6β-[D-2-[[4-(3,4-Dihydroxybenzyl)-2,3-dioxopiperazin-1- yl]carbonylamino]-2-[(4-aminophenyl)acetamido]-6α-formamidopenicillanic acid;

7β-[D-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;

7β-[D-2-[[4-(3,4-Diacetoxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylic acid;

7β-[D-2-[[4-(3,4-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;

6β-[D-2-(3,4-Diacetoxyphenyl)-2-[(4-(3,4-dihydroxybenzyl)-2,3-dioxopiperazin-1-yl)carbonylamino] acetamido]-6α-(hydroxymethylpenicillanic acid;

7β-[2-[[4-(3,4-Dihydroxybenzyl)-2,3-dioxopiperazin-1-yl]carbonylamino]-2-(2-thienyl)acetamido]-7α-formamido-3-[(1,3,4-thiadiazol-2-yl)thiomethyl]ceph3-em-4-carboxylic acid;

7β-[D-2-[[4-(3,4-Dihydroxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylic acid;

6β-[2-[[4-(2,3-dihydroxybenzyl)-2,3-dioxopiperazin-1-yl]carbonylamino]-2-phenylacetamido]-6α-formamidopenicillanic acid;

7β-[D-2-[[4-(2,3-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;

6β-[D-2-[4-(3,4-Dihydroxyphenyl)-2,3-dioxopiperazin-1-ylcarbonylamino]-2-phenylacetamido]-6α-formamidopenicillanic acid;

6β-[D-2-[4-[2-(3,4-Dihydroxyphenyl)ethyl]-2,3-dioxopiperazin-1-ylcarbonylamino]-2-phenylacetamido]-6α-formamidopenicillanic acid;

7β-[DL-2-[4-(3,4-Dihydroxyphenyl)-2,3-dioxopiperazin-1-ylcarbonylamino]-2-phenylacetamido]-7α-formamido-3-[(1,3,4-thiadiazol-2-yl)thiomethyl]ceph-3-em-4-carboxylic acid;

3-Acetoxymethyl-7β-[D-2-[4-(3,4-dihydroxybenzyl)-2,3-dioxopiperazin-1-ylcarbonylamino]-2-phenylacetamido]ceph-3-em-4-carboxylic acid;

3-Acetoxymethyl-7β-[D-2-[4-(1,4-dihydro-5-hydroxy-4-oxopyridin-2-yl)methyl-2,3-dioxopiperazin-1-ylcarbonylamino]-2-phenylacetamido]ceph-3-em-4-carboxylic acid; and

[6R,7R] 3-(1,3,4-Thiadiazol-2-yl)thiomethyl-7[[R]-2[2,3-dioxo-4(3,4-dihydroxybenzylideneamino)piper azin-1-yl-carbonylamino]-2-phenylacetamido]-7-formamido-ceph-3-em-4-carboxylic acid.

The antibiotic compounds according to the invention may be formulated for administration in any convenient way for use in human or veterinary medicine, according to techniques and procedures _per se_ known in the art with reference to other antibiotics, and the invention therefore includes within its scope a pharmaceutical composition comprising an antibiotic compound according to the present invention such as, for example a compound of formula (I) above together with a pharmaceutically acceptable carrier or excipient. The compositions may be formulated for administration by any suitable route, such as oral or parenteral, or by topical application. The compositions may be in the form of tablets, capsules, powders, granules, lozenges, creams or liquid preparations, such as oral or sterile parenteral solutions or suspensions.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine, tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, for example sorbitol, methyl cellulose, glucose syrup, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl _p_-hydroxybenzoate or sorbic acid, and if desired conventional flavouring or colouring agents.

Suppositories will contain conventional suppository base, eg cocoa-butter or other glyceride.

For parenteral administration, fluid unit dosage forms are prepared utilising the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing. Advantageously, agents such as local anaesthetic, preservative and buffering agents can be dissolved in the vehicle. To enhance the stability, the composition can be frozen after filling into the vial and the water removed under vacuum. The dry lyophilised powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The composition may contain from 0.1% to 99.5% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 mg to 12 g per day for an average adult patient (70 kg), for instance 1500 mg per day depending on the route and frequency of administration. Such dosages correspond to 1.5 to 170 mg/kg per day. Suitably the dosage is from 1 to 6g per day. The daily dosage is normally given by administering the compound 1-4 times daily.

The antibiotic compounds according to the present invention may be the sole therapeutic agent in the compositions of the invention or a combination with other antibiotics and/or β-lactamase inhibitor may be employed.

Advantageously the compositions also comprise a compound of formula (B) or a pharmaceutically acceptable salt or ester thereof:

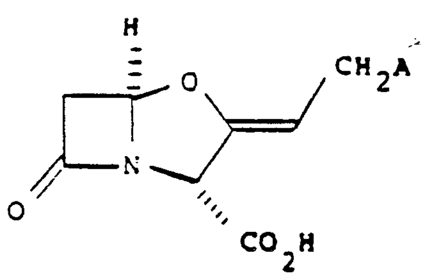

(B)

wherein A is hydroxyl; substituted hydroxyl; thiol; a group of formula $SO_2R^j$ wherein $R^j$ is $C_{1-6}$ alkyl; substituted thiol; amino; mono- or di-hydrocarbyl substituted amino; mono- or di-acylamino; an optionally substituted triazolyl group; or an optionally substituted tetrazolyl group as described in EP O 053 893.

A further advantageous composition comprises an antibiotic compound according to the invention and a pharmaceutically acceptable carrier or excipient together with a β-lactamase inhibitor of formula (C) or a pharmaceutically acceptable salt or in vivo hydrolysable ester thereof:

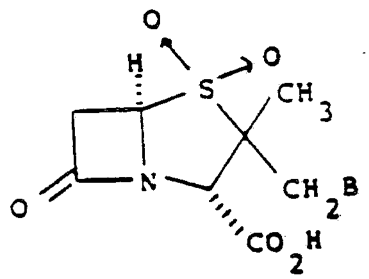
(C)

wherein B is hydrogen, halogen or a group of formula:

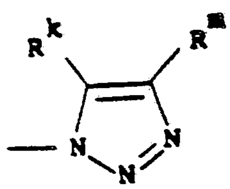

in which $R^k$ and $R^m$ are the same or different and each is hydrogen, $C_{1-6}$ alkoxycarbonyl, or carboxy or a pharmaceutically acceptable salt thereof.

Further suitable β-lactamase inhibitors include 6-alkylidene penems as described in European Patent Application No. 81301683.9 (Publication Number 0 041 768).

Further suitable β-lactamase inhibitors include 6β-bromopenicillanic acid and salts and in vivo hydrolysable esters thereof and 6β-iodopenicillanic acid and salts and in vivo hydrolysable esters thereof.

Such compositions of this invention comprising a β-lactamase inhibitor are formulated in conventional manner.

The present invention also includes a method of treating bacterial infections in humans and animals which comprises the administration of a therapeutically effective amount of an antibiotic compound of this invention.

The antibiotic compounds of the present invention are active against a broad range of Gram positive and Gram negative bacteria, for example they are useful for treatment of respiratory tract and urinary tract infections in humans and are particularly useful in the treatment of bacterial infections in immunocompromised patients. A particular advantage of the antibacterially active compounds of the invention in which $R^6$ is formamido, is their stability to β-lactamase enzymes and they are therefore effective against β-lactamase producing organisms.

The present invention further provides a process for the preparation of a compound of formula (I) which process comprises treating a compound of formula (II):

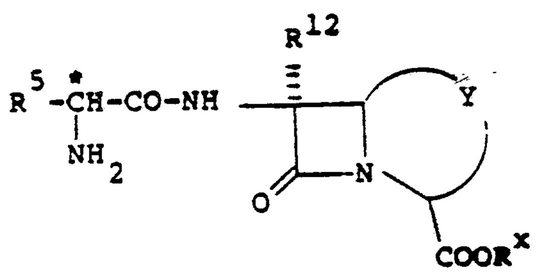
(II)

wherein Y, $R^5$ and * are as defined for formula (I), $R^{12}$ is hydrogen, -NHCHO, $CH_2OH$, -OMe or a suitable intermediate group known for the production of -NHCHO or -OMe, and wherein any reactive groups may be protected and $R^x$ represents hydrogen or a readily removable carboxyl protecting group, with an N-acylating derivative of an acid of formula (III):

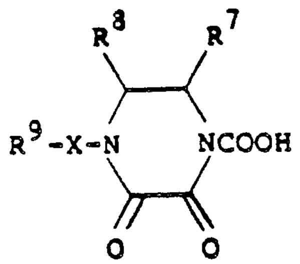

(III)

wherein $R^7$, $R^8$, $R^9$ and X are as defined for formula (I), and wherein any reactive groups may be protected; and thereafter if necessary carrying out one or more of the following steps:

(i) converting an intermediate group $R^{12}$ to -NHCHO or OMe ;

(ii) removing any carboxyl protecting group $R^x$,

(iii) removing any protecting group on $R^9$,

(iv) removing any protecting group on $R^5$,

(v) converting the product into a salt or in vivo hydrolysable ester.

It will be understood that OMe is an abbreviation for methoxy.

Preferably the intermediate group $R^{12}$ known for the production of -NHCHO or -OMe is an amino group or $-SR^{13}$ group wherein $R^{13}$ is $C_{1-6}$ alkyl, aryl or benzyl. A preferred $R^{12}$ group for use in the present process is $-SCH_3$. Processes suitable for preparing compounds of formula (II) wherein $R^{12}$ is $-NH_2$ are disclosed in U.S. Patent No. 3962214 and U.K. Patent No. 1348984. Processes suitable for preparing compounds of formula (II) wherein $R^{12}$ is $-SR^{13}$ are well known in the art, and were first disclosed in Tetrahedron Lett.1973, 273. Processes suitable for preparing compounds of formula (II) wherein $R^{12}$ is $CH_2OH$ are analogous to processes disclosed in J. Antibiotics, 37, 1729-1731, December 1984 (Dixon, Edmondson, Hardy and Milner).

Suitable readily removable carboxyl protecting groups for the group $R^x$ include salt and ester derivatives of the carboxylic acid. The derivative is preferably one which may readily be cleaved.

Suitable ester-forming carboxyl-protecting groups are those which may be removed under conventional conditions. Such groups for $R^x$ include benzyl, p-methoxybenzyl, benzoylmethyl, p-nitrobenzyl, 4-pyridylmethyl, t-butyl, t-amyl, allyl, diphenylmethyl, triphenylmethyl, adamantly, 2-benzyloxyphenyl, 4-methylthiophenyl, tetrahydrofuran-2-yl, tetrahydropyran-2-yl, pentachlorophenyl, acetonyl, toluenesulphonylethyl, methoxymethyl, a silyl, stannyl or phosphorous- containing group or an in vivo hydrolysable ester radical such as defined above.

A carboxyl group may be regenerated from any of the above esters by usual methods appropriate to the particular $R^x$ group, for example, acid- and base-catalysed hydrolysis, or by enzymically-catalysed hydrolysis, or by hydrogenolysis.

Compounds of formula (III) may be prepared by treating a compound of formula (IV):

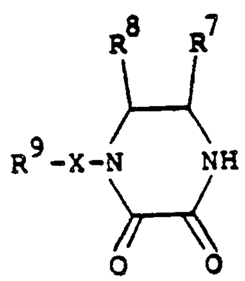

(IV)

wherein X, $R^7$, $R^8$ and $R^9$ are as defined for formula (I) and any reactive groups may be protected with a compound of formula (V):

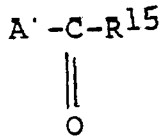

(V)

wherein A′ represents a halogen atom and $R^{15}$ represents a halogen atom or an ester residue, and then if desired converting

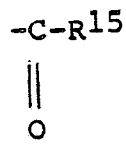

to a different reactive derivative in the carboxyl group by a conventional method.

Suitable groups which permit acylation to take place and which are optionally present on the amino group of the starting material of the formula (II) include N-silyl, N-stannyl and N-phosphorus groups, for example trialkylsilyl groups such as trimethylsilyl, trialkyltin groups such as tri-n-butyltin, groups of formula $-P.R^pR^q$ wherein $R^p$ is an alkyl, haloalkyl, aryl, aralkyl, alkoxy, haloalkyl, aryl, aralkyl, alkoxy, haloalkoxy, aryloxy, aralkyloxy or dialkylamino group, $R^q$ is the same as $R^p$ or is halogen or $R^p$ and $R^q$ together form a ring; suitable such phosphorus groups being $-P(OC_2H_5)_2$, $-P(C_2H_5)_2$,

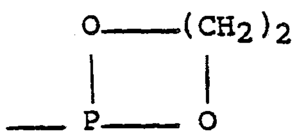

and

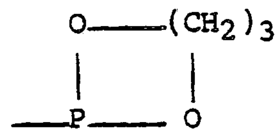

Compounds of formula (IV) in which X is $-CH_2-$ may be prepared by treating the compound of formula (VI):

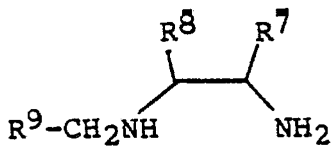

(VI)

wherein $R^7$, $R^8$ and $R^9$ are as defined in respect of formula (I) with an ester or activated derivative of oxalic acid of formula:

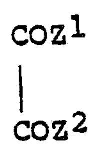

wherein $Z^1$ and $Z^2$ are the same or different ester or activating group. Suitable derivatives of oxalic acid include diethyl oxalate .

Suitably this reaction is carried out under reflux in a solvent such as ethanol.

Compounds of formula (VII):

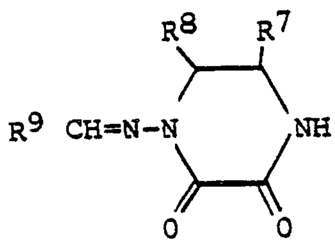

(VII)

wherein $R^7$, $R^8$ and $R^9$ are as hereinbefore defined may be prepared by processes analogous to those disclosed in US Patent 4,189,482 and US Patent 4,515,789; and may readily be converted to an N-acylating derivative of the acid of formula (VIII):

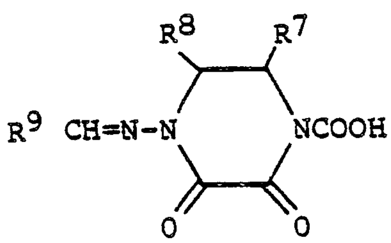

(VIII)

by activation and treatment with a compound of formula (V) as disclosed hereinabove.

Compounds of formula (IX):

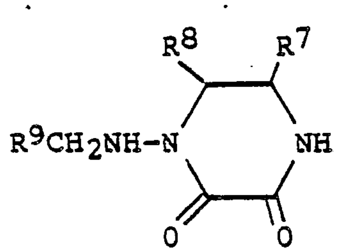

(IX)

wherein $R^7$, $R^8$ and $R^9$ are as hereinbefore defined are prepared by a reduction of compound of formula (VII), for example by hydrogenation.

Compounds of formula (X):

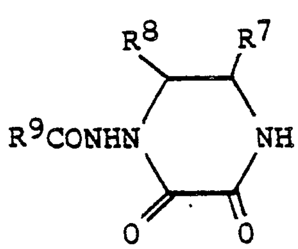

(X)

wherein $R^7$, $R^8$ and $R^9$ are as hereinbefore defined may be prepared by treatment of a compound of formula (XI):

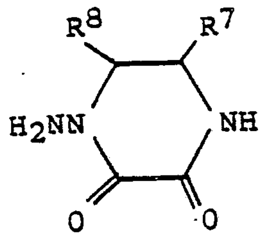

(XI)

with an acylating derivative of the acid $R^9$ COOH wherein $R^9$ is as hereinbefore defined. Compounds of formula (XI) are known from British Patent 1554799 and also may be prepared by acid hydrolysis of a compound of formula (XII):

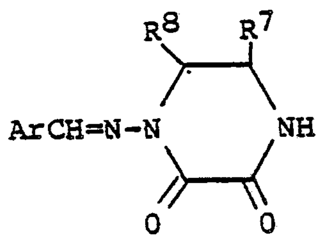

(XII)

wherein Ar represents aryl and $R^7$ and $R^8$ are as hereinbefore defined.

Compounds of formula (XIII):

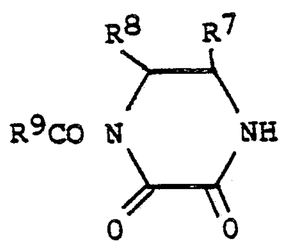

(XIII)

wherein $R^7$, $R^8$ and $R^9$ are as hereinbefore defined may be prepared from a compound of formula (XIV):

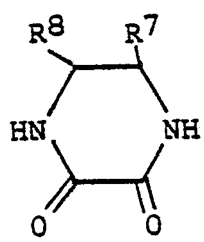

(XIV)

optionally substituted by a group which permits acylation to take place, by treatment with an acylating derivative of the acid $R^9COOH$.

Compounds of formula (XIV) are prepared by processes analogous to those disclosed in J.Am.Chem.Soc., 1948, 70, 1967 (C.E. Goulding and C.B. Pollard).

The compounds of formula (I) may also be prepared by treating a compound of formula (XV):

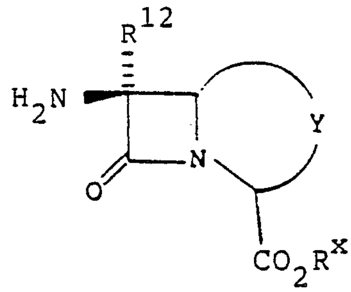

(XV)

wherein Y, $R^{12}$ and $R^x$ are as hereinbefore defined and any reactive groups may be protected or a compound of formula (XV) wherein the amino group is substituted by a group which permits acylation; with an N-acylating derivative of an acid of formula (XVI):

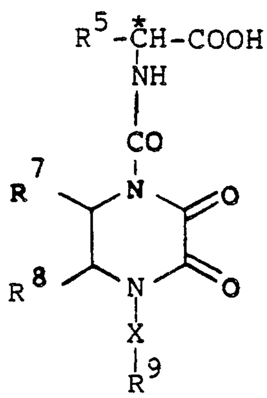

(XVI)

wherein *, $R^5$, $R^7$, $R^8$ and $R^9$ are as defined in respect of formula (I) and wherein any reactive groups therein may be protected and thereafter if necessary carrying out one or more of the following steps:

(i) removing any carboxyl protecting groups $R^x$;

(ii) removing any protecting groups on $R^9$;

(iii) removing any protecting groups on $R^5$;

(iv) converting the product into a salt or in vivo hydrolysable ester.

Suitable groups which permit acylation to take place and which are optionally present on the amino group of the starting material of the formula (XV) are as described hereinabove in respect of formula (II).

An appropriate reactive N-acylating derivative of the acid (XVI) is employed in the above process.

Suitable N-acylating derivatives include an acid halide, preferably the acid chloride or bromide. Acylation with an acid halide may be effected in the presence of an acid binding agent for example, tertiary amine (such as pyridine, 4-N,N-dimethylaminopyridine or dimethylaniline), an inorganic base (such as calcium carbonate or sodium bicarbonate) or an oxirane, which binds hydrogen halide liberated in the acylation reaction. The oxirane is preferably a $(C_{1-6})$-1,2-alkylene oxide - such as ethylene oxide or propylene oxide. The acylation reaction using an acid halide may be carried out at a temperature in the range -50°C to +50°C, preferably -20°C to +20°c, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (XVI) or a salt thereof with a halogenating (eg chlorinating or brominating) agent such as phosphorus pentachloride, thionyl chloride or oxalyl chloride.

Alternatively, the N-acylating derivative of the acid (XVI) may be a symmetrical or mixed anhydride. Suitable mixed anhydrides are alkoxyformic anhydrides, or anhydrides with, for example, carbonic acid monoesters, trimethyl acetic acid, thioacetic acid, diphenylacetic acid, benzoic acid, phosphorus acids (such as phosphoric or phosphorous acids) or aliphatic or aromatic sulphonic acids (such as methanesulphonic acid or p-toluenesulphonic acid). When a symmetrical anhydride is employed, the reaction may be carried out in the presence of 2,6-lutidine as catalyst.

Alternative N-acylating derivatives of acid (XVI) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (VII) with an oxime.

Other reactive N-acylating derivatives of the acid (XVI) include the reactive intermediates formed by reaction in situ with a condensing agent such as a carbodiimide, for example, N,N'-diethyl-, di-n-propyl- or diisopropylcarbodiimide, N,N'-di-cyclohexylcarbodiimide, or N-ethyl-N'-[3-(dimethylamino)propyl]-carbodiimide; a suitable carbonyl compound, for example, N,N'-carbonyldiimidazole or N,N'-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

Compounds of formula (II) may be prepared by reacting a compound of formula (XV) with a N-acylating derivative of an acid of formula (XVII):

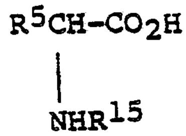

(XVII)

wherein $R^5$ and * are as hereinbefore defined and $R^{15}$ is an amino protecting group: and thereafter removing the protecting group $R^{15}$.

Suitable amino-protecting groups $R^{15}$ include those disclosed hereinabove in respect of $R^5$, $R^7$ and $R^8$.

Particularly preferred groups $R^{15}$ are 3-ethoxycarbonyl prop-2-en-2-yl 4-nitrobenzyloxycarbonyl and trichloroethyloxycarbonyl.

Compounds of formula (II) may also be prepared by reacting a compound of formula (XV) with an N-acylating derivative of an α-azido acid of formula (XVIII):

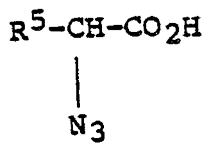

(XVIII)

wherein $R^5$ and * are as hereinbefore defined; followed by reduction of the azido group to an amino group by conventional methods such as catalytic hydrogenation, or dissolving metal reduction.

Processes for the preparation of compounds of formula (XV) in which $R^{12}$ is formamido are disclosed in the U.K. patent 2107307B.

The present invention further provides a process for the preparation of a compound of formula (I) wherein $R^6$ is formamido by reacting a compound of formula (XIX):

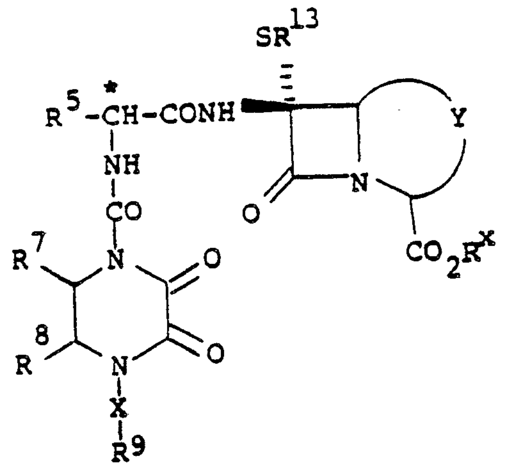

(XIX)

wherein $R^5$, $R^7$, $R^8$, $R^9$ $R^x$, X, $R^{13}$ and * are as hereinbefore defined and wherein any reactive groups may be protected; with a heavy metal ion such as mercury, silver, thallium, lead or copper and thereafter in situ with a nucleophilic derivative of formamide. This process is analogous to processes disclosed in European Patent Application No. 84300338.5 (Publication No. 0115405).

Compounds of formula (XIX) may be prepared from compounds of formula (I) in which $R^6$ is hydrogen by processes such as those disclosed in UK Patent GB-2107307B.

Compounds of formula (I) in which $R^6$ is formamido may also be prepared by formylating a compound of formula (XX):

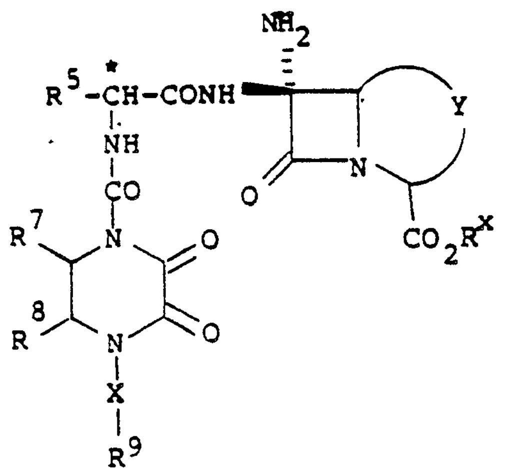

(XX)

wherein $R^5$, $R^7$, $R^8$, $R^9$, $R^x$, X and * are as hereinbefore defined and wherein any reactive groups may be protected. This process is analogous to processes disclosed in UK Patent GB-2107307B.

References to processes suitable for preparing compounds of formula (XIX) and formula (XX) have been discussed hereinabove with respect to formula (II)

The present invention still further provides a process for the preparation of a compound of formula (I) wherein $R^6$ is methoxy by reacting a compound of formula (XIX) with methyl alcohol in the presence of metal ions such as silver, thallium or mercuric ions. This process is analogous to processes disclosed in UK Patent 1439898 and European Patent publications 0027010 and 0070103.

The antibiotic compounds of the invention are active against a wide range of Gram-negative and Gram-positive organisms including E.coli such as NCTC 10418, DC2 and DCO, Klebsiella pneumoniae T767 and Pseudomonas Spp. such as P.aeruginosa for example K 79961 and 10662. Compounds of formula (I) in which $R^6$ is not hydrogen exhibit improved $\beta$-lactamase stability over those in which $R^6$ is hydrogen.

The following Examples illustrate the preparation and biological activity of the compounds of the invention.

Example 1

Sodium 6β-[D-2[[4-(3,4-Dihydroxybenzyl)-2,3-dioxopiperazin-1-yl]carbonylamino]-2-phenylacetamido]-penicillanate

(a) 1-(3,4-Dibenzyloxybenzyl)-2,3-dioxopiperazine

N-(3,4-Dibenzyloxybenzyl)ethylenediamine (7.8g) and diethyl oxalate (5.84g) were refluxed in ethanol for 4h. The solvent was removed under reduced pressure and the residue recrystallised from ethanol to give the title compound (5.0g; 60%), mp 194-195° C (Found: C, 71.7; H, 5.8; N, 6.5. $C_{25}H_{24}N_2O_4$ requires C, 72.1; H, 5.8; N, 6.7%); $\nu_{max}$ (KBr) 1704 and 1661cm$^{-1}$; $\delta_H$ (250MHz; $CDCl_3$) 3.24 (4H, bs, $N(CH_2)_2N$), 4.54 (2H, bs, $NCH_2Ar$), 5.17 (4H, bs, 2 × $CH_2Ph$), 6.73-7.47 (13H, m, Ar), 7.98 (1H, bs, exchangeable, NH) (Found: m/z 416.1737. M$^+$, $C_{25}H_{24}N_2O_4$ requires 416.1736).

(b) Sodium 6β-[D-2-[[4-(3,4-Dibenzyloxybenzyl)-2,3-dioxopiperazin-1-yl]carbonylamino]-2-phenylacetamido]-penicillanate

1-(3,4-Dibenzyloxybenzyl)-2,3-dioxopiperazine (416mg) was dissolved in dry dichloromethane (10ml) under Argon, cooled to 0-5° C and treated with trimethylsilyl chloride (240mg). After 15 min, triethylamine (111mg) was added and the mixture was stirred at 0-5° C for 1.5h. The reaction mixture was treated with excess phosgene (liquid) and stirred at 0-5° C for 15 min before being allowed to warm to ambient temperature over 0.5h. The reaction mixture was stirred at ambient temperature for 1h then evaporated to dryness under reduced pressure. The residue, $\nu_{max}$ ($CHCl_3$) 1800cm$^{-1}$, was suspended in acetone (10ml) and the suspension added, dropwise, to a solution of sodium 6β-[D-2-amino-2-phenylacetamido]-penicillanate (371mg) in water (10ml) and acetone (10ml). The pH of the reaction was maintained between 7 and 8 by addition of saturated aqueous sodium hydrogen carbonate when necessary. The reaction mixture was stirred at ambient temperature for 15 min after the addition was complete before it was diluted with ethyl acetate (75ml) and water (25ml). The phases were separated, the aqueous phase washed with ethyl acetate (50ml) and then acidified to pH 2 with 5M hydrochloric acid in the presence of ethyl acetate (50ml). The phases were separated, the aqueous phase further extracted with ethyl acetate (25ml), the extracts combined, washed with water at pH 2 (25ml), water (25ml), saturated brine (25ml), dried ($MgSO_4$) and evaporated to dryness under reduced pressure to give the free acid (350mg; 44%) as a white powder.

The free acid was dissolved in acetone (minimum) and treated with a 1.93M solution of sodium 2-ethylhexanoate in methyl iso-butyl ketone (227μl). Diethyl ether (50ml) was added and the title compound collected by filtration, washed with diethyl ether and dried under reduced pressure (324mg; 40%); $\nu_{max}$ (KBr) 1766, 1713, 1683, and 1604cm$^{-1}$; $\delta_H$ [250MHz; $(CD_3)_2SO$ + $D_2O$] 1.38 and 1.51 (6H, 2s, 2-$C(CH_3)_2$), 3.68-3.87 (4H, bm, $N(CH_2)_2N$), 3.90 (1H, s, 3-H), 4.38-4.57 (2H, m, $NCH_2Ar$), 5.12 (4H, s, 2 × $CH_2Ph$), 5.28 (1H, d, J 4Hz, 5-H), 5.41 (1H, d, J 4Hz, 6-H), 5.73 (1H, bs, 2'-H), and 6.84-7.50 (18H, m, Ar); m/z (F.A.B.) 836 (MNa$^+$, 8%), 461 (50), 439 (100), 420 (20), 257 (36), and 204 (22).

(c) Sodium 6β-[D-2-[[4-(3,4-Dihydroxobenzyl)-2,3-dioxopiperazin-1-yl]carbohylamino]-2-phenylacetamido]-penicillanate

Sodium 6β-[D-2-[[4-(3,4-dibenzyloxybenzyl)-2,3-dioxopiperazin-1-yl]carbonylamino]-2-phenylacetamido]-penicillanate (180mg) was dissolved in water (20ml) and hydrogenated at standard temperature and pressure over 10% palladium on carbon (180mg) and palladium black (180mg). After 2.5h, the mixture was filtered through 'Celite'. The filtrate was washed with ethyl acetate (20ml) and acidified to pH 2 with 5M hydrochloric acid in the presence of ethyl acetate (50ml). The phases were separated, the aqueous phase further extracted with ethyl acetate (20ml), the extracts combined, washed with water and saturated brine, dried ($MgSO_4$) and evaporated to dryness under reduced pressure to give the acid (92mg; 66%).

The acid was dissolved in acetone (minimum) and treated with a 1.93M solution of sodium 2-ethylhexanoate in methyl iso-butyl ketone. Diethyl ether (50ml) was added and the title compound (87mg;

60%) collected by filtration, washed with diethyl ether and dried under reduced pressure, $\nu_{max}$ (KBr) 1764, 1712, 1676, and 1605cm$^{-1}$; $\delta_H$ [250MHz; $(CD_3)_2SO$] 1.41 and 1.54 (6H, 2s, 2-$C(CH_3)_2$), 3.32-3.51 (4H, m, N-$(CH_2)_2N$), 3.83 (1H, s, 3-H), 4.41 (2H, bs, $NCH_2Ar$), 5.24 (1H, d, J 4Hz, 5-H), 5.32-5.43 (1H, m, 6-H), 5.73 (1H, d, J 7Hz, 2'-H), 6.50-6.70 (3H, m, $C_6H_3$), 7.24-7.50 (5H, m, $C_6H_5$) 9.28 (1H, bd, J 5Hz, exchangeable, 6β-CONH), and 9.84 (1H, d, J 7Hz, exchangeable, 2'-NH); m/z (F.A.B.) 656 ($MNa^+$, 28%), 634 ($MH^+$, 19), 556 (15), 530 (8), 420 (80), 398 (58), 394 (33), 302 (31), and 301 (100).

Example 2

Sodium 6β-[D-2[[4-(3,4-Dihydroxybenzyl)-2,3-dioxopiperazin-1-yl]carbonylamino]-2-phenylacetamido]-6α-formamidopenicillanate

(a) Benzyl 6β-[D-2-[[4-(3,4-Dibenzyloxybenzyl)-2,3-dioxopiperazin-l-yl]carbonylamino]-2-phenylacetamido]-6α-methylthiopenicillanate

A solution of benzyl 6β-[D-2-amino-2-phenylacetamido]-6α-methylthiopenicillanate (485mg) in tetrahydrofuran (10ml) containing triethylamine (101mg) was treated with a suspension of 4-(3,4-dibenzyloxybenzyl)-2,3-dioxopiperazin-1-ylcarbonyl chloride in tetrahydrofuran (10ml) (prepared from 1-(3,4-dibenzyloxybenzyl)-2,3-dioxopiperazine (416mg) as described in Example 1). After 0.5h, the reaction mixture was diluted with ethyl acetate (50ml) and water (50ml) and the phases separated. The organic phase was washed with water (2 × 25ml), saturated brine (25ml), dried ($MgSO_4$) and evaporated to dryness under reduced pressure. The crude ester was purified by chromatography through silica (<230 mesh ASTM), eluting with 50% ethyl acetate in cyclohexane to give the title compound (650mg; 70%) as an off-white foam, $\nu_{max}$ (KBr) 1779, 1742, 1714, and 1683cm$^{-1}$; $\delta_H$ (250MHz; $CDCl_3$) 1.01 and 1.23 (6H, 2s, 2-$C(CH_3)_2$), 2.29 (3H, s, $SCH_3$), 3.00-3.30, 3.41-3.55, and 3.71-3.84 (4H, 3m, $N(CH_2)_2N$), 4.33 (1H, s, 3-H), 4.41 and 4.58 (2H, ABq, J 12Hz, $NCH_2Ar$), 5.10-5.25 (6H, m, 3 × $CH_2Ph$), 5.50 (1H, d, J 7Hz, 2'-H), 5.54 (1H, s, 5-H), 6.66-6.89, 7.07-7.17, and 7.22-7.56 (24H, 3m, aromatics and 6β-CONH, exchangeable), 10.02 (1H, d, J 7Hz, exchangeable, 2'-NH); m/z (F.A.B.) 928 ($MH^+$, 20%), 884 (13), 548 (20), 325 (20), 303 (78), 279 (41), 250 (100), and 232 (27).

(b) Benzyl 6β-[D-2-[[4-(3,4-Dibenzyloxybenzyl)-2,3-dioxopiperazin-1-yl]carbonylamino]-2-phenylacetamido]-6α-formamidopenicillanate

Benzyl 6β-[D-2-[[4-(3,4-dibenzyloxybenzyl)-2,3-dioxopiperazin-1-yl]carbonylamino]-2-phenylacetamido]-6α-methylthiopenicillanate (400mg) in dry N,N-dimethylformamide (5ml) was treated with mercuric acetate (138mg) followed by ammonia in dry N,N-dimethylformamide (1.1 eq). After 15 min, the reaction mixture was diluted with ethyl acetate (50ml) and filtered through 'Celite'. The filtrate was washed with water (5 × 25ml), saturated brine (25ml), dried ($MgSO_4$) and evaporated to dryness under reduced pressure. The residue was dissolved in dry dichloromethane (20ml) under Argon and treated at ambient temperature with pyridine (341mg) and acetic formic anhydride (190mg). After 3h, the reaction mixture was washed with water (20ml), saturated aqueous sodium hydrogen carbonate (20ml), water (20ml), saturated aqueous copper (II) sulphate (20ml), water (20ml) and saturated brine (20ml) before being dried ($MgSO_4$) Evaporation to dryness under reduced pressure gave a foam, which was purified by chromatography trough silica (<230 mesh ASTM), eluting with 20% cyclohexane in ethyl acetate (100ml), 10% cyclohexane in ethyl acetate (100ml), ethyl acetate (100ml) and finally 10% ethanol in ethyl acetate. The title compound was obtained as a foam (203mg, 51%), $\nu_{max}$ (KBr) 1785, 1740, 1715, and 1687cm$^{-1}$; $\delta_H$ [250MHz; $(CD_3)_2CO$] 0.90 and 1.15 (6H, 2s, 2-$C(CH_3)_2$), 3.35-3.54 and 3.73-3.90 (4H, 2m, $N(CH_2)_2N$), 4.39 (1H, s, 3-H), 4.48 and 4.68 (2H, ABq, J 14Hz, $NCH_2Ar$), 5.11-5.28 (6H, m, 2 × $OCH_2Ph$ and $CO_2CH_2Ph$), 5.59 (1H, s, 5-H), 5.71 (1H, d, J 7Hz, 2'-H), 6.86-7.09 (3H, m, $C_6H_3$), 7.17-7.63 (20H, m, 4 × $C_6H_5$), 8.18 (1H, d, J 1Hz, CHO), 8.26 (1H, bs, exchangeable, NHCHO), 8.78 (1H, s, exchangeable, 6β-CONH), and 10.08 (1H, d, J 7Hz, exchangeable, 2'-NH); m/z (F.A.B.) 942 ($MNa^+$, 16%), 925 ($MH^+$, 20), 303 (12), 250 (100), and 232 (10).

(c) Sodium 6β-[D-2-[[4-(3,4-Dihydroxybenzyl)-2,3-dioxopiperazin-1-yl]carbonylamino]-2-phenylacetamido]-6α-formamidopenicillanate

Benzyl 6β-[D-2-[[4-(3,4-dibenzyloxybenzyl)-2,3-dioxopiperazin-1-yl]carbonylamino]-2-phenylacetamido]-6α-formamidopenicillanate (276mg) was dissolved in redistilled tetrahydrofuran [25ml) and hydrogenated at standard temperature and pressure for 2h over 10% palladium on carbon (276mg). The catalyst was removed by filtration through Celite, the filter bed washed with redistilled tetrahydrofuran (25ml) and the filtrate hydrogenated for a further 2.5h at standard temperature and pressure over a mixture of 10% palladium on carbon (280mg) and palladium black (280mg). The catalyst was removed by filtration through Celite, th filter bed washed with redistilled tetrahydrofuran (25ml) and the filtrate evaporated to dryness under reduced pressure to yield the free acid (190mg; 97%). This was dissolved in acetone (minimum) and the solution treated with 1.93M sodium 2-ethylhexanoate in methyl iso-butyl ketone (0.15ml). Diethyl ether was added (50ml) and the product collected by filtration, washed with diethyl ether and dried under reduced pressure (160mg; 79%), $\nu_{max}$ (KBr) 1770, 1710, 1676, and 1608cm$^{-1}$; $\delta_H$ (250MHz; $D_2O$) 0.84 and 1.23 (6H, 2s, 2-$C(CH_3)_2$), 3.49-3.61 and 3.83-3.96 (4H, 2m, $N(CH_2)_2N$), 4.13 (1H, s, 3-H), 4.54 (2H, bs, $NCH_2C_6H_3$), 5.44 (1H, s, 5-H), 5.56 (1H, s, 2'-H), 6.76 (1H, bd, J 8Hz, Ar 6-H), 6.84 (1H, bs, Ar 2-H), 6.88 (1H, d, J 8Hz, Ar 5-H), 7.36-7.54 (5H, m, $C_6H_5$), and 8.09 (1H, s, CHO); m/z (F.A.B.) 677 ($MH^+$, 98%), 655 (62), 441 (100), and 413 (47).

## Example 3

### Sodium 6β-[D-2-[[4-(3,4-Dihydroxybenzyl)-2,3-dioxopiperazin-1-yl]carbonylamino]-2-phenylacetamido]-6α-methoxypenicillanate

(a) Benzyl 6β-[D-2-[[4-(3,4-Dibenzyloxybenzyl)-2,3-dioxopiperazin-1-yl]carbonylamino]-2-phenylacetamido]-6α-methoxypenicillanate

Benzyl 6β-[D-2-[[4-(3,4-dibenzyloxybenzyl)-2,3-dioxopiperazin-1-yl]carbonylamino]-2-phenylacetamido]-6α-methylthiopenicillanate (204mg) was suspended in methanol (20ml) and complete dissolution obtained by addition of tetrahydrofuran (minimum). The reaction was purged with Argon and treated with mercuric acetate (70mg). After 0.5h, the reaction mixture was filtered through Celite and evaporated to dryness under reduced pressure. The crude foam was purified by chromatography through silica (<230 mesh ASTM), eluting with 50% ethyl acetate in cyclohexane grading to ethyl acetate giving the title compound (177mg; 88%), $\nu_{max}$ (KBr) 1776, 1742, 1714, and 1685cm$^{-1}$; $\delta_H$ [250MHz; $(CD_3)_2CO$] 0.95 and 1.20 (6H, 2s, 2-$C(CH_3)_2$), 3.47 (3H, s, $OCH_3$), 3.37-3.52 and 3.75-3.94 (4H, 2m, $N(CH_2)_2N$), 4.38 (1H, s, 3-H), 4.58 (2H, bs, $NCH_2C_6H_3$), 5.09-5.27 (6H, m, 2 × $OCH_2Ph$ and $CO_2CH_2Ph$), 5.46 (1H, s, 5-H), 5.69 (1H, d, J 7Hz, 2'-H), 6.86-7.07 (3H, m, $C_6H_3$), 7.16-7.63 (20H, m, 4 × $C_6H_5$), 8.79 (1H, bs, exchangeable, 6β-CONH), and 9.98 (1H, d, J 7Hz, exchangeable, 2'-NH); m/z (F.A.B.) 912 ($MH^+$, 10%), 576 (7), 548 (25), 518 (8), 458 (12), 303 (81), 250 (100), 246 (22), and 235 (9).

(b) Sodium 6β-[D-2-[[4-(3,4-Dihydroxybenzyl)-2,3-dioxopiperazin-1-yl]carbonylamino]-2-phenylacetamido]-6α-methoxypenicillanate

Benzyl 6β-[D-2-[[4-(3,4-dibenzyloxybenzyl)-2,3-dioxopiperazin-1-yl]carbonylamino]-2-phenylacetamido]-6α-methoxypenicillanate (160mg) was dissolved in redistilled tetrahydrofuran (25ml) and hydrogenated at standard temperature and pressure over 10% palladium on carbon (160mg). After 2h, the catalyst was removed by filtration through Celite, the filter bed washed with redistilled tetrahydrofuran (25ml) and the filtrate evaporated to dryness under reduced pressure. The residue was redissolved in redistilled tetrahydrofuran (20ml) and sodium hydrogen carbonate (14.7mg) in water (5ml) added. This mixture was hydrogenated at standard temperature and pressure over a mixture of 10% palladium on carbon (100mg) and palladium black (100mg) for 3h. The catalyst was removed by filtration through Celite, the filter bed washed with water (20ml) and the filtrate washed with ethyl acetate (25ml) before being acidified to pH 2

with 5M hydrochloric acid in the presence of ethyl acetate (25ml). The phases were separated, the aqueous phase further extracted with ethyl acetate (25ml), the extracts combined, washed with water (25ml), water at pH 2 (25ml), water (25ml) and saturated brine (25ml) before being dried ($MgSO_4$). Evaporation of the solvents under reduced pressure gave the acid (76.5mg; 70%), which was redissolved in acetone (minimum) and treated with 1.93M sodium 2-ethylhexanoate in methyl iso-butyl ketone (61µl). Diethyl ether (50ml) was added and the title compound (91mg; 78%) collected by filtration, washed with diethyl ether and dried under reduced pressure, $\nu_{max}$ (KBr) 1763, 1712, 1683, and 1609cm$^{-1}$; $\delta_H$ (250MHz; $D_2O$) 0.81 and 1.23 (6H, 2s, 2-$C(CH_3)_2$), 3.34-3.52 and 3.68-3.88 (4H, 4m, $N(CH_2)_2N$), 3.49 (3H, s, $OCH_3$), 4.09 (1H, s, 3-H), 4.45 (2H, m, $NCH_2C_6H_3$), 5.41 (1H, s, 5-H), 5.44 (1H, s, 2′-H), 6.66-6.86 (3H, m, $C_6H_3$), and 7.30-7.47 (5H, m, $C_6H_5$); m/z (F.A.B.) 686 ($MNa^+$, 7%), 664 ($MH^+$, 13), 428 (20), 391 (25), 282 (28), 279 (19), 259 (31), 239 (100), 237 (55), and 221 (16).

## Example 4

### Sodium 6β-[D-2-[[4-(3,4-Dihydroxybenzyl)-2,3-dioxopiperazin-1-yl]carbonylamino]-2-(4-hydroxyphenyl)-acetamido]-6α-formamidopenicillanate

#### (a) Benzyl 6β-[D-2-(4-Benzyloxycarbonyloxyphenyl)-2-[[4-(3,4-dibenzyloxybenzyl)-2,3-dioxopiperazin-1-yl]-carbonylamino]acetamido]-6α-methylthiopenicillanate

Benzyl 6β-[D-2-(4-benzyloxycarbonyloxyphenyl)-2-(4-nitrobenzyloxycarbonylamino)acetanido]-6α-methylthiopenicillanate (814mg) was dissolved in tetrahydrofuran (minimum), the solution diluted to turbidity with water then clarified by addition of tetrahydrofuran. The pH of the solution was adjusted to 2 with 5M hydrochloric acid and zinc powder (1.6g) was added. The mixture was stirred vigorously and the pH maintained at 2-3 by addition of more 5M hydrochloric acid when necessary. When deprotection was complete, the mixture was diluted with ethyl acetate (50ml) and filtered through Celite. The filter bed was washed with ethyl acetate (25ml) and the phases in the filtrate separated. The organic phase was washed with water (25ml) and saturated brine (25ml) before drying ($MgSO_4$). Removal of the solvents gave crude benzyl 6β-[D-2-amino-2-(4-benzyloxycarbonyloxyphenyl)acetamido]-6α-methylthiopenicillanate. This was purified by chromatography through silica (<230 mesh ASTM), eluting with chloroform then 10% methanol in chloroform, giving the amine as a foam (558mg; 88%). The amine was dissolved in tetrahydrofuran (10ml), triethylamine (101mg) added and this solution treated with a suspension of 4-(3,4-dibenzyloxybenzyl)2,3-dioxopiperazin-1-ylcarbonyl chloride (formed from 1-(3,4-dibenzyloxybenzyl)-2,3-dioxopiperazine (416mg) as described in Example 1). After 1h at room temperature, the reaction mixture was diluted with ethyl acetate (50ml) and water (20ml) and the phases separated. The organic phase was washed with water (25ml), saturated brine (25ml), dried ($MgSO_4$) and the solvents removed under reduced pressure. Chromatography through silica (<230 mesh ASTM), eluting with 50% ethyl acetate in cyclohexane, gave the title compound (471mg; 44%), $\nu_{max}$ ($CHCl_3$) 1760, 1715, and 1685cm$^{-1}$; $\delta_H$ [250MHz; $(CD_3)_2CO$] 1.01 and 1.18 (6H, 2s, 2-$C(CH_3)_2$), 2.27 (3H, s, $SCH_3$), 3.37-3.55 and 3.74-3.95 (4H, 2m, $N(CH_2)_2N$), 4.38 (1H, s, 3-H), 4.57 (2H, bs, $NCH_2C_6H_3$), 5.14-5.27 (8H, m, 4 × $CH_2Ph$), 5.45 (1H, s, 5-H), 5.74 (1H, d, J 7Hz, 2′-H), 6.95-7.68 (27H, m, Ar), 8.82 (1H, s, exchangeable, 6β-CONH), and 10.07 (1H, d, J 7Hz, exchangeable, 2′-NH); m/z (F.A.B.) 1100 ($MNa^+$, 10%), 859 (9), 505 (20), 482 (65), 460 (21), 434 (20) and 411 (100).

#### (b) Benzyl 6β-[D-2-(4-Benzyloxycarbonyloxyphenyl)-2-[[4-(3,4-dibenzyloxybenzyl)-2,3-dioxopiperazin-1-yl]-carbonylamino]acetamido]-6α-formamidopenicillanate

Benzyl 6β-[D-2-(4-benzyloxycarbonyloxyphenyl)-2-[[4-(3,4-dibenzyloxybenzyl)-2,3-dioxopiperazin-1-yl]-carbonylamino]acetamido]-6α-methylthiopenicillanate (470mg) was dissolved in dry N,N-dimethylformamide (5ml) and treated at ambient temperature with mercuric acetate (139mg) and then a 10% excess of ammonia in dry N,N-dimethylformamide. After 15 min, the reaction mixture was diluted with ethyl acetate (50ml) and filtered through Celite. The filter bed was washed with ethyl acetate (25ml) and the filtrate washed with water (5 × 25ml), saturated brine (25ml), dried ($MgSO_4$) and evaporated to dryness under

reduced pressure. The residue was redissolved in dry dichloromethane (20ml), pyridine (345mg) and acetic formic anhydride (192mg) added and the mixture stirred at ambient temperature for 3h. The reaction mixture was washed with water (25ml), saturated aqueous sodium hydrogen carbonate (25ml), water (25ml), saturated aqueous copper (II) sulphate (25ml), water (25ml), saturated brine (25ml) and dried ($MgSO_4$). Evaporation under reduced pressure then chromatography through silica (<230 mesh ASTM) eluting with chloroform then ethyl acetate, gave the title compound (350mg; 75%), $\nu_{max}$ (KBr) 1781, 1761, 1710, and 1687cm$^{-1}$; $\delta_H$ [250MHz; $(CD_3)_2CO$] 0.94 and 1.19 (6H, 2s, 2-$C(CH_3)_2$), 3.33-3.55 and 3.71-3.94 (4H, 2m, N-$(CH_2)_2$N), 4.40 (1H, s, 3-H), 4.39 and 4.77 (2H, ABq, J 15Hz, $NCH_2C_6H_3$), 5.13-5.31 (8H, m, 4 × $CH_2$Ph), 5.60 (1H, s, 5-H), 5.77 (1H, d, J 7Hz, 2′-H), 6.87-7.72 (27H, m, Ar), 8.18 (1H, s, CHO), 8.28 (1H, bs, exchangeable, NHCHO), 8.90 (1H, s, exchangeable, 6β-CONH), and 10.13 (1H, d, J 7Hz, exchangeable, 2′-NH); m/z (F.A.B.) 1075 ($MH^+$, 44%), 323 (7), 303 (11), and 250 (100).

(c) Sodium 6β-[D-2-[[4-(3,4-Dihydroxybenzyl)-2,3-dioxopiperazin-1-yl]carbonylamino]-2-(4-hydroxyphenyl)-acetamido]-6α-formamidopenicillanate

Benzyl 6β-[D-2-(4-benzyloxycarbonyloxyphenyl)-2-[[4- (3,4-dibenzyloxybenzyl)-2,3-dioxopiperazin-1-yl]-carbonylamino]acetamido]-6α-formamidopenicillanate (350mg) in redistilled tetrahydrofuran (25ml) was hydrogenated over 10% palladium on carbon (350mg) for 2h. Filtered through Celite, the filter bed washed with redistilled tetrahydrofuran (10ml) and 10% palladium on carbon (350mg) and palladium black (350mg) added to the filtrate. The mixture was then hydrogenated at standard temperature and pressure for a further 4h before the above process was repeated. When deprotection was complete (by HPLC) the catalyst was removed by filtration through Celite, the filter bed washed with redistilled tetrahydrofuran (10ml) and the filtrate evaporated to dryness under reduced pressure to give the free acid (200mg). This was redissolved in acetone (minimum) and treated with 1.93M sodium 2-ethylhexanoate in methyl iso-butyl ketone (0.15ml). Diethyl ether (50ml) was added and the sodium salt (130mg; 59%) collected by filtration, washed with diethyl ether (50ml) and dried under reduced pressure, $\nu_{max}$ (KBr) 1772, 1715, 1683 and 1609cm$^{-1}$; $\delta_H$ - (250MHz; $D_2O$) 0.80 and 1.27 (6H, 2s, 2-$C(CH_3)_2$), 3.30-3.48 and 3.68-3.90 (4H, 2m, $N(CH_2)_2N$), 4.11 (1H, s, 3-H), 4.44 and 4.47 (2H, ABq, J 15Hz, $NCH_2C_6H_3$), 5.29 (1H, s, 5-H), 5.54 (1H, s, 2′-H), 6.64-7.30 (7H, m, Ar), and 8.06 (1H, s, CHO); m/z (F.A.B.) 693 ($MH^+$, 3%), 457 (6), 439 (6), 323 (5), 295 (7), 279 (10), 257 (5), 237 (100), 232 (18), 217 (80), 215 (52), 214 (38), and 205 (11).

Example 5

Sodium 6β-[D-2-(3,4-Diacetoxyphenyl)-2-[[4-(3,4-dihydroxybenzyl)-2,3-dioxopiperazin-1-yl]carbonylamino] acetamido]-6α-formamidopenicillanate

(a) Benzyl 6β-[D-2-(3,4-Diacetoxyphenyl)-2-[[4-(3,4-dibenzyloxybenzyl)-2,3-dioxopiperazin-1-yl]-carbonylamino]acetamido]-6α-methylthiopenicillanate

Benzyl 6β-[D-2-(3,4-diacetoxyphenyl)-2-(4-nitrobenzyloxycarbonylamino)acetamido]-6α-methylthiopenicillanate (780mg) was dissolved in tetrahydrofuran (20ml). Water was added to turbidity then tetrahydrofuran was added to obtain a clear solution. Zinc (1.6g) was added, the pH adjusted to 2 with 5M hydrochloric acid and the mixture stirred vigorously until deprotection was complete (by TLC). The pH was maintained between 2 and 3 by addition of 5M hydrochloric acid when necessary. When deprotection was complete, the reaction mixture was diluted with ethyl acetate (50ml) and water (20ml) and filtered through Celite. The filter bed was washed with ethyl acetate (20ml) and the phases in the filtrate separated. The organic phase was washed with water (2 × 25ml), saturated brine (25ml), dried ($MgSO_4$) and evaporated to dryness under reduced pressure. Chromatography through silica (<230 mesh ASTM), eluting with chloroform then 10% ethanol in chloroform, gave benzyl 6β-[D-2-(3,4-diacetoxyphenyl)-2-aminoacetamido]-6α-methylthiopenicillanate as a white foam. This was redissolved in tetrahydrofuran (10ml), triethylamine (101mg) added followed by a suspension of 4-(3,4-dibenzyloxybenzyl)-2,3-dioxopiperazin-1-ylcarbonyl chloride (prepared as described in Example 1 from 1-(3,4-dibenzyloxybenzyl)-2,3-dioxopiperazine (416mg)).

After 0.5h, the mixture was diluted with ethyl acetate (50ml) and water (25ml) and the phases separated. The organic phase was washed with water (25ml), saturated brine (25ml), dried (mgSO4) and evaporated to dryness under reduced pressure Chromatography through silica (<230 mesh ASTM), eluting with 50% ethyl acetate in cyclohexane, gave the title compound (480mg; 47%) as a white foam, $\nu_{max}$ (KBr) 1774, 1750 sh, 1713 and 1687cm$^{-1}$; $\delta_H$ [250MHz; $(CD_3)_2CO$] 1.07 and 1.24 (6H, 2s, 2-$C(CH_3)_2$), 2.14, 2.16 and 2.18 (9H, 3s, 2 × $CH_3CO_2$ and $SCH_3$), 3.38-3.56 and 3.74-3.95 (4H, 2m, $N(CH_2)_2N$), 3.58 (2H, bs, $NCH_2Ar$), 4.39 (1H, s, 3-H), 5.15-5.21 (6H, m, 3 × $CH_2Ph$), 5.44 (1H, s, 5-H), 5.75 (1H, d, J 7Hz, 2'-H), 6.87-7.56 (21H, m, Ar), 8.88 (1H, s, exchangeable, 6β-CONH), 10.08 (1H, d, J 7Hz, exchangeable, 2'-NH); m/z (F.A.B.) 1066 ($MNa^+$, 32%), 672 (12), 650 (100), 602 (14), 482 (14), 461 (23), 439 (35), 434 (9) and 411 (24).

(b) Benzyl 6β-[D-2-(3,4-Diacetoxyphenyl)-2-[[4-(3,4-dibenzyloxybenzyl)-2,3-dioxopiperazin-1-yl]-carbonylamino]acetamido]-6α-formamidopenicillanate

Benzyl 6β-[D-2-(3,4-diacetoxyphenyl)-2-[[4-(3,4-dibenzyloxybenzyl)-2,3-dioxopiperazin-1-yl]-carbonylamino] acetamido]-6α-methylthiopenicillanate (480mg) was dissolved in dry N,N-dimethylformamide (5ml) and treated with mercuric acetate (147mg) then a 10% excess of ammonia in dry N,N-dimethylformamide. After 15 min, the reaction mixture was diluted with ethyl acetate (50ml) and filtered through Celite. The filter bed was washed with ethyl acetate (25ml) and the filtrate washed with water (5 × 25ml), saturated brine (25ml), dried ($MgSO_4$) and evaporated to dryness under reduced pressure. The residue was dissolved in dry dichloromethane (20ml) and treated with pyridine (364mg) then acetic formic anhydride (202mg). After 3h, the reaction mixture was washed with water (20ml), saturated aqueous sodium hydrogen carbonate (20ml), water (20ml), saturated copper (II) sulphate (20ml), water (20ml) and saturated brine (20ml) before drying ($MgSO_4$) and evaporation to dryness under reduced pressure. Chromatography through silica (<230 mesh ASTM), eluting with chloroform then 80% ethyl acetate in cyclohexane grading to ethyl acetate, gave the title compound (276mg; 58%) as a foam, $\nu_{max}$ (KBr) 1776, 1745, 1715, and 1688cm$^{-1}$; $\delta_H$ [250MHz; $(CD_3)_2CO$] 0.98 and 1.23 (6H, 2s, 2-$C(CH_3)_2$), 2.24 and 2.25 (6H, 2s, 2 × $CH_3CO_2$), 3.35-3.56 and 3.72-3.95 (4H, 2m, $N(CH_2)_2N$), 4.40 (1H, s, 3-H), 4.44 and 4.64 (2H, ABq, J 15Hz, $NCH_2C_6H_3$), 5.11-5.24 (6H, m, 3 × $CH_2Ph$), 5.59 (1H, s, 5-H), 5.76 (1H, d, J 7Hz, 2'-H), 6.86-7.58 (21H, m, Ar), 8.17 (1H, d, J 1Hz, CHO), 8.28 (1H, bs, exchangeable, NHCHO), 8.93 (1H, bs, exchangeable, 6β-CONH), and 10.12 (1H, d, J 7Hz, exchangeable, 2'-NH); m/z (F.A.B.) 1041 ($MH^+$, 7%), 303 (13), and 250 (100).

(c) Sodium 6β-[D-2-(3,4-Diacetoxyphenyl)-2-[[4-(3,4-dihydroxybenzyl)-2,3-dioxopiperazin-1-yl]-carbonylamino]acetamido]-6α-formamidopenicillanate

Benzyl 6β-[D-2-(3,4-diacetoxyphenyl)-2-[[4-(3,4-dibenzyloxybenzyl)-2,3-dioxopiperazin-1-yl]-carbonylamino] acetamido]-6α-formamidopenicillanate (250mg) in redistilled tetrahydrofuran (20ml) was hydrogenated at standard temperature and pressure over 10% palladium on carbon (250mg) for 1.5h. The reaction mixture was filtered through Celite and the filter bed washed with redistilled tetrahydrofuran (20ml). The filtrate was hydrogenated at standard temperature and pressure for 4h over 10% palladium on carbon (200mg) and palladium black (200mg). The catalyst was removed by filtration through Celite, washed with redistilled tetrahydrofuran (20ml) and the filtrate evaporated to dryness under reduced pressure to give the acid (185mg; 100%). The free acid was redissolved in acetone (minimum) and treated with 1.93M sodium 2-ethylhexanoate in methyl iso-butyl ketone (0.10ml). Diethyl ether (100ml) was added and the title compound (149mg; 78%) collected by filtration, washed with dietyl ether and dried under reduced pressure, $\nu_{max}$ (KBr) 1770, 1715, 1680, and 1608cm$^{-1}$; $\delta_H$ (250MHz; $D_2O$) 0.84 and 1.23 (6H, 2s, 2-$C(CH_3)_2$), 2.25 and 2.26 (6H, 2s, 2 × $CH_3CO_2$), 3.24-3.52 and 3.61-3.85 (4H, 2m, $N(CH_2)_2N$), 4.14 (1H, s, 3-H), 4.40 and 4.48 (2H, ABq, J 15Hz, $NCH_2C_6H_3$), 5.42 (1H, s, 5-H), 5.57 (1H, s, 2'-H), 6.68-6.83 (3H, m, $C_6H_3(OH)_2$), 7.15-7.42 (3H, m, $C_6H_3(OAc)_2$), and 8.07 (1H, s, CHO); m/z (F.A.B.) 793 ($MH^+$, 7%), 579 (7), 557 (48), 259 (28), 206 (28), 160 (60), 131 (100), 123 (80), and 115 (76).

Example 6

Sodium 6β-[D-2-[[4-(4,5-Dihydroxypyridin-2-ylmethyl)-2,3-dioxopiperazin-1-yl]carbonylamino]-2-phenylacetamido]-6α-formamidopenicillanate

(a) 4,5-Dibenzyloxy-2-hydroxymethylpyridine

A mixture of 5-benzyloxy-4-hydroxy-2-hydroxymethylpyridine (2.31g), benzyl chloride (1.27ml, 1.1 eq) and potassium carbonate (2.76g, 2 eq) in DMF (50ml) was heated at 50°C for 18h. The reaction mixture was poured into water (100ml) and extracted with dichloro methane. After washing with brine, the combined extracts were dried ($MgSO_4$) and evaporated to dryness. The residue was triturated with dichloromethane and the crystalline 1-benzyl-5-benzyloxy-2-hydroxymethylpyrid-4-one filtered off (0.81g, 25%) m.p. 143-4°C (Found: C, 74.5; H, 6.0; N, 4.3. $C_{20}H_{19}NO_3$ requires C, 74.7; H, 6.0; N, 4.4%), $\nu_{max}$ (KBr) 3166, 1628, 1553, 1524, 1494, 1479, and 1450cm$^{-1}$; $\delta_H$ ($CDCl_3$-$CD_3OD$) 4.33 (2H, s, C$\underline{H}_2$OH), 5.00 and 5.08 (4H, 2s, PhC$\underline{H}_2$O and PhC$\underline{H}_2$N), 6.40 (1H, s, 3-H), and 6.9-7.3 (11H, m, 2 × $C_6H_5$ and 6-H).

The mother liquor was chromatographed on silica (25g) eluting with 0 to 6% methanol in dichloromethane to give the desired product (1.83g, 57%) m.p. 84-5°C (Found: C, 74.5; H, 5.9; N, 4.3. $C_{20}H_{19}NO_3$ requires C, 74.7; H, 6.0; N, 4.4%), $\nu_{max}$ (KBr) 2400-3400 (broad), 1594, 1566, 1508, 1498, and 1452cm$^{-1}$; $\delta_H$ ($CDCl_3$) 4.55 (2H, s, C$\underline{H}_2$OH), 5.08 (4H, s, 2 × PhC$\underline{H}_2$O), 6.88 (1H, s, 3-H), 7.32 (10H, m, 2 × $C_6H_5$), and 7.98 (1H, s, 6-H).

(b) N-(4,5-Dibenzyloxypyridin-2-ylmethyl)piperazine-2,3-dione

Thionyl chloride (4.5ml, 10 eq) was added to a solution of 4,5-dibenzyloxy-2-hydroxymethylpyridine (1.0g) in dichloromethane (50ml). The solution was refluxed for lh then cooled and evaporated to dryness to give 4,5-dibenzyloxy-2-chloromethylpyridine hydrochloride (1.15g, 100%); $\nu_{max}$ ($CH_2Cl_2$) 2250, 1960, 1867, 1608, 1542, 1482, and 1450cm$^{-1}$; $\delta_H$ ($CDCl_3$) 5.03 and 5.16 (4H, 2s, 2 × PhC$\underline{H}_2$), 5.42 (2H, s, $CH_2Cl$), 7.35 (10H, m, 2 × $C_6H_5$), 7.62 (1H, s, 3-H), and 8.11 (1H, s, 6-$\underline{H}$). The hydrochloride, (1.15g) was redissolved in dichloromethane (100ml) and treated with ethylene diamine (30ml). After refluxing for 2.5h the reaction mixture was treated with sodium hydroxide (0.4g) in water (20ml). The organic layer was separated, washed with brine and dried ($MgSO_4$) then evaporated to dryness (1.05g, 93%), $\delta_H$ ($CDCl_3$) 2.42 (4H, m, $NCH_2CH_2$), 2.70 (3H, br, NH and $NH_2$), 3.74 (2H, s, C$\underline{H}_2$NH), 5.15 (4H, 2s, 2 × PhC$\underline{H}_2$), 6.88 (1H, s, 3-H), 7.35 (10H, m, 2 × $C_6H_5$), and 8.03 (1H, s, 6-H). Diethyl oxalate (0.8ml, 1.5 eq) was added to a solution of ethylene diamine derivative (1.05g) in ethanol (20ml) and the mixture heated at 80°C for 5h. After cooling the product was filtered off and washed with diethyl ether to give the title compound (0.69g, 57%) m.p. 229-230°C (Found: C, 68.4; H, 5.6; N, 9.9. $C_{24}H_{23}N_3O_4$ requires C, 69.0; H, 5.6; N, 10.1%), $\nu_{max}$ (nujol) 3205, 1709, 1680, and 1662cm$^{-1}$; $\delta_H$ [$(CD_3)_2SO$] 3.29 and 3.45 (4H, 2m, $NCH_2CH_2N$), 4.56 (2H, s, $NCH_2$), 5.16 and 5.23 (4H, 2s, 2 × PhC$\underline{H}_2$), 7.05 (1H, s, 3-H), 7.3-7.5 (10H, m, 2 × $C_6H_5$), 8.15 (1H, s, 6-H), and 8.0 (1H, br, NH); $\underline{m/z}$ 417 ($\underline{M}^+$, 3%), 326 (13), 305 (3), 237 (4), 199 (3) and 91 (100).

(c) N-[4,5-Dibenzyloxypyridin-2-ylmethyl]-2,3-dioxopiperazine N′-carbonyl chloride hydrochloride

Trimethylsilylchloride (0.56ml, 4.4 eq) was added to a suspension of N-(4,5-dibenzyloxypyridin-2-ylmethyl)-piperazine-2,3-dione (0.417g) in dichloromethane (20ml). After stirring for 0.25h triethylamine (0.31ml, 2.2 eq) was added and the reaction stirred for a further 1.5h. The solution was treated dropwise with condensed phosgene (excess) for 10 min. and stirred for 1h. The precipitated product was filtered off, washed with dichloromethane and dried to yield (0.408g, 79%) m.p. >250°C, $\nu_{max}$ (nujol) 2500 (broad), 1960, 1795, 1711, 1688, 1615 and 1438cm$^{-1}$

(d) Benzyl 6β-[D-2-[[4-(4,5-Dibenzyloxypyridin-2-ylmethyl)-2,3-dioxopiperazin-1-yl]carbonylamino]-2-phenylacetamido]-6α-methylthiopenicillanate

N-[4,5-Dibenzyloxypyridin-2-ylmethyl]-2,3-dioxopiperazine N′-carbonyl chloride hydrochloride (0.258g) was added to a suspension of benzyl 6β-(D-2-amino-2-phenylacetamido)-6α-methylthiopenicillanate (0.292g) and triethylamine (0.104ml) in THF (25ml). After stirring at room temperature for 1h, the reaction mixture

was filtered and the filtrate diluted with ethyl acetate. The solution was washed with brine, dried ($MgSO_4$) and evaporated to an oil which was chromatographed on silica (5g), eluting with 0 to 6% methanol in dichloromethane. Pure fractions were combined and evaporated to yield a colourless oil (0.4g, 86%), $\nu_{max}$ ($CH_2Cl_2$) 3400, 3300, 1782, 1746, 1718, 1693, 1589, 1510, and 1498cm$^{-1}$; $\delta_H$ ($CDCl_3$) 0.93 and 1.20 (6H, 2s, 2-C$\underline{Me}_2$), 2.22 (3H, s, $SCH_3$), 3.47 and 3.73 (4H, 2m, $NCH_2CH_2N$), 4.20 (1H, s, 3-H), 4.50 (2H, brs, $NCH_2$), 5.05, 5.07, and 5.10 (6H, 3s, 3 × $PhCH_2$), 5.42 (1H, s, 5-H), 5.60 (1H, d, PhC$\underline{H}$), 6.78 (1H, s, pyridyl 3-H), 7.24 (16H, m, 3 × $C_6H_5$ + NHCO), 7.90 (1H, s, pyridyl 6-H), and 9.91 (1H, d, NHCO); m/z (F.A.B.) 951 ($\underline{M}Na^+$, 73%), 929 ($\underline{M}H^+$, 6), 879 (7), 534 (78), 462 (55), and 440 (100).

(e) Benzyl 6β-[D-2-[[4-(4,5-Dibenzyloxypyridin-2-ylmethyl)-2,3-dioxopiperazin-1-yl]carbonylamino]-2-phenylacetamido]-6α-formamidopenicillanate

A solution of benzyl 6β-[D-2-[[4-(4,5-dibenzyloxypyridin-2-ylmethyl)-2,3-dioxopiperazin-1-yl]-carbonylamino]-2-phenylacetamido]-6α-methylthiopenicillanate (0.4g) in DMF (15ml) at 0°C under argon was treated with mercuric acetate (0.151g, 1.1 eq) followed by ammonia (0.011g, 1.1 eq) in DMF (0.5ml). The reaction was allowed to warm to room temperature over 1h then diluted with ethyl acetate (80ml), filtered and the filtrate washed with water (4 × 20ml) and brine before drying ($MgSO_4$) and evaporating to dryness. A solution of this 6α-amino derivative in dichloromethane (20ml) at 0°C was treated with pyridine (0.35ml, 10 eq) followed by acetic formic anydride (0.19g, 5 eq). The reaction was stirred at 0°C for 0.5h, room temperature for 1h and then diluted with dichloromethane. The solution was washed with 0.5M hydrochloric acid, dilute aqueous sodium hydrogen carbonate and then dried ($MgSO_4$). The solvent was removed under reduced pressure and the residual oil was chromatographed on silica (6g) eluting with 0 to 6% methanol in dichloromethane to give the title compound (0.25g, 63%), $\nu_{max}$ ($CH_2Cl_2$) 3400, 3295, 1785, 1744, 1718, 1692, 1588, 1509, 1461, and 1455cm$^{-1}$; $\delta_H$ ($CDCl_3$) 0.90 and 1.18 (6H, 2s, 2-$CMe_2$), 3.4-4.0 (4H, m, $NCH_2CH_2N$), 4.40 (1H, s, 3-H), 4.65 (2H, m, $NCH_2$), 5.15 (6H, m, 3 × PhC$\underline{H}_2$), 5.50 (1H, d, PhC$\underline{H}$), 5.56 (1H, s, 5-H), 7.04 (1H, s, pyridyl 3-H), 7.15-7.55 (15H, m, 3 × $C_6H_5$), 5.71 and 7.83 (2H, 2brs, 2 × CON$\underline{H}$), 8.03 and 8.12 (2H, 2s, NHC$\underline{H}$O and pyridyl 6-H), and 10.05 (1H, d, PhCHN$\underline{H}$); m/z (F.A.B.) 948 ($\underline{M}Na^+$, 29%), 531 (100), 462 (82), and 440 (90).

(f) Sodium 6β-[D-2-[[4-(4,5-Dihydroxypyrdin-2-ylmethyl)-2,3-dioxopiperazin-1-yl]carbonylamino]-2-phenylacetamido]-6α-formamidopenicillanate

Benzyl 6β-[D-2-[[4-(4,5-dibenzyloxypyridin-2-ylmethyl)-2,3-dioxopiperazin-1-yl]carbonylamino]-2-phenylacetamido]-6α-formamidopenicillanate (0.125g) in THF (20ml) with 10% palladium on charcoal (0.125g) and palladium black (0.125g) was shaken in an atmosphere of hydrogen for 6h. The catalysts were filtered off through Celite and the residues washed with 50% methanol in THF. The filtrate and washings were evaporated to dryness (0.055g) and the residue redissolved in 50% methanol in THF (20ml). The solution was treated with 0.084M sodium hydrogen carbonate (1ml, 1 eq). After diluting the solution with water (20ml), the organic solvent was removed under reduced pressure and tee aqueous fraction freeze-dried to give the title compound (0.055g, 60%), $\nu_{max}$ (KBr) 3281, 1770, 1682, 1606, 1508, and 1395cm$^{-1}$; $\delta_H$ ($D_2O$) 0.84 and 1.25 (6H, 2s, 2-$CMe_2$), 3.69 and 4.01 (4H, 2m, $NCH_2CH_2N$), 4.17 (1H, s, 3-H), 4.66 (2H, s, $NCH_2$), 5.47 (1H, s, 5-H), 5.56 (1H, s, PhC$\underline{H}$), 6.53 (1H, s, pyridyl 3-H), 7.37-7.60 (5H, m, $C_6H_5$), and 8.11 (1H, s, NHC$\underline{H}$O); m/z (F.A.B.) 678 ($\underline{M}H^+$, 14%), 656 (12), 490 (6), and 237 (100).

Example 7

Sodium 6β-[D-2-[[4-(3,4-dihydroxybenzyl)-2,3-dioxopiperazin-1-yl]carbonylamino]-2-phenylacetamido]-6α-(hydroxymethyl)penicillanate

(a) D-2-[[4-(3,4-Dibenzyloxybenzyl)-2,3-dioxopiperazin-1-yl)carbonylamino]-2-phenylacetic acid

D-Phenylglycine (0.151g; 1.0 mmol) was suspended in 1,1,1,3,3,3-hexamethyldisilazane (5ml) with chlorotrimethylsilane (0.1ml) and was heated at reflux under argon for 1h, after which time complete solution was attained. Excess reagent was removed by evaporation under reduced pressure and the residue was dissolved in dry tetrahydrofuran (10ml). The solution was stirred at 0°C and was treated with a filtered solution of [4-(3,4-dibenzyloxybenzyl)-2,3-dioxopiperazin-1-yl] carbonyl chloride in dry tetrahydrofuran (10ml) [prepared from 1-(3,4-dibenzyloxybenzyl)-2,3-dioxopiperazine (0.416mg; 1.0mmol) as described in Example 1]. The mixture was allowed to warm to room temperature and stirring was continued for 18h. After this time the reaction mixture was evaporated to dryness under reduced pressure. The residue was dissolved in ethyl acetate and was washed with dilute hydrochloric acid (2 × 25ml) and saturated brine (25ml), before being dried ($MgSO_4$). Evaporation to dryness under reduced pressure gave the title compound as a white foam (0.393g, 66%); $\nu_{max}$ (KBr) 3289, 3032, 1740 (sh), 1715, 1688, and 1510cm$^{-1}$; $\delta_H$ [250MHz; $(CD_3)_2CO$] 3.45 (2H, m, piperazine $CH_2$), 3.89 (2H, m, piperazine $CH_2$), 4.58 (2H, s, $NCH_2Ar$), 5.16 and 5.17 (4H, 2s, 2 × $.OCH_2Ph$), 5.51 (1H, d, J 7Hz, NHCHPh), 6.90 (1H, dd, J 8Hz and 2Hz, Ar 6-H), 7.04 (2H, m, Ar-H), 7.16-7.67 (15H, m, 3 × $C_6H_5$) and 9.97 (1H, d, J 7Hz, CONHCH); m/z (positive xenon F.A.B.; 3-nitrobenzylalcohol - NaOAc) $MNa^+$ 616.

b) Benzyl 6β-[D-2-[[4-(3,4-dibenzyloxybenzyl)-2,3-dioxopiperazin-1-yl]carbonylamino]-2-phenylacetamido]-6α-(hydroxymethyl)penicillanate

D-2-[[4-(3,4-Dibenzyloxybenzyl)-2,3-dioxopiperazin-1-yl]carbonylamino]-2-phenylacetic acid (0.297g; 0.5mmol) in dichloromethane (10ml) containing N,N-dimethylformamide (1 drop) was converted to the acid chloride by treatment with oxalyl chloride (0.08ml; 1mmol). After stirring at room temperature for 1.5h the solution was evaporated to dryness under reduced pressure, treated with dichloromethane, and re-evaporated. The residue [$\nu_{max}$ ($CH_2Cl_2$) 1800cm$^{-1}$] was dissolved in dichloromethane (10ml) and added dropwise to a solution of benzyl 6β-amino-6α-(hydroxy methyl)penicillanate (0.168g; 0.5mmol) and pyridine (0.06ml; 0.75mmol) in dichloromthane (10ml) at 0°C. The reaction mixture was stirred at 0-5°C for 0.5h, followed by 2h at room temperature before being evaporated to dryness under reduced pressure. The residue was dissolved in ethyl acetate and was washed with dilute hydrochloric acid (3 × 20ml), dilute aqueous sodium hydrogen carbonate (3 × 20ml) and saturated brine (20ml) before being dried ($MgSO_4$), and evaporated to dryness under reduced pressure to yield the crude product. Chromatography on silica gel 60 (<230 mesh ASTM) with ethyl acetate/hexane 4:1 as eluant gave the title compound as a white foam (0.049g; 11%); $\nu_{max}$ ($CH_2Cl_2$) 3280, 1780, 1750, 1720, 1695, and 1510 cm$^{-1}$; $\delta_H$(250MHz, $CDCl_3$) 1.07 and 1.21 (6H, 2s, 2-$C(CH_3)_2$), 3.01-3.30 (2H, m, piperazine $CH_2$), 3.49 (1H, m, piperazine CH), 3.65 (1H, m, piperazine CH), 4.02 (1H, m, exch. on $D_2O$, $CH_2OH$), 4.08-4.37 (2H, m, ABq on $D_2O$ J 12Hz, $CH_2OH$), 4.38 (1H, s, 3-H), 4.45 and 4.54 (2H, ABq, J 14Hz, $NCH_2Ar$), 5.06-5.22 (6H, m, 2 × $OCH_2Ph$, $CO_2CH_2Ph$), 5.41 (1H, d, J 6Hz, NHCHPh), 5.48 (1H, s, 5-H), 6.69 (1H, d, J 2Hz, Ar-H), 6.74 (1H, dd, J 8Hz and 1Hz, Ar-H), 6.84 (1H, d, J 8Hz, Ar-H), 7.00-7.59 (20H, m, 4 × $C_6H_5$), 7.62 (1H, s, NHCO) and 9.96 (1H, d, J 6Hz, NHCH); m/z (positive xenon F.A.B.; 3-nitrobenzyl-alcohol-NaOAc) $MNa^+$ 935.

(c) Sodium 6β-[D-2-[[4-(3,4-dihydroxybenzyl)-2,3-dioxopiperazin-1-yl)carbonylamino]-2-phenylacetamido]-6α-(hydroxymethyl)penicillanate

Benzyl 6β-[D-2-[[4-(3,4-dibenzyloxybenzyl)-2,3-dioxopiperazin-1-yl]carbonylamino]-2-phenylacetamido]-6α-(hydroxymethyl)penicillanate (0.037g; 0.05mmol) was dissolved in dry tetrahydrofuran (10ml) and hydrogenolysed at room temperature and pressure for 3h over 10% palladium on carbon (0.130g). The catalyst was removed by filtration through "Celite" and the filtrate was evaporated to dryness under reduced pressure. The residue was suspended in water and the pH was adjusted to 6.5 with dilute aqueous sodium hydrogen carbonate. The resultant aqueous solution was washed with ethyl acetate and freeze dried to yield the title compound as a white solid (0.014g, 42%); $\nu_{max}$ (KBr) 3301, 1763, 1714, 1676, 1605, and 1509cm$^{-1}$; $\delta_H$ (250MHz, $D_2O$) 1.06 and 1.33 (6H, 2s, 2-$C(CH_3)_2$), 2.79 (2H, m, piperazine $CH_2$), 3.83-4.02 (2H, m, piperazine $CH_2$), 4.01 and 4.13 (2H, ABq, J 12Hz, $CH_2OH$), 4.16 (1H, s, 3-H), 4.57 (2H, s, $NCH_2Ar$), 5.48 and 5.49 (2H, 2s, 5-H and NHCH), 6.76 (1H, dd, J 8Hz and 2Hz, Ar 6-H), 6.85 (1H, d, J 2Hz, Ar 1-H), 6.89 (1H, d, J 8Hz, Ar 5-H) and 7.39-7.62 (5H, m, $C_6H_5$); m/z (positive xenon F.A.B.; thioglycerol) $MH^+$ 664.

Example 8

Sodium 7β-[D-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate

(a) 1-(3,4-Dihydroxybenzyl)-2,3-dioxopiperazine

1-(3,4-Dibenzyloxybenzyl)-2,3-dioxopiperazine (Example 1a) (1.0g) was dissolved in a mixture of methanol (50ml) and tetrahydrofuran (50ml) and hydrogenated at standard temperature and pressure over 10% palladium on carbon (1.0g). The catalyst was removed by filtration through 'Celite', the filter bed washed with methanol and tetrahydrofuran (1:1, 2 × 25ml) and the filtrate evaporated to dryness under reduced pressure. The resulting foam was triturated under diethyl ether and the resulting, white solid (467mg; 82%) collected by filtration, m.p. 248 °C (dec.); $\nu_{max}$ (KBr) 3460, 3271, 1707, 1678 and 1653cm$^{-1}$; $\delta_H$ [90MHz; $(CD_3)_2SO$ + $D_2O$] 3.34 (4H, s, $N(CH_2)_2N$), 4.60 (2H, s, $NCH_2Ar$), 6.46-6.79 (3H, m, Ar) (Found: m/z 236.0788. $M^+$, $C_{11}H_{12}N_2O_4$ requires 236.0796).

(b) 1-(3,4-Diacetoxybenzyl)-2,3-dioxopiperazine

1-(3,4-Dihydroxybenzyl)-2,3-dioxopiperazine (740mg) was suspended in dry dichlorometane (20ml) and treated with pyridine (2 drops), 4-(N,N-dimethylamino)pyridine (2mg) and acetic anhydride (704mg). After 18h, the reaction mixture was concentrated to low volume under reduced pressure. Ethanol was added and a white solid collected by filtration, washed with a little ethanol and diethyl ether to give the product (838mg; 84%), m.p. 148-149 °C (Found: C, 56.21; H, 5.09; N, 8.73. $C_{15}H_{16}N_2O_6$ requires C, 56.25; H, 5.00; N, 8.75%); $\nu_{max}$ (KBr) 3241, 1769, 1700, 1670, 1502, 1424, 1368, 1260, 1210, 1183 and 1112cm$^{-1}$; $\delta_H$ [250MHz; $(CD_3)_2SO$] 2.27 (6H, s, 2 × $CH_3CO_2$), 3.30-3.41 and 3.41-3.53 (4H, 2m, $N(CH_2)_2N$), 4.60 (2H, s, $CH_2Ar$), 7.16-7.29 (3H, m, Ar), 8.60 (1H, bs, exchangeable, NH) (Found: m/z 320.1018. $M^+$, $C_{15}H_{16}N_2O_6$ requires 320.1007).

(c) D-2-[[4-[3,4-Diacetoxybenzyl)-2,3-dioxopiperazin-1-yl]carbonylamino]-2-phenylacetic acid

1-(3,4-Diacetoxybenzyl)-2,3-dioxopiperazine (481mg) in dry dichloromethane (20ml) was treated at room temperature under argon with chlorotrimethylsilane (326mg) and, after a further 15 minutes, with triethylamine (152mg). After 1.5h, excess phosgene was condensed into the reaction mixture and the mixture stirred for a further 2.5h. Evaporation to dryness under reduced pressure gave a mixture of triethylammonium chloride and 4-(3,4-diacetoxybenzyl)-2,3-dioxopiperazin-1-yl carbonyl chloride [$\nu_{max}$ ($CH_2Cl_2$) 1790, 1720 and 1690cm$^{-1}$]. The mixture was suspended in dry tetrahydrofuran (10ml) and used in the next stage.

D-phenylglycine (227mg) was suspended in a mixture of N,N-hexamethyldisilazane (10ml) and chlorotrimethylsilane (1ml) and the mixture refluxed until a clear solution was obtained. Evaporation to dryness under reduced pressure gave an oily residue, which was suspended in dry tetrahydrofuran (10ml). This suspension was treated at room temperature with the carbonyl chloride suspension and the mixture stirred 1h. Ethyl acetate (50ml) and water (20ml) were added, the mixture vigorously agitated and acidified to pH 2 with 5M hydrochloric acid. After 15 min, the phases were separated, the aqueous phase further extracted with ethyl acetate (20ml), the organic extracts combined, washed with water at pH 2 (20ml), water (20ml), saturated brine (20ml), dried ($MgSO_4$) and evaporated to dryness under reduced pressure to give the title compound (387mg; 52%) as a foam; $\nu_{max}$ ($CH_2Cl_2$) 1765, 1715, 1685, 1203 and 1180cm$^{-1}$; $\delta_H$ (60MHz; $CDCl_3$) 2.20 (6H, s, 2 × $CH_3CO_2$), 3.10-3.50 and 3.60-3.90 (4H, 2m, $N(CH_2)_2N$), 4.50 (2H, bs, $NCH_2Ar$), 5.35 (1H, d, J 7Hz, 2-H), 7.00-7.45 (8H, m, aromatics) 7.92 (1H, bs, exchangeable, $CO_2H$), 9.73 (1H, d, J 7Hz, exchangeable, NH); m/z (F.A.B.) 498 ($MH^+$, 20%), 454 (34), 321 (34), 165 (53) and 123 (100).

(d) Benzhydryl 7β-[D-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate

Benzhydryl 7β-amino-7α-formamido-3-[(1,3,4-thiadiazol-2-yl)thiomethyl]ceph-3-em-4-carboxylate (217mg) and N,N'-dicyclohexylcarbodiimide (91mg) were dissolved in minimum tetrahydrofuran and the resulting solution treated dropwise at ambient temperature with a solution of D-2-[[4-(3,4-diacetoxybenzyl)-2,3-dioxopiperazin-1-yl]carbonylamino]-2-phenylacetic acid (200mg) in tetrahydrofuran (minimum). After 60h, the reaction mixture was diluted with ethyl acetate (50ml) filtered and washed with water (25ml), saturated aqueous sodium hydrogen carbonate (25ml), water (25ml), saturated brine (25ml), dried ($MgSO_4$) and evaporated to dryness under reduced pressure. Careful chromatography through silica (<230 mesh ASTM), eluting with 80% ethyl acetate in cyclohexane (200ml) then 90% ethyl acetate in cyclohexane, gave the title compound (164mg; 40%), $\nu_{max}$ ($CH_2Cl_2$) 3300 br, 1790, 1730, 1700, 1500, 1380, 1270 and 1195cm$^{-1}$; $\delta_H$ [250MHz; $(CD_3)_2CO$] 2.25 and 2.26 (6H, 2s, 2 × $CH_3CO_2$), 3.12 and 3.36 (2H, ABq, J 16Hz, 2-$CH_2$), 3.63-3.68 and 4.03-4.08 (4H, 2m, $N(CH_2)_2N$), 4.21 and 4.85 (2H, ABq, J 14Hz, $CH_2$SHet), 4.71 (2H, s, $CH_2$Ar), 5.29 (1H, s, 6-H), 5.73 (1H, d, J 7Hz, 2'-H), 6.94 (1H, s, $CHPh_2$), 7.20-7.68 (18H, m, 3 × $C_6H_5$ and $C_6H_3$), 8.28 (1H, s, CHO), 8.54 and 8.74 (2H, 2s, exchangeable, 2 × CONH), 9.28 (1H, s, thiadiazole 5-H), 10.03 (1H, d, J 7Hz, exchangeable, 2'-NH); m/z (F.A.B.) 1019 ($MH^+$, 27%), 735 (14), 679 (12), 480 (17), 452 (100) and 424 (45).

(e) Sodium 7β-[D-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate

Benzhydryl 7β-[D-2-[[4-(3,4-diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3- em-4-carboxylate (114mg) was dissolved under argon in dry dichloromethane (10ml). The resulting solution was treated with anisole (363mg) then trifluoroacetic acid (383mg) at 0-5° C for 5 min, then stirred at room temperature for 15 min. The volatiles were removed under reduced pressure and the residue triturated under diethyl ether. The resulting off-white solid (71mg; 75%) was collected by filtration, washed with diethyl ether and suspended in water (25ml). The pH of the suspension was adjusted to 6.2 by addition of dilute aqueous sodium hydrogen carbonate solution and the mixture filtered. The filtrate was subjected to HP20 chromatography to give the title compound (21mg; 22%), after freeze-drying; $\nu_{max}$ (KBr) 3297, 1768, 1710, 1682, 1624, 1502, 1396, 1368, 1260, 1209, 1182, 1112 and 1072cm$^{-1}$; $\delta_H$ (250MHz; $D_2O$) 2.33 (6H, s, 2 × $CH_3CO_2$), 3.01 and 3.41 (2H, ABq, J 17Hz, 2-$H_2$), 3.58-3.69 and 3.90-4.00 (4H, 2m, $N(CH_2)_2N$), 3.97 and 4.41 (2H, ABq, J 14Hz, 3-$CH_2S$), 4.67-4.72 (2H, m, $NCH_2C_6H_3$), 5.23 (1H, s, 6-H), 5.50 (1H, s, 2'-H), 7.22-7.58 (8H, m, $C_6H_5$ and $C_6H_3$), 8.01 (1H, s, CHO) and 9.37 (1H, s, thiadiazole 5-H); m/z (F.A.B.) 897 ($MNa^+$, 6%), 875 ($MH^+$, 11), 853 (21), 452 (23) and 343 (100).

## Example 9

### Sodium 6β-[D-2-[[4-(3,4-Dihydroxybenzyl)-2,3-dioxopiperazin-1-yl]carbonylamino]-2-(4-aminophenyl)acetamido]-6α-formamidopenicillanate

(a) 2-(2,2,2-Trichloroethoxycarbonylamino)-2-(4-benzyloxycarbonylaminophenyl)acetic acid

N-Benzyloxycarbonylaminobenzene (2.3g, 10mmol) and N-2,2,2-trichloroethoxycarbonyl-2-hydroxyglycine (2.7g, 10mmol) were added to a vigorously stirred mixture of concentrated hydrochloric acid and acetic acid (3ml; 17ml). After 0.25h the reaction mixture was partitioned between ethyl acetate (200ml) and water (200ml). The organic phase was washed with water (200ml), dried ($MgSO_4$) and evaporated to afford a brown oil which was azeotroped with chloroform to give a brown foam (5.0g; 100%). Chromatography on

silica, eluting with a dichloromethane-ethyl acetate gradient, afforded the product as a white foam (2.8g, 58%), $\nu_{max}$ (KBr) 3331, 2958, 1725, 1601, 1527, 1416, 1319, 1220, 1091 and 1047cm$^{-1}$; $\delta_H$ [90MHz, $(CD_3)_2CO$] 4.80 (2H, s, $CH_2CCl_3$), 5.15 (2H, s, $CH_2Ph$), 5.35 (1H, d, J 7Hz), $CHCO_2H$), 7.2-7.7 (10H, m, 9 × ArH, $NHCO_2CH_2CCl_3$) and 8.7 (1H, bs, PhNH), m/z (F.A.B.) 475 ($MH^+$, 15%).

(b) Benzyl 6β-[D,L-2-(2,2,2-Trichloroethoxycarbonylamino)-2-(4-benzyloxycarbonylaminophenyl) acetamido]-6α-formamidopenicillanate

A solution of benzyl 6β-amino-6α-formamidopenicillanate (1.39g, 4mmol) and dicyclohexylcarbodiimide (0.82g, 4mmol) in tetrahydrofuran (4ml) at 0°C was treated with a solution of 2-(2,2,2-trichloroethoxycarbonylamino)-2-(4-benzyloxycarbonylaminophenyl)acetic acid (1.90g, 4mmol) in THF (9ml). The reaction mixture was stirred at room temperature for 3h, then diluted with ethyl acetate (100ml), filtered through celite, washed with water (100ml), dried ($MgSO_4$) and evaporated to afford a white foam (3.6g). Chromatography on silica eluting with 0-4% methanol in dichloromethane afforded first the L-isomer (0.42g; 13%) then the D-isomer (0.49g; 15%) both as white foams. L-isomer $\nu_{max}$ ($CH_2Cl_2$) 3400, 3300, 2950, 1780, 1730, 1690, 1595, 1500 and 1310cm$^{-1}$; $\delta_H$ [250MHz; $(CD_3)_2CO$] 1.33 (3H, s, $CH_3$), 1.42 (3H, s, $CH_3$), 4.50 (1H, s, 3-H), 4.77 (1H, d, J 12.3Hz, $CHHCCl_3$), 4.85 (1H, d, J 12.3Hz, $CHHCCl_3$), 5.18 (2H, s, $CH_2Ph$), 5.23 (2H, s, $CH_2Ph$), 5.55 (1H, d, J 8.9Hz, CHNH), 5.63 (1H, s, 5-H), 7.3-7.6 (15H, m, 14ArH and $NHCO_2CH_2CCl_3$), 8.12 (1H, d, J. 1.0Hz, NHCHO), 8.30 (1H, bs, NH), 8.60 (1H, bs, NH) and 8.80 (1H, bs, NH); m/z (F.A.B.) 828 ($MNa^+$, 45%): D-isomer $\nu_{max}$ ($CH_2Cl_2$) 3400, 3300, 2950, 1780, 1735, 1690, 1600, 1590 and 1500cm$^{-1}$; δ - [250MHz; $(CD_3)_2CO$] 1.03 (3H, s, $CH_3$), 1.20 (3H, s, $CH_3$), 4.40 (1H, s, 3-H), 4.77 (2H, s, $CH_2CCl_3$), 5.17 (2H, s, $CH_2Ph$), 5.20 (2H, s, $CH_2Ph$), 5.46 (1H, d, J 7.8Hz, NHCH), 5.58 (1H, s, 5-H), 7.3-7.6 (15H, m, 14ArH and $NHCO_2CH_2CCl_3$), 8.14 (1H, bs, NH), 8.18 (1H, d, J 1.1Hz, NHCHO), 8.62 (1H, bs, NH), and 8.77 (1H, bs, NH), m/z (F.A.B.) 828 ($MNa^+$, 50%).

(c) Benzyl 6β-[D-2-Amino-2-(4-benzyloxycarbonylaminophenyl)acetamido]-6α-formamidopenicillanate

A solution of benzyl 6β-[D-2-(2,2,2-trichloroethoxycarbonylamino)-2-(4-benzyloxycarbonylaminophenyl)-acetamido]-6α-formamidopenicillanate (235mg, 0.29mmol) in tetrahydrofuran (15ml) was treated with 1M potassium dihydrogen phosphate (1.5ml, 1.5mmol) then water (5ml). Freshly acid-washed zinc (0.8g) was added with vigorous stirring. After 0.5h the reaction mixture was filtered, and the filtrate partitioned between ethyl acetate (100ml) and half-saturated brine (100ml). The organic phase was dried ($MgSO_4$) and evaporated affording the product as a white glass (170mg; 95%) which was immediately used in the next reaction, $\nu_{max}$ ($CH_2Cl_2$) 3400-3100, 1780, 1730, 1680, 1590, 1500 and 1315cm$^{-1}$; δ [250MHz; $(CD_3)_2CO$] 1.48 (3H, s, $CH_3$), 1.52 (3H, s, $CH_3$), 4.50 (1H, s, 3-H), 4.52 (1H, s, NHCH), 5.17 (2H, s, $CH_2Ph$), 5.20 (2H, s, $CH_2Ph$), 5.65 (1H, s, 5-H), 7.3-7.6 (14H, m, ArH), 8.20 (1H, d, J 1.0Hz, NHCHO), 8.30 (1H, bs, NH), 8.50 (1H, bs, NH), and 8.80 (1H, bs, NH); m/z (F.A.B.) 654 ($MNa^+$, 28%).

(d) Benzyl 6β-[D-2-[[4-(3,4-Dibenzyloxybenzyl)-2, 3-dioxopiperazin-1-yl]carbonylamino]-2-(4-benzyloxycarbonylaminophenyl)acetamido]-6α-formamidopenicillanate

A solution of benzyl 6β-[D-2-amino-2-(4-benzyloxycarbonylaminophenyl)acetamido]-6α-formamidopenicillanate (170mg, 0.27mmol) and pyridine (0.25ml, 240mg, 3mmol) in tetrahydrofuran (10ml) was treated with a solution of 4-(3,4-dibenzyloxybenzyl)-2,3-dioxopiperazine-1- carbonyl chloride (144mg, 0.3mmol) in tetrahydrofuran (5ml).

After 0.5h the reaction mixture was partitioned between ethyl acetate (200ml) and dilute aqueous hydrochloric acid (200ml). The organic phase was washed with dilute acid (200ml), half-saturated brine (2 × 200ml), dried ($MgSO_4$) and evaporated to afford a white foam (308mg; 100%). Chromatography, eluting with a 0-3% gradient of methanol in dichloromethane afforded the product as a colourless glass (135mg, 46%); $\nu_{max}$ ($CH_2Cl_2$) 1780, 1735, 1710, 1685, 1600, 1580, 1360 and 1315cm$^{-1}$; δ [250MHz; $(CD_3)_2CO$] 0.96 (3H, s, $CH_3$), 1.12 (3H, s, $CH_3$), 3.45 (2H, m, piperazine $CH_2$), 3.85 (2H, m, piperazine $CH_2$), 4.40 (1H, s, 3-H), 4.55 (1H, d, J 14.6Hz, NCHHAr), 4.62 (1H, d, J 14.6Hz, NCHHAr), 5.15 (8H, m, 4 × $CH_2Ph$), 5.59 (1H, s, 5-H), 5.67 (1H, d, J 7Hz, CHNH), 6.90 (1H, dd, J 8.2Hz, J 1.9Hz, ArH), 7.05 (1H, bs, ArH), 7.22 (1H, d, - J 8.2Hz, ArH), 7.30-7.70 (20H, m, 4 × Ph), 8.16 (1H, d, J 1.3Hz, NHCHO), 8.25 (1H, s, NH), 8.76 (1H, s, NH), 8.80 (1H, s, NH) and 10.03 (1H, d, J 7Hz, CHNH).

(e) Sodium 6β-[D-2-[(4-(3,4-Dihydroxybenzyl-2,3-dioxopiperazin-1-yl)carbonylamino]-2-(4-aminophenyl) acetamido]-6α-formamidopenicillanate

A solution of benzyl 6β-[D-2-[[4-(3,4-dibenzyloxybenzyl)-2,3-dioxopiperazin-1-yl]carbonylamino]-2-(4-benzyloxycarbonylaminophenyl)acetamido]-6α-formamidopenicillanate (50mg, 0.05mmol) in tetrahydrofuran-water (15ml-5ml) was hydrogenated over 10% palladium on charcoal (50mg) and palladium black (50mg) in the presence of sodium hydrogen carbonate (5mg, 0.06mmol) for 4.5h. The reaction mixture was filtered and the residue was washed with a solution of sodium hydrogen carbonate (3mg, 0.03mmol) in water (5ml). The filtrate and washings were combined and the tetrahydrofuran was removed on a rotary evaporator. The resulting solution was freeze-dried affording the product as a light grey powder (33mg, 95%), $\nu_{max}$ (KBr) 3377, 2967, 1768, 1710, 1676, 1611, 1516, 1460, 1397 and 1365cm$^{-1}$; $\delta_H$ (250MHz; $D_2O$) 0.89 (3H, s, $CH_3$), 1.27 (3H, s, $CH_3$), 3.45 (2H, m, piperazine $CH_2$), 3.90 (2H, m, piperazine, $CH_2$), 4.15 (1H, s, 3-H), 4.52 (2H, s, $NC\underline{H}_2Ar$), 5.33 (1H, s, 5-H), 5.57 (1H, s, C<u>H</u>NH), 6.7-6.9 (5H, m, ArH), 7.30 (2H, d, J 8.4Hz, ArH), and 8.10 (1H, s, NHC<u>H</u>O).

Example 10

Sodium 7β-[D-2-[[4-(3,4-Diacetoxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylate

a) Benzhydryl 7β-(D-2-t-Butoxycarbonylamino-2-phenylacetamido)-7α-formamido-3-[(1-methyl-1H-tetrazol-5-yl)thiomethyl]ceph-3-em-4-carboxylate

A solution of benzhydryl 7β-amino-7α-formamido-3-[(1- methyl-1H-tetrazol-5-yl)thiomethyl]ceph-3-em-4-carboxylate (640mg, 1.19mmole) and DCCI (319mg, 1.54mmole) in dichloromethane (3ml) was treated with D-N-t-butoxycarbonylphenylglycine (299mg, 1.19mmole) in dichloromethane (2ml). The resulting heterogeneous mixture was stirred for 144 hours. Fresh dichloromethane (50ml) was then added and the insoluble DCU removed by filtration. The resulting solution was then washed with 0.5M-hydrochloric acid (2 × 50ml), saturated sodium hydrogen carbonate (3 × 50ml) and brine (1 × 50ml) then dried ($MgSO_4$). Chromatography on silica gel using chloroform-methanol mixtures as eluant provided the title compound (508mg, 0.66mmole, 56%) as a white amorphous solid; $\nu_{max}$ (KBr) 1787 and 1688cm$^{-1}$; $\delta_H$ ($CD_3COCD_3$) 8.6-8.45 (2H, m, NHCHO and 6-NH), 8.29 (1H, d, NHCHO), 7.7-7.2 (15H, m, 3 × Ph), 6.92 (1H, s, C<u>H</u>Ph), 6.6 (1H, m, $NHCO_2$), 5.49 (1H, d, PhC<u>H</u>CO), 5.27 (1H, s, 6-H), 4.54-4.29 (2H, m, 3-$CH_2$), 3.93 (3H, s, N-$CH_3$), 3.36 (2H, ABq, 2-$H_2$), and 1.4 (9H, s, $Bu^t$); m/z F.A.B. (3-Nitrobenzyl alcohol/sodium) (+ve ion) 793 ($MNa^+$).

b) Sodium 7β-[D-2-[[4-(3,4-Diacetoxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylate

Benzhydryl 7β-(D-2-t-butoxycarbonylamino-2-phenylacetamido)-7α-formamido-3-[(1-methyl-1H-tetrazol-5-yl) thiomethyl]ceph-3-em-4-carboxylate (125mg) was dissolved in trifluoroacetic acid (5ml) at room temperature and the mixture stirred for 1h under Argon. The solution was evaporated to dryness under reduced pressure and the residue azeotroped (2×) with dichloromethane (5ml) then triturated under diethyl ether. The resulting solid was collected by filtration, washed with diethyl ether and dried quickly under reduced pressure to give 7β-(D-2-amino-2-phenylacetamido)-7α-formamido-3-[(1-methyl-1H-tetrazol-5-yl)-thiomethyl]ceph-3-em-4-carboxylic acid, trifluoroacetic acid salt (100mg, 100%). This was suspended in dry tetrahydrofuran (4ml), under Argon, and treated with N,O-bistrimethylsilylacetamide (0.2ml) and the mixture stirred at room temperature until a clear solution was obtained. This solution was treated with a suspension of triethylammonium chloride and 4-(3,4-diacetoxybenzyl)-2,3-dioxopiperazin-1-ylcarbonyl chloride [prepared for 1-(3,4-diacetoxybenzyl)-2,3-dioxopiperazine (51.5mg) as described in example 8c] in dry tetrahydrofuran (10ml). After 1h at room temperature, the reaction mixture was diluted with water (25ml) and ethyl acetate (25ml) and stirred vigorously for 15 min. The phases were separated, the aqueous phase further extracted with ethyl acetate (10ml), the extracts combined, washed with water (10ml), water at pH 2

(10ml), water (10ml), saturated brine (10ml), dried ($MgSO_4$) and evaporated to dryness under reduced pressure to give a glass. The free acid was dissolved in dilute aqueous sodium hydrogen carbonate and purified by chromatography on HP20 to give the title compound (19.8mg, 14%) as a white foam, after freeze-drying; $\nu_{max}$ (KBr) 3300, 1770, 1715, 1686, 1609, 1500, 1391, 1367, 1261, 1211, 1185, and 1113cm$^{-1}$; $\delta_H$ (250MHz; $D_2O$) 2.30 (6H, s, 2 × $CH_3CO_2$), 3.03 and 3.39 (2H, ABq, J 17Hz, 2-H2), 3.48-3.69 (2H, m, piperazine $CH_2$), 3.80-4.21 (7H, m, N-$CH_3$, piperazine $CH_2$ and 3-$CH_2$S), 4.66 (2H, m, NC$H_2$Ar), 5.22 (1H, s, 6-H), 5.47 (1H, s, 2'-H), 7.16-7.47 (8H, m, aromatics), and 8.10 (1H, s, CHO); m/z (F.A.B.) 873 ($MH^+$, 13%), 452 (22), 343 (100) and (34).

Example 11

Sodium 7β-[D-2-[[4-(3,4-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate

7β-[DL-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid (200mg) was suspended and stirred in water (10ml). Acetone (1ml) was added and the pH of the mixture adjusted to 8.0 with 2.5% aqueous sodium carbonate. Sodium sulphite (72mg) was added and the pH adjusted to 9.0 by addition of 0.5M-hydrochloric acid. Stirring was continued at pH 9.0, maintained by addition of 2.5% aqueous sodium carbonate solution when necessary, until the reaction was complete (hplc). The reaction mixture was immediately subjected to chromatography through HP20 and the product (22.7mg) obtained as a white powder after freeze-drying; $\nu_{max}$ (KBr) 3289 (br), 1771, 1710, 1677, 1609, 1512, 1396, 1365, 1277, 1202, 1117, and 1064cm$^{-1}$; $\delta_H$ (250MHz; $D_2O$) 2.79 and 3.37 (2H, ABq, J 17Hz, 2-$H_2$), 3.49-3.62 and 3.82-4.01 (4H, 2m, N($CH_2$)2N), 3.90 and 4.32 (2H, ABq, J 14Hz, 3-$CH_2$S), 4.47-4.58 (2H, bm, NC$H_2$Ar), 5.21 (1H, s, 6-H), 5.48 (1H, s, 2'-H), 6.73-6.86 (3H, m, $C_6H_3$), 7.24-7.56 (5H, m, $C_6H_5$), 8.09 (1H, s, CHO), 9.37 (1H, s, thiadiazole-5-H); m/z (F.A.B.) 813 ($MNa^+$, 4%) and 791 ($MH^+$, 10).

Example 12

Sodium 6β-[D-2-(3,4-Diacetoxyphenyl)-2-[(4-(3,4-dihydroxybenzyl)-2,3-dioxopiperazin-1-yl)carbonylamino] acetamido]-6α-(hydroxymethyl)penicillanate

a) Benzyl 6β-[D-2-(3,4-Diacetoxyphenyl)-2-[(2,2,2-trichloroethoxy)carbonylamino]acetamido]-6α-(hydroxymethyl)penicillanate

D-2-(3,4-Diacetoxyphenyl)-2-[(2,2,2-trichloroethoxy) carbonylamino]phenylacetic acid (0.423g, 1mmol) in dichloromethane (10ml) containing N,N-dimethylformamide (1 drop) was converted to the acid chloride by treatment with oxalyl chloride (0.16ml, 2mmol). After stirring at room temperature for 1h the solution was evaporated to dryness under reduced pressure, treated with dichloromethane, and re-evaporated. The residue [$\nu_{max}$ ($CH_2Cl_2$) 1800 (sh.) cm$^{-1}$] was dissolved in dichloromethane (10ml) and added dropwise to a solution of benzyl-6β-amino-6α-(hydroxymethyl)penicillanate (0.336g, 1mmol) and pyridine (0.12ml, 1.5mmol) in dichloromethane (10ml) at 0°C. The reaction mixture was stirred at 0-5°C for 0.5h followed by 2h at room temperature before being evaporated to dryness under reduced pressure. The residue was dissolved in ethyl acetate and was washed with dilute hydrochloric acid (3 × 20ml), dilute aqueous sodium hydrogen carbonate (3 × 20ml) and saturated brine (20ml) before being dried ($MgSO_4$), and evaporated to dryness under reduced pressure to yield the crude product. Chromatography on silica gel 60 (<230 mesh ASTM) with ethyl acetate/hexane 1:1 as eluant, followed by chromatography on silica gel 60 (<230 mesh ASTM) with 2% ethanol/chloroform gave the title compound as a white foam (0.111g, 15%); $\nu_{max}$ ($CH_2Cl_2$) 3420, 1770, 1750 (sh), 1690, and 1210cm$^{-1}$; $\delta_H$ (250MHz, $CDCl_3$) 1.08 and 1.26 (6H, 2s, 2-C($CH_3$)$_2$), 2.24 and 2.26 (6H, 2s, 2 × $CH_3CO_2$), 3.89-4.00 (1H, m, exch. on $D_2O$, $CH_2OH$), 4.10 and 4.26 (2H, d, ABq, J

12Hz, J 4Hz, ABq on $D_2O$ exch., $CH_2OH$), 4.40 (1H, s, 3-H), 4.62 and 4.76 (2H, ABq, J 12Hz, $CH_2CCl_3$), 5.14 and 5.20 (2H, ABq, J 12Hz, $CH_2Ph$), 5.30 (1H, d, J 7Hz, NHCH), 5.47 (1H, s, 5-H), 5.81 (1H, d, J 7Hz, CHNH), 7.15 (1H, d, J 8Hz, Ar-H), 7.24-7.50 (7H, m, Ar-H), 8.13 (1H, s, NH); m/z (positive xenon F.A.B.; 3-nitrobenzyl-alcohol-NaOAc) $MNa^+$ 782.

b) Benzyl 6β-[D-2-(3,4-Diacetoxyphenyl)-2-[[4-(3,4-dibenzyloxybenzyl)-2,3-dioxopiperazin-1-yl]carbonylamino]acetamido]-6α-(hydroxymethyl) penicillanate

Benzyl 6β-[D-2-(3,4-diacetoxyphenyl)-2-[(2,2,2-trichloroethoxy)carbonylamino]acetamido]-6α-(hydroxymethyl)penicillanate (0.079g, 0.1mmol) in tetrahydrofuran (10ml) was treated with 1M-potassium dihydrogen phosphate (2ml) and fresh, acid-washed zinc (0.2g). The reaction mixture was stirred at pH 4 for 0.75h before addition of more zinc (0.3g). After a further 1h more zinc (0.4g) was added, followed by a further quantity (0.6g) after an additional 1h. The reaction mixture was filtered and the filtrate evaporated to small volume and diluted with ethyl acetate. The mixture was washed with brine, dried ($MgSO_4$) and then evaporated to yield benzyl 6β-[D-2-amino-2-(3,4-diacetoxyphenyl)acetamido]-6α-(hydroxymethyl)-penicillanate [$\nu_{max}$ ($CH_2Cl_2$) 1780, 1750cm$^{-1}$]. This, in dichloromethane (5ml) with pyridine (0.02ml, 0.16mmol) was treated dropwise with a solution of [4-(3,4-(dibenzyloxy)benzyl)-2,3-dioxopiperazin-1-yl]-carbonyl chloride in dichloromethane (4ml) [prepared from 1-(3,4-(dibenzyloxy)benzyl)-2,3-dioxopiperazine (0.043g, 0.1mmol) as described in Example 1b]. The reaction mixture was stirred at room temperature for 0.5h before being evaporated to dryness under reduced pressure. The residue was dissolved in ethyl acetate and was washed with dilute hydrochloric acid (3 × 15ml), dilute aqueous sodium hydrogen carbonate (3 × 15ml) and saturated brine (15ml) before being dried ($MgSO_4$) and evaporated to dryness under reduced pressure to yield the crude product. Chromatography on silica gel 60 (<230 mesh ASTM) eluting with chloroform grading to 5% ethanol/chloroform afforded the title compound as a slightly impure white foam (0.043g, 41%); $\nu_{max}$ ($CH_2Cl_2$) 3300, 1780, 1760 (sh.), 1720, 1695, 1510, 1215cm$^{-1}$; $\delta_H$ (400MHz, $CDCl_3$) 1.07 and 1.25 (6H, 2s, $C(CH_3)_2$), 2.25 (6H, s, 2 × $CH_3CO_2$, 3.08-3.25 (2H, m, piperazine $CH_2$), 3.48-3.71 (2H, m, piperazine $CH_2$), 3.98-4.83 (2H, m, $CH_2OH$), 4.38 (1H, s, 3-H), 4.47 and 4.52 (2H, ABq, J 15Hz, $NCH_2Ar$), 5.10-5.21 (6H, m, 3 × $CH_2Ph$), 5.45 (1H, d, J 6Hz, NHCH), 5.45 (1H, s, 5-H), 6.70-6.87 (3H, m, Ar-H), 7.10-7.46 (18H, m, Ar-H), 7.99 (1H, s, CONH), 9.99 (1H, d, J 6Hz, NHCH); m/z (positive xenon F.A.B.; 3-nitrobenzyl-alcohol-NaOAc) $MNa^+$ 1050.

c) Sodium 6β-[D-2-(3,4-Diacetoxyphenyl)-2-[(4-(3,4-dihydroxybenzyl)-2,3-dioxopiperazin-1-yl)-carbonylamino]acetamido]-6α-(hydroxymethyl)penicillanate

Benzyl 6β-[D-2-(3,4-diacetoxyphenyl)-2-[[4-(3,4-dibenzyloxybenzyl)-2,3-dioxopiperazin-1-yl]-carbonylamino]acetamido]-6α-(hydroxymethyl)penicillanate (0.030g, 0.03mmol) was dissolved in dry tetrahydrofuran (10ml) and hydrogenolysed at room temperature and pressure for 2h over 10% palladium on carbon (0.130g). The catalyst was removed by filtration through "Celite" and the filtrate was evaporated to dryness under reduced pressure. The residue was suspended in water and the pH was adjusted to 6.5 with dilute aqueous sodium hydrogen carbonate. The resultant aqueous solution was washed with ethyl acetate and freeze dried to yield the title compound as a white solid (0.017g, 73%); $\nu_{max}$ (KBr) 3311, 1764, 1713 and 1676cm$^{-1}$; $\delta_H$ (250MHz, $D_2O$) 0.99 and 1.27 (6H, 2s, $C(CH_3)_2$), 2.28 and 2.31 (6H, 2s, 2 × $CH_3CO_2$), 3.39-3.60 (2H, m, piperazine $CH_2$), 3.75-3.95 (2H, m, piperazine $CH_2$), 3.95 and 4.07 (2H, ABq, J 12Hz, $CH_2OH$), 4.11 (1H, s, 3-H), 4.50 (2H, s, $NCH_2Ar$), 5.44 (2H, s, CHNH, 5-H), 6.70-6.90 (3H, m, Ar-H), 7.18-7.50 (3H, m, Ar-H), m/z (positive xenon F.A.B.; thioglycerol) $MH^+$ 780.

## Example 13

Sodium 7β-[2-[[4-(3,4-Dihydroxybenzyl)-2,3-dioxopiperazin-1-yl]carbonylamino]-2-(2-thienyl)acetamido]-7α-formamido-3-[(1,3,4-thiadiazol-2-yl)thiomethyl]ceph-3-em-4-carboxylate

a) DL-2-[[4-(3,4-Diacetoxybenzyl)-2,3-dioxopiperazin-1-yl]carbonylamino]-2-(2-thienyl)acetic acid

DL-2-(2-thienyl)glycine (314mg) was suspended in 1,1,1, 3,3,3-hexamethyldisilazane (10ml) containing chlorotrimethylsilane (1ml) under Argon and the mixture refluxed for 4h and then cooled and evaporated to dryness under reduced pressure. The residue was suspended under Argon in dry tetrahydrofuran (10ml) and treated with a suspension of 4-(3,4-diacetoxybenzyl)-2,3-dioxopiperazin-1-ylcarbonyl chloride [prepared as described in example 8c from 1-(3,4-diacetoxybenzyl)-2,3-dioxopiperazine (640mg), in tetrahydrofuran (10ml)]. The mixture was stirred at room temperature for 1h then diluted with ethyl acetate (25ml) and water (25ml) and stirred vigorously for 15 min. The phases were separated, the organic phase retained and washed with water (25ml), saturated brine (25ml), dried ($MgSO_4$) and evaporated to dryness under reduced pressure to give the title compound (948mg, 89%) as a brown foam; $\nu_{max}$ ($CH_2Cl_2$) 3270 br, 1765, 1710, 1685, 1500, 1360, 1210 and 1185cm$^{-1}$; $\delta_H$ (90MHz, $CDCl_3$) 2.23 (6H, s, 2 × $CH_3CO_2$), 3.27-3.54 and 3.82-4.07 (4H, 2m, $N(CH_2)_2N$), 4.58 (2H, bs, NC$\underline{H}_2$Ar), 5.70 (1H, d, J 7Hz, 2-H), 6.80-7.25 (6H, m, Ar and thienyl), 7.49 (1H, bs, exchangeable, $CO_2H$), 9.72 (1H, d, J 7Hz, exchangeable, NH); m/z (F.A.B.) 526 ($MNa^+$, 21%), 521 ($MNH_4^+$, 37), 504 ($MH^+$, 64), 343 (53), 321 (100), 279 (46), 232 (34) and 217 (72).

b) Benzhydryl 7β-[DL-2-[[4-(3,4-Diacetoxybenzyl)-2,3-dioxopiperazin-1-yl]carbonylamino]-2-(2-thienyl)-acetamido]-7α-formamido-3-[(1,3,4-thiadiazol-2-yl)thiomethyl]ceph-3-em-4-carboxylate

Benzhydryl 7β-amino-7α-formamido-3-[(1,3,4-thiadiazol-2-yl)thiomethyl]ceph-3-em-4-carboxylate (983mg) was dissolved in tetrahydrofuran (min.) and treated with N,N′-dicyclohexylcarbodiimide (412mg). The resulting solution was treated dropwise with a solution of DL-2-[[4-(3,4-diacetoxybenzyl)-2,3-dioxopiperazin-1-yl]carbonylamino]-2-(2-thienyl)acetic acid (917mg) in tetrahydrofuran (min.) at room temperature and the reaction mixture stirred at room temperature for 84h. The precipitate was removed by filtration and washed with ethyl acetate (50ml). The filtrate was washed with water (25ml), dilute aqueous sodium hydrogen carbonate, water, saturated brine and dried ($MgSO_4$) then evaporated to dryness under reduced pressure to give an orange/red foam. Chromatography through silica (<230 mesh ASTM), eluting with 70% ethyl acetate in cyclohexane, grading in 200ml portions and 10% steps, to 100% ethyl acetate gave a partially-purified foam, which was redissolved in ethyl acetate and diluted with diethyl ether. The resulting precipitate was collected by filtration, washed with diethyl ether and dried under reduced pressure (1.09g, 54%). The title compound was recrystallised from ethyl acetate-ethanol as a light-brown, amorphous solid, m.p. 146-152°C; $\nu_{max}$ (KBr) 1784, 1711, 1690, 1501, 1369, 1258, 1209 and 1184cm$^{-1}$; $\delta_H$ [250MHz, $(CD_3)_2CO$] 2.89 (6H, s, 2 × $CH_3CO_2$), 3.38-3.66 (4H, m, piperazine $CH_2$ and 2-$H_2$, isomers), 4.04-4.14 (2H, m, piperazine $CH_2$), 4.32-4.45 and 4.67-4.81 (4H, m, NC$\underline{H}_2$Ar and 3-$CH_2$, isomers), 5.32 and 5.33 (1H, 2s, 6-H, isomers), 5.97-6.12 (1H, m, 2′-H, isomers), 6.89-7.70 (17H, m, thienyl, 2 × $C_6H_5$, $C_6H_3$ and C$\underline{H}Ph_2$), 8.28 and 8.9 (1H, 2s, CHO, isomers), 8.61 and 8.64 (1H, 2 × bs, exchangeable, N$\underline{H}$CHO, isomers), 8.73 and 8.83 (1H, 2s, exchangeable, 7β-CONH, isomers), 9.28 (1H, s, thiadiazole 5-H), 9.94-10.03 (1H, m, exchangeable, 2′-NH, isomers); m/z (F.A.B.) 1047 ($MNa^+$, 19%), 727 (11), 462 (25), 365 (36) and 343 (100). The isomer ratio D:L appears by n.m.r. to be approximately 4:3.

c) Sodium 7β-[2-[[4-(3,4-Dihydroxybenzyl)2,3-dioxopiperazin-1-yl]carbonylamino]-2-(2-thienyl)acetamido]-7α-formamido-3-[(1,3,4-thiadiazol-2-yl)thiomethyl]ceph-3-em-4-carboxylate

Benzhydryl 7β-[2-[[4-(3,4-diacetoxybenzyl)-2,3-dioxopiperazin-1-yl]carbonylamino]-2-(2-thienyl)-acetamido]-7α-formamido-3-[(1,3,4-thiadiazol-2-yl)thiomethyl]ceph-3-em-4-carboxylate (500mg, D:L ca 4:3) was dissolved in dry dichloromethane (10ml) under Argon. Cooled to 0-5°C and treated with trifluoroacetic acid (1.6g). Cooling was removed immediately and the mixture stirred at room temperature for 0.5h. The volatiles were removed under reduced pressure and the residue azetotroped with toluene (3 × 5ml). Diethyl ether was added to the residue and the mass triturated to yield a brown solid, which was collected by filtration, washed with diethyl ether and dried under reduced pressure (414mg, 100%).

The above acid was suspended in water (10ml) and the pH adjusted to 8.0 with 2.5% aqueous sodium carbonate solution. Sodium sulphite (146mg) was added and the pH of the solution maintained at pH 9.0 until the reaction was complete (hplc). The reaction mixture was immediately chromatographed through HP20, eluting with water grading to 25% tetrahydrofuran in water in 2 steps and 100ml portions. The requisite fractions were combined, concentrated to low volume under reduced pressure and freeze-dried to give the title compound (40mg, 11%) as a white powder. The isomer ratio was determined by high

performance liquid chromatography and nuclear magnetic resonance spectroscopy to be ca. 2:1, D:L; $\nu_{max}$ (KBr) 3283 br, 1777, 1710, 1676, 1512, 1395, 1365, 1197, and 1116cm$^{-1}$; $\delta_H$ [250MHz; $(CD_3)_2SO$] 3.14-3.55 (4H, m, piperazine $CH_2$ and 2-$H_2$, isomers), 3.89 (2H, bs, piperazine $CH_2$), 4.16-4.34 and 4.57-4.68 (2H, 2m, 3-$CH_2$, isomers), 4.41 (2H, s, $CH_2Ar$), 5.10-5.23 (1H, m, 6-H, isomers), 5.82-6.04 (1H, m, 2′-H, isomers), 6.50-6.75 (3H, m, $C_6H_3$), 6.91-7.57 (3H, m, thienyl) 7.99-8.36 (1H, m, CHO, isomers), 8.97 (2H, bs, exchangeable, NHCHO and 7β-CONH), and 9.38-10.02 (2H, m, thiadiazole 5-H and 2′-NH, exchangeable).

## Example 14

### Sodium 7β-[D-2-[[4-(3,4-Dihydroxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylate

7β-[D-2-amino-2-phenylacetamido)-7α-formamido-3-[(1-methyl-1H-tetrazol-5-yl)thiomethyl]ceph-3-em-4-carboxylic acid trifluoroacetic acid salt (100mg) [prepared as described in Example 10b] was suspended in dry tetrahydrofuran (100ml) under Argon and treated with N,O-bistrimethylsilylacetamide (3 × 0.2ml) until a clear solution was obtained (ca. 0.5hr). The resulting, clear solution was treated with 4-(3,4-diacetoxybenzyl)-2,3-dioxopiperazin-1-ylcarbonyl chloride [prepared as described in example 8c from 1-(3,4- diacetoxybenzyl)-2,3-dioxopiperazine (51.5mg), in tetrahydrofuran (10ml)] and the mixture stirred at room temperature for 0.5h. Water (25ml) and ethyl acetate (50ml) were added and the mixture stirred vigorously for 15 min. The phases were separated, the organic phase washed with water (25ml), 0.5M-hydrochloric acid (25ml), water (25ml), saturated brine (25ml), dried ($MgSO_4$) and evaporated to dryness under reduced pressure. The resulting powder was suspended in water (10ml), the pH adjusted to 8.0 with 2.5% aqueous sodium carbonate and treated with sodium sulphite (50mg). The pH was adjusted to and maintained at 9.0 until de-acetylation was complete (ca. 1.5h) (hplc). At this point, the reaction mixture was chromatographed through HP20, eluting with water, grading to 25% tetrahydrofuran in water in 2% steps and 100ml portions. The product-containing fractions were combined, concentrated under reduced pressure and freeze-dried to give the title compound (42mg, 17%); $\nu_{max}$ (KBr) 1771, 1710, 1677, 1609, 1512, 1395, 1364, 1285, 1200, 1117 and 1003cm$^{-1}$; $\delta_H$ (250MHz, $D_2O$) 2.92-3.08 and 3.31-3.42 (2H, m, 2-$H_2$), 3.40-3.58 (2H, bm, piperazine $CH_2$); 3.78-4.17 (7H, m, 3-$CH_2$, piperazine $CH_2$ and N-$CH_3$), 4.43-4.57 (2H, bm, $NCH_2Ar$), 5.19 (1H, s, 6-H), 5.45 (1H, s, 2′-H), 6.61-6.87 (3H, m, $C_6H_3$), 7.30-7.53 (5H, m, $C_6H_5$) and 8.07 (1H, s, CHO); m/z (F.A.B.) 789 ($MH^+$, 9%), 239 (37), and 217 (100).

## Example 15

### Sodium 6β-[2-[[4-(2,3-dihydroxybenzyl)-2,3-dioxopiperazin-1-yl]carbonylamino]-2-phenylacetamido]-6α-formamidopenicillanate

#### a) 1-(2,3-Dibenzyloxybenzyl)-2,3-dioxopiperazine

Ethylenediamine (17g) in toluene (20ml) under Argon was treated with a solution of 2,3-dibenzyloxybenzyl chloride (1.92g) in toluene (5ml). The mixture was refluxed 4h, cooled to room temperature and treated with a solution of sodium hydroxide (1g) in water (20ml). The phases were separated, the organic phase washed with water (25ml), saturated brine and evaporated to dryness under reduced pressure. The residue was redissolved in ethanol (20ml), diethyl oxalate (1.65g) added and the mixture refluxed 4h. Concentrated under reduced pressure, diethyl ether added to the residue, the white solid collected by filtration and recystallised from ethanol (1.36g, 58%), m.p. 178-179°C (Found: C, 72.09; H, 5.75; N, 6.60%. $C_{25}H_{24}N_2O_4$ requires C, 72.11; H, 5.79; N, 6.73%); $\nu_{max}$ ($CHCl_3$) 1705, 1680, 1470, 1450, 1360, 1265, 1083 and 1060 cm$^{-1}$; $\delta_H$ (60MHz, $CDCl_3$) 3.49 (4H, bs, $N(CH_2)_2N$), 4.57 (2H, bs, $NCH_2Ar$), 5.01 and 5.10 (4H, 2s, 2 × $OCH_2Ph$), 6.72-7.02 (3H, m, $C_6H_3$), 7.16-7.50 (10H, m, 2 × $C_6H_5$), 7.68-8.07 (1H, bs, exchangeable, NH).

b) Benzyl 6β-[D-2-[[4-(2,3-Dibenzyloxybenzyl)-2,3-dioxopiperazin-1-yl]carbonylamino]-2-phenylacetamido]-6α-methylthiopenicillanate

1-(2,3-Dibenzyloxybenzyl)-2,3-dioxopiperazine (416mg) was dissolved in dry dichloromethane (10ml) under Argon and the solution treated with chlorotrimethylsilane. After 15 min, triethylamine (111mg) was added and the mixture stirred at room temperature for 1.5h. Excess phosgene was condensed into the reaction mixture, which was then stirred at room temperature for 3h. The volatiles were removed under reduced pressure to give an off-white solid; $\nu_{max}$ ($CH_2Cl_2$) 1800cm$^{-1}$. This solid was suspended in dry tetrahydrofuran (10ml) and the suspension added to a solution of benzyl 6β-(D-2-amino-2-phenylacetamido)-6α-formamidopenicillanate (485mg) and triethylamine (111mg) in dry tetrahydrofuran (10ml). After 0.5h, the reaction mixture was diluted with ethyl acetate (50ml) and washed with water (20ml), saturated brine (20ml), dried ($MgSO_4$) and evaporated to dryness under reduced pressure. Chromatography through silica (<230 mesh ASTM), eluting with 30% ethyl acetate in cyclohexane, gave the title compound (220mg, 24%) as a white foam; $\nu_{max}$ ($CH_2Cl_2$) 1770, 1730, 1705, 1685, 1200 and 1180cm$^{-1}$; $\delta_H$ [250MHz, $(CD_3)_2CO$] 1.02 and 1.21 (6H, 2s, 2-$(CH_3)_2$), 2.31 (3H, s, $SCH_3$), 3.39-3.57 and 3.89-4.08 (4H, 2m, $N(CH_2)_2N$), 4.40 (1H, s, 3-H), 4.66 (2H, bs, $NCH_2Ar$), 5.10-5.26 (6H, m, $CO_2CH_2Ph$ and 2 × $OCH_2Ph$), 5.45 (1H, s, 5-H), 5.70 (1H, d, J 7Hz, 2'-H), 6.92-7.18 (3H, m, $C_6H_3$), 7.27-7.62 (20H, m, 4 × $C_6H_5$), 8.75 (1H, s, exchangeable, 6β-CONH), 10.01 (1H, d, J 7Hz, exchangeable, 2'-NH); m/z (F.A.B.) 950 ($MNa^+$, 97%), 534 (55), 462 (75) and 439 (100).

c) Benzyl 6β-[D-2-[[4-(2,3-dibenzyloxybenzyl)-2,3-dioxopiperazin-1-yl]carbonylamino]-2-phenyl acetamido]-6α-formamidopenicillanate

Benzyl 6β-[D-2-[[4-(2,3-dibenzyloxybenzyl)-2,3-dioxopiperazin-1-yl]carbonylamino]-2-phenylacetamido]-6α-methylthiopenicillanate (200mg) was dissolved in dry N,N-dimethylformamide (5ml) under Argon at 0-5°C and treated with mercuric acetate (69mg) followed by a 10% excess of ammonia in dry N,N-dimethylformamide. Cooling was removed immediately and the reaction mixture diluted with ethyl acetate (50ml) after 15 min and filtered. The filtrate was washed with water (5 × 20ml), saturated brine (20ml), dried and evaporated to dryness under reduced pressure to give a foam. This foam was redissolved in dry dichloromethane (10ml) under Argon at 0-5°C and treated with pyridine (170mg) then acetic formic anhydride (95mg). Cooling was removed immediately and the mixture stirred at room temperature for 3h. The mixture was washed with water (10ml), saturated aqueous sodium hydrogen carbonate (10ml), water (10ml), saturated aqueous copper sulphate (10ml), water (10ml), saturated brine (10ml), then dried ($MgSO_4$) and evaporated to dryness under reduced pressure. Purification by chromatography through silica (<230 mesh ASTM), eluting with 50% ethyl acetate in cyclohexane, grading to 90% ethyl acetate in cyclohexane in 10% 'steps' and 100ml portions, gave the title compound (128mg, 64%) as a white foam; $\nu_{max}$ ($CH_2Cl_2$) 1785, 1740, 1720, 1690, 1500, 1205, 1185, 1090, 1065, and 1005cm$^{-1}$; $\delta_H$ [250MHz, $(CD_3)_2CO$] 0.91 and 1.16 (6H, 2s, 2-$(CH_3)_2$), 3.40-3.5 and 3.90-4.00 (4H, 2m, $N(CH_2)_2N$), 4.39 (1H, s, 3-H), 4.66 (2H, s, $NCH_2C_6H_3$), 5.07-5.16 (6H, m, 3 × $CH_2Ph$), 5.58 (1H, s, 5-H), 5.68 (1H, d, J 7Hz, 2'-H), 6.87-7.19 (3H, m, $C_6H_3$), 7.26-7.61 (20H, m, 4 × $C_6H_5$), 8.17 (1H, s, CHO), 8.24 (1H, bs, exchangeable, NHCHO), 8.77 (1H, s, exchangeable, 6β-CONH), 10.03 (1H, d, J 7Hz, exchangeable, 2'-NH); m/z (F.A.B.) 947 ($MNa^+$, 78%) and 531 (100).

d) Sodium 6β-[D-2-[[4-(2,3-dihydroxybenzyl)-2,3-dioxopiperazin-1-yl]carbonylamino]-2-phenylacetamido]-6α-formamidopenicillanate

Benzyl 6β-[D-2-[[4-(2,3-dibenzyloxybenzyl)-2,3-dioxopiperazin-1-yl]carbonylamino]-2-phenylacetamido]-6α-formamidopenicillanate (120mg) was dissolved in redistilled, dry tetrahydrofuran (minimum) and hydrogenated over a mixture of 10% palladium on carbon (120mg) and palladium black (120mg) for 3h. The catalyst was removed by filtration through 'Celite' and the process repeated with fresh catalyst. After a further 2.5h, the catalyst was removed by filtration through 'Celite' and the filtrate evaporated to dryness under reduced pressure. The residue was dissolved in AR acetone (minimum) and treated with 1.93M-sodium 2-ethylhexanoate in methyl iso-butylketone (62µl). Diethyl ether was added and the title compound collected by filtration, washed with diethyl ether and dried under reduced pressure (52mg, 59%); $\nu_{max}$ (KBr) 1772, 1710, 1677, 1611, 1508, 1477, 1396, 1365, 1284 and 1200cm$^{-1}$; $\delta_H$ [250MHz, $(CD_3)_2SO$] 0.85 and

1.16 (6H, 2s, 2-$(CH_3)_2$), 3.76 (1H, s, 3-H), 3.80-4.00 (2H, m, piperazine $CH_2$), 4.51 (2H, bs, $CH_2C_6H_3$), 5.38 (1H, s, 5-H), 5.64 (1H, d, J 7Hz, 2'-H), 6.52-6.78 (3H, m, $C_6H_3$), 7.19-7.62 (5H, m, $C_6H_5$), 8.00 (1H, s, CHO), 9.07-9.23 (1H, bs, exchangeable, NHCHO), 9.72-10.04 (2H, m, exchangeable, 6β-CONH and 2'-NH); m/z - (F.A.B.) 699 ($MNa^+$, 27%), 677 ($MH^+$, 100), 463 (40) and 441 (100).

Example 16

Sodium 7β-[D-2-[[4-(2,3-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate

a) 1-(2,3-Dihydroxybenzyl)-2,3-dioxopiperazine

1-(2,3-Dibenzyloxybenzyl)-2,3-dioxopiperazine (3.2g) was dissolved in a 1:1 mixture of tetrahydrofuran (150ml) and methanol (150ml). 10% Palladium on carbon (1.0g) was added and the mixture hydrogenated for 0.5h before more palladium on carbon (0.5g) was added and hydrogenation continued for a further 1h. The catalyst was removed by filtration through 'Celite', the filter bed washed with 1:1 tetrahydrofuran/methanol and the filtrate evaporated to dryness under reduced pressure. Ethanol (20ml) was added to the residue and the product collected by filtration, washed with a little, cold ethanol and dried under reduced pressure (1.65g; 91%), m.p. 258°C; $\nu_{max}$ (Nujol) 3360, 3150, 1690 and 1650$cm^{-1}$; $\delta_H$ - [60MHz; $(CD_3)_2SO$] 3.39 (4H, bs, $N(CH_2)_2N$), 4.55 (2H, bs, $NCH_2Ar$), 6.66 (3H, m, $C_6H_3$, (Found: m/z 236.0797. $M^+$, $C_{11}H_{12}N_2O_4$ requires 236.0797).

b) 1-(2,3-Diacetoxybenzyl)-2,3-dioxopiperazine

1-(2,3-Dihydroxbenzyl)-2,3-dioxopiperazine (1.65g) was suspended in dry dichloromethane (25ml) and treated with 4-dimethylaminopyridine (10mg), pyridine (4 drops) and acetic anhydride (1.57g). After 18h at room temperature, the isolubles were removed by filtration and washed with dichloromethane. The filtrate produced more insoluble material, which was collected by filtration. The two crops were combined and recrystallised from ethanol (2.2g; 98%), m.p. 175°C; $\nu_{max}$ (Nujol) 3205, 1760, 1705, 1660, 1220 br, 1180 and 1160$cm^{-1}$; $\delta_H$ [90MHz; $(CD_3)_2SO$] 2.21 (6H, s, 2 × $CH_3CO_2$), 3.23 (4H, bs, $N(CH_2)_2N$), 4.53 (2H, s, $NCH_2Ar$), 7.10-7.35 (3H, m, $C_6H_3$), 8.48 (1H, bs, exchangeable, NH). (Found: m/z 320.1008. $M^+$, $C_{15}H_{16}N_2O_6$ requires 320.1008).

c) D-2-[[4-(2,3-Diacetoxybenzyl)-2,3-dioxopiperazin-1-yl]carbonylamino]-2-phenylacetic acid

D-Phenylglycine (378mg) was suspended in hexamethyldisilazane (10ml) containing chlorotrimethylsilane (1ml) and the mixture heated at reflux under argon until a clear solution had been obtained. The volatiles were removed under reduced pressure and the residue suspended in dry tetrahydrofuran (10ml) under argon. 1-(2,3-Diacetoxybenzyl)-2,3-dioxopiperazine (800mg) was suspended in dry dichloromethane (10ml) under argon and treated with chlorotrimethylsilane (298mg) then triethylamine (255mg). After 2h, the reaction mixture was treated with excess phosgene in tetrahydrofuran and stirred for a further 2h. The volatiles were then removed under reduced pressure to yield a solid, $\nu_{max}$ ($CH_2Cl_2$) 1790, 1770, 1730 and 1690$cm^{-1}$, which was suspended in dry tetrahydrofuran (10ml) and this suspension added to the persilylated amino acid. After 1h at room temperature, the reaction mixture was treated with water (20ml) and 5M hydrochloric acid to pH 1. It was stirred vigorously for 15 mins. then extracted well with ethyl acetate (50ml). The extracts were combined, washed with water (3 × 10ml), dilute hydrochloric acid, water (10ml) and saturated brine (10ml). Drying ($MgSO_4$) and evaporation to dryness under reduced pressure gave the title compound as a foam (895mg; 72%); $\nu_{max}$ (KBr) 1771, 1715, 1690, 1207 and 1164$cm^{-1}$; $\delta_H$ (90MHz; $CDCl_3$) 2.24 and 2.26 (6H, 2s, 2 × $CH_3CO_2$), 3.17-3.45 and 3.80-4.00 (4H, 2m, $N(CH_2)_2N$), 4.63 (2H, bs, $NCH_2Ar$), 5.45 (1H, d, J 6Hz, 2-H), 7.14-7.50 (8H, m, Ar), 9.78 (1H, d, J 6Hz, NH, exchangeable); m/z (F.A.B.) 520 ($MNa^+$, 23%), 475 (11), 462 (17), 413 (10) and 365 (42).

d) Benzhydryl 7β-[D-2-[[4-(2,3-Diacetoxybenzyl)-2,3-dioxopiperazin-1-yl]carbonylamino]-2-phenyl acetamido]-7α-formamido-3-[(1,3,4-thiadiazol-2-yl-) thiomethyl]ceph-3-em-4-carboxylate

Benzhydryl 7β-amino-7α-formamido-3-[(1,3,4-thiadiazol-2- yl)thiomethyl]ceph-3-em-4-carboxylate (269mg) and N,N′-dicyclohexylcarbodiimide (113mg) were dissolved in the minimum quantity of tetrahydrofuran and treated, dropwise, at room temperature with a solution of D-2-[[4-(2,3-diacetoxybenzyl)-2,3-dioxopiperazin-1- yl]carbonylamino]2-phenylacetic acid (248mg) in tetrahydrofuran. After 18h, the mixture was filtered and the filtrate evaporated to dryness under reduced pressure. The residue was purified by chromatography through silica (<230 mesh ASTM), eluting with ethyl acetate in cyclohexane in a gradient from 70% to 90% ethyl acetate, tc give the product (267mg, 53%), $\nu_{max}$ (KBr) 1775, 1715, 1690, 1495, 1466, 1369, 1258, 1207 and 1162cm$^{-1}$; $\delta_H$ [250MHz; $(CD_3)_2CO$] 2.28 and 2.32 (6H, 2s, 2 × $CH_3CO_2$), 3.31 and 3.17 (2H, ABq, J 20Hz, 2-$H_2$), 3.43-3.57 and 3.95-4.04 (4H, 2m, $N(CH_2)_2N$), 4.38 and 4.68 (2H, ABq, J 13Hz, $CH_2$SHet), 4.69 and 4.77 (2H, ABq, J 15Hz, $NCH_2Ar$), 5.31 (1H, s, 6-H), 5.72 (1H, d, J 7Hz, 2′-H), 6.92 (1H, s, $CHPh_2$), 7.20-7.70 (18H, m, 3 × $C_6H_5$ and $C_6H_3$), 8.30 (1H, s, CHO), 8.54 (1H, bs, exchangeable, NHCHO), 8.74 (1H, bs, exchangeable, 7β-CONH), 9.31 (1H, s, thiadiazole 5-H), 10.02 (1H, d, J 7Hz, exchangeable, 2′-NH); m/z (F.A.B.) 1019 ($MH^+$, 20%), 735 (19), 679 (13), 480 (24), 452 (95), 410 (100), 359 (41), 343 (55) and 321 (46).

e) Sodium 7β-[D-2-[[4-(2,3-Dihydroxybenzyl)-2,3-dioxo piperazin-1-yl]carbonylamino]-2-phenylacetamido]-7α-formamido-3-[(1,3,4-thiadiazol-2-yl)thiomethyl] ceph-3-em-4-carboxylate

Benzhydryl 7β-[D-2-[[4-(2,3-diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylate (267mg) was dissolved in dry dichloromethane (10ml) under argon and treated with anisole (850mg) then trifluoroacetic acid (890mg) at 0-5°C. Cooling was removed immediately and the mixture stirred at room temperature for 0.5h. The volatiles were removed under reduced pressure and the residue azeotroped with dichloromethane (3 × 10ml). Trituration under diethyl ether gave a white solid (222mg, 100%), which was collected by filtration, washed with diethyl ether and dried under reduced pressure.

The above solid (100mg) was suspended in water (3ml) and a trace of acetone and the pH adjusted to 8.0 with 2.5% aqueous sodium carbonate. Sodium sulphite (36mg) was added and the pH of the reaction mixture maintained between 8.9 and 9.1 by addition of 2.5% aqueous sodium carbonate when necessary. After 1.75h the mixture was chromatographed through HP20, eluting with tetrahydrofuran/water mixtures, and the product (22.5mg: 25%) was obtained after freeze-drying as a white powder; $\nu_{max}$ (KBr) 1769, 1710, 1676, 1609, 1508 (br), 1396, 1365, 1283, 1203 and 1064cm$^{-1}$; $\delta_H$ [250MHz; $(CD_3)_2CO$ + $D_2O$] 3.03 and 3.41 (2H, ABq, J 16Hz, 2-$H_2$), 3.58-3.73 and 3.90-4.03 (4H, 2m, $N(CH_2)_2N$), 4.08 and 4.42 (2H, ABq, J 14Hz, 3-$CH_2$), 4.67 (2H, bs, $NCH_2Ar$), 5.25 (1H, s, 6-H),5.52 (1H, s, 2′-H), 6.71-6.90 (3H, m, $C_6H_3$), 7.34-7.56 (5H, m, $C_6H_5$), 8.13 (1H, s, CHO), 9.40 (1H, s, thiadiazole 5-H).

## Example 17

Sodium 6β-[D-2-[4-(3,4-Dihydroxyphenyl)-2,3-dioxopiperazin-1-ylcarbonylamino]-2-phenylacetamido]-6α-formamidopenicillanate

a) 1-(3,4-Dibenzyloxyphenyl)-2,3-dioxopiperazine

3,4-Dibenzyloxyaniline (11.38g) and N-(2-bromoethyl)phthalimide (4.7g) were mixed together and heated to 120°C under argon. After 1h, the mixture was cooled to room temperature and triturated under dichloromethane (250ml). The insolubles were removed by filtration and the filtrate evaporated to dryness under reduced pressure. The residue was chromatographed rapidly through silica (<230 mesh ASTM), eluting with ethyl acetate/cyclohexane mixtures in a gradient of 0-30% ethyl acetate. The partially purified N-[2-(3,4-dibenzyloxyanilino)ethyl]phthalimide (7.4g) was heated under reflux in ethanol (100ml) in the presence of N-methylhydrazine (2ml) for 4h. The solvents were removed under reduced pressure and the

residue shaken well with excess 10% aqueous sodium hydroxide and dichloromethane (250ml). The phases were separated, the organic phase washed with water (50ml), saturated brine (50ml) and filtered through phase separation filter paper. The filtrate was evaporated to dryness under reduced pressure and the residue chromatographed through silica (<230 mesh ASTM), eluting with dichloromethane then 10% methanol in dichloromethane.

The partially-purified 2-(3,4-dibenzyloxyanilino)ethylamine (2.8g) was heated under reflux in diethyl oxalate (10ml) for 1h. After cooling the mixture to room temperature, diethyl ether (100ml) was added and the resulting solid was collected by filtration, washed with diethyl ether and recrystallised from ethanol to give the title compound (1.63g; 22%), m.p. 173°C (EtOH) (Found: C, 71.23; H, 5.44; N, 6.95. $C_{24}H_{22}N_2O_4$ requires C, 71.64; H, 5.47; N, 6.96%); $\nu_{max}$ (KBr) 3198, 1675, 1510, 1364, 1265, 1234, 1063, 1020, 737 and 696cm$^{-1}$; $\delta_H$ [250MHz; $(CD_3)_2SO$] 3.40-3.56 and 3.75-3.89 (each 2H, m, $N(CH_2)_2N$), 5.10 and 5.16 (each 2H, s, 2 × $CH_2Ph$), [6.89 (1H, dd, J 9 and 2Hz), 7.07 (1H, d, J 9Hz) and 7.19 (1H, d, J 2Hz) $C_6H_3$] and 8.74 (1H, s, exchangeable, NH) (Found: m/z 402.1584. <u>M</u>$^+$, $C_{24}H_{22}N_2O_4$ requires 402.1579).

b) <u>Benzyl 6β-[D-2-[4-(3,4-Dibenzyloxyphenyl)-2,3-dioxopiperazin-1-ylcarbonylamino]-2-phenylacetamido]-6α-methylthiopenicillanate</u>

1-(3,4-Dibenzyloxyphenyl)-2,3-dioxopiperazine (201mg) was heated under argon and under reflux for 4h in 1,1,1,3,3,3-hexamethyldisalazane (10ml) and chlorotrimethylsilane (1ml). The mixture was evaporated to dryness under reduced pressure and the residue, $\nu_{max}$ ($CH_2Cl_2$) 1685 and 1670cm$^{-1}$, redissolved in dry dichloromethane (10ml) under argon and cooled to 0-5°C. Trichloromethyl chloroformate (120μl) was added, cooling was removed and the mixture was stirred at room temperature for 1h. The volatiles were removed under reduced pressure and the residue, $\nu_{max}$ ($CH_2Cl_2$) 1800, 1690 br cm$^{-1}$, redissolved in dry dichloromethane (10ml). This solution was added at room temperature to a solution of benzyl 6β-(D-2-amino-2-phenylacetamido)6α-methylthiopenicillanate (242mg) and triethylamine (70μl) in dry dichloromethane (10ml). The solution was stirred for 0.5h, washed with water (10ml), saturated brine (10ml), dried ($MgSO_4$) and evaporated to dryness under reduced pressure. Chromatography through silica (<230 mesh ASTM), eluting with ethyl acetate/cyclohexane mixtures in a gradient of 20-40% ethyl acetate, gave the title compound (272mg; 61%); $\nu_{max}$ (KBr) 1775, 1740, 1710 and 1690cm$^{-1}$; $\delta_H$ [250MHz; $(CD_3)_2CO$] 1.04 and 1.22 [each 3H, s, 2-$(CH_3)_2$]·2.33 (3H, s, $SCH_3$), 4.01-4.27 (4H, m, $N(CH_2)_2N$), 4.41 (1H, s, 3-H), 5.13-5.27 (6H, m, 3 × $C\underline{H}_2Ph$), 5.46 (1H, s, 5-H), 5.74 (1H, d, J 7Hz, PhC<u>H</u>CO), 6.98-7.61 (23H, m, 4 × $C_6H_5$ and $C_6H_3$), 8.78 (1H, s, exchangeable, 6β-CONH), 10.04 (1H, d, J 7Hz, exchangeable, N<u>H</u>CHCO).

c) <u>Benzol 6β-[D-2-[4-(3,4-Dibenzyloxyphenyl)-2,3-dioxopiperazin-1-ylcarbonylamino]-2-phenylacetamido]-6α-formamidopenicillanate</u>

Benzyl 6β-[D-2-[4-(3,4-dibenzyloxyphenyl)-2,3-dioxopiperazin-1-ylcarbonylamino]-2-phenylacetamido]-6α-methylthiopenicillanate (272 mg) was dissolved at room temperature in dry <u>N</u>,<u>N</u>-dimethylformamide (5ml). Mercuric acetate (98mg) was added followed immediately by a solution of ammonia in <u>N</u>,<u>N</u>-dimethylformamide (10% excess of ammonia). After 15 min., the reaction mixture was diluted with ethyl acetate (50ml) and filtered. The filtrate was washed with water (5 × 25ml), saturated brine (25ml), dried ($MgSO_4$) and evaporated to dryness under reduced pressure. The residue was redissolved in dry dichloromethane (10ml), cooled to 0-5°C under argon and treated with pyridine (248μl) followed by aceticformic anhydride (121μl) and the cooling removed. After 3h at room temperature, the reaction mixture was washed with water (10ml), saturated aqueous sodium hydrogen carbonate (10ml), water (10ml), saturated aqueous copper sulphate (10ml), water (10ml), saturated brine (10ml), dried ($MgSO_4$) and evaporated to dryness under reduced pressure. The residue was chromatographed through silica (<230 mesh ASTM), eluting with 75% ethyl acetate in cyclohexane, to give the title compound (130mg; 48%); $\nu_{max}$ ($CH_2Cl_2$) 1780, 1740, 1690 br cm$^{-1}$; $\delta_H$ [250MHz; $(CD_3)_2CO$] 0.90 and 1.17 (each 3H, s, 2-$(CH_3)_2$), 4.03-4.24 (4H, m, $N(CH_2)_2N$), 4.41 (1H, s, 3-H), 5.12-5.29 (6H, m, 3 × $C\underline{H}_2Ph$), 5.60 (1H, s, 5-H), 5.74 (1H, d, J 7Hz, PhC<u>H</u>CO), 6.96-7.67 (23H, m, 4 × $C_6H_5$ and $C_6H_3$), 8.18 (1H, s, CHO), 8.29 (1H, br s, exchangeable, N<u>H</u>CHO), 8.80 (1H, s, exchangeable, 6β-CONH), 10.14 (1H, d, J 7Hz, N<u>H</u>CHCO); <u>m/z</u> (F.A.B.) 933 (<u>M</u>Na+, 50%), 911 (<u>M</u>H$^+$, 4) and 531 (100).

d) Sodium 6β-[D-2-[4-(3,4-Dihydroxyphenyl)-2,3-dioxopiperazin-1-ylcarbonylamino]-2-phenylacetamido]-6α-formamidopenicillanate

Benzyl 6β-[D-2-[4-(3,4-dibenzyloxyphenyl)-2,3-dioxopiperazin-1-ylcarbonylamino]-2-phenylacetamido]-6α-formamidopenicillanate (130mg) was dissolved in redistilled tetrahydrofuran (15ml) and hydrogenated over a mixture of 10% palladium on carbon (100mg) and palladium black (100mg) until complete deprotection had been achieved. The catalyst was removed by filtration through Celite and the filtrate evaporated to dryness under reduced pressure. The residue was redissolved in the minimum quantity of acetone and treated with a 1.93M solution of sodium 2-ethylhexanoate in methylisobutyl ketone (64µl). The volatiles were then removed under reduced pressure and the residue triturated under diethyl ether and the title compound (81mg; 86%) obtained by filtration; $\nu_{max}$ (KBr) 1777, 1684, and 1610cm$^{-1}$; $\delta_H$ [250MHz; $D_2O$ + $(CD_3)_2CO$] 1.00 and 1.40 (each 3H, s, 2-$(CH_3)_2$), 4.11-4.23 and 4.27-4.38 (each 2H, m, $N(CH_2)_2N$), 4.25 (1H, s, 3-H), 5.72 (1H, s, 5-H), 5.75 (1H, s, PhCHCO), 6.88-7.09 (3H, m, $C_6H_3$), 7.49-7.74 (5H, m, $C_6H_5$), 8.27 (1H, s, CHO).

Example 18

Sodium 6β-[D-2-[4-[2-(3,4-Dihydroxyphenyl)ethyl]-2,3-dioxopiperazin-1-ylcarbonylamino]-2-phenylacetamido]-6α-formamidopenicillanate

a) 1-Amino-2-[2-(3,4-dibenzyloxyphenyl)ethylamino] ethane

1-Amino-2-(3,4-dibenzyloxyphenyl)ethane (4.7g) and 2-bromoethylamine hydrobromide (1.45g) were heated at reflux in toluene (50ml) for 5h. The reaction mixture was cooled to room temperature and made basic (ca. pH 14) with 10% aqueous sodium hydroxide solution. The phases were separated and the organic phase washed with water (25ml) and saturated brine (25ml) before being filtered through phase separation filter paper. The filtrate was evaporated to dryness under reduced pressure and chromatographed through silica (<230 mesh ASTM), eluting with mixtures of methanol in dichloromethane in a gradient of 0-30% methanol. The title compound was obtained as a dark-brown oil (0.73g; 27%); $\delta_H$ (60MHz; $CDCl_3$) 2.73 (8H, brs, 2 × $CH_2CH_2$), 3.50 (3H, brs, exchangeable, NH and $NH_2$), 5.00 (4H, m, 2 × $PhCH_2O$), 6.56-6.82 (3H, m, $C_6H_3$), 7.01-7.50 (10H, m, 2 × $C_6H_5$).

b) 1-[2-(3,4-Dibenzyloxyphenyl)ethyl]-2,3-dioxopiperazine

1-Amino-2-[2-(3,4-dibenzyloxyphenyl)ethylamino]ethane (0.73g) was heated at reflux in diethyl oxalate (10ml) for 1h. The mixture was evaporated to dryness under reduced pressure and the residue chromatographed through silica (<230 mesh ASTM), eluting with ethyl acetate then 10% ethanol in ethyl acetate, to give the product (0.35g; 42%); $\nu_{max}$ ($CH_2Cl_2$) 3380, 1700 and 1678cm$^{-1}$; $\delta_H$ (60MHz; $CDCl_3$) 2.51-3.75 (8H, m, 2 × $CH_2CH_2$), 5.10 (4H, s, 2 × $CH_2Ph$), 6.60-7.75 (13H, m, 2 × $C_6H_5$ and $C_6H_3$), 8.43 (1H, brs, exchangeable, NH).

c) Benzyl 6β-[D-2-[4-[2-(3,4-Dibenzyloxyphenyl)ethyl] -2,3-dioxopiperazin-1-ylcarbonylamino]-2-phenylacetamido]-6α-methylthiopenicillanate

1-[2-(3,4-Dibenzyloxyphenyl)ethyl]-2,3-dioxopiperazine (350mg) was dissolved in dry dichloromethane (10ml) under argon and treated with chlorotrimethylsilane (88.3mg) and triethylamine (82.2mg). More chlorotrimethylsilane (44.15mg) and triethylamine (41.1mg) were added after 2h and the mixture stirred for a further 1h. Infra-red analysis of the mixtur, $\nu_{max}$ ($CH_2Cl_2$) 1670, 1665 sh cm$^{-1}$; showed complete silylation and the mixture was treated with trichloromethyl chloroformate (162mg). After 0.75h, the volatiles were removed by evaporation under reduced pressure and the residue, $\nu_{max}$ ($CH_2Cl_2$), 1800, 1720 and 1685cm$^{-1}$, redissolved in dry dichloromethane (10ml). This solution was added to a solution of benzyl 6β-(D-2-amino-2-phenylacetamido)-6α-methylthiopenicillanate (394mg) and triethylamine (82.2mg) in dry dichloromethane (10ml). After 0.5h, the mixture was washed with water, saturated brine, dried ($MgSO_4$) and evaporated to dryness under reduced pressure. The residue was chromatographed through silica (<230 mesh ASTM), eluting with 50% ethyl acetate in cyclohexane then 60% ethyl acetate in cyclohexane to give the title compound (629mg; 82%); $\nu_{max}$ ($CH_2Cl_2$), 1780, 1740 sh, 1710, 1690 and 1180cm$^{-1}$; $\delta_H$ [250MHz; $(CD_3)_2CO$] 1.03 and 1.21 (each 3H, s, 2-$(CH_3)_2$), 2.31 (3H, s, $SCH_3$), 2.85 (2H, t, J 7Hz, $ArCH_2CH_2$), 3.32-3.42 (2H, m, $CH_2NCH_2CH_2N$), 3.66 (2H, t, J 7Hz, $CH_2NCH_2CH_2N$), 3.76-3.87 (2H, m, $NCH_2CH_2NCO$), 4.40 (1H, s, 3-H), 5.14 and 5.18 (each 2H, s, 2 × $ArOCH_2Ph$), 5.18 and 5.24 (2H, ABq, J 14Hz, $CO_2CH_2Ph$), 5.45 (1H, s, 5-H), 5.70 (1H, d, J 7Hz, PhCHCO) [6.81 (1H, dd, J 8 and 2Hz), 6.98 (1H, d, J 8Hz), 7.05 (1H, d, J 2Hz), $C_6H_3$], 7.25-7.62 (20H, m, 4 × $C_6H_5$), 8.76 (1H, s, exchangeable, 6β-CONH), 10.00 (1H, d, J 7Hz, NHCHCO); m/z - (F.A.B.) 964 ($MNa^+$, 100%) and 534 (50).

d) Benzyl 6β-[D-2-[4-[2-(3,4-Dibenzyloxyphenyl)ethyl] -2,3-dioxopiperazin-1-ylcarbonylamino]-2-phenylacetamido]-6α-formamidopenicillanate

Benzyl 6β-[D-2-[4-[2-(3,4-dibenzyloxyphenyl)ethyl]-2,3-dioxopiperazin-1-ylcarbonylamino]-2-phenylacetamido]-6α-formamidopenicillanate (629mg) was dissolved in dry N,N-dimethylformamide (10ml) under argon and treated with mercuric acetate (213mg) then an excess of ammonia in dry N,N-dimethylformamide. After 0.5h, the mixture was diluted with ethyl acetate (50ml) and filtered. The filtrate was washed with water (5 × 20ml), saturated brine (20ml), dried ($MgSO_4$) and evaporated to dryness under reduced pressure. The residue was dissolved in dry dichloromethane (20ml) under argon and treated with pyridine (528mg) then acetic formic anhydride (294mg) and the resulting mixture stirred at room temperature for 3h. The reaction mixture was washed with water (20ml), saturated, aqueous sodium hydrogencarbonate (20ml), water (20ml), saturated, aqueous cupric sulphate (20ml), water (20ml), saturated brine (20ml), dried ($MgSO_4$) and evaporated to dryness under reduced pressure. The residual foam was chromatographed through silica (<230 mesh ASTM), eluting with cyclohexane/ethyl acetate mixtures in a gradient of 70-100% ethyl acetate, to give the title compound (446mg; 71%); $\nu_{max}$ (KBr) 1786, 1740, 1710 and 1690cm$^{-1}$; $\delta_H$ [250MHz; $(CD_3)_2CO$] 0.90 and 1.18 (each 3H, s, 2-$(CH_3)_2$), 2.88 (2H, t, J 7Hz, $ArCH_2CH_2N$), 3.31-3.41 (2H, m, $NCH_2CH_2NCO$), 3.67 (2H, t, J 7Hz, $ArCH_2CH_2N$), 3.76-3.87 (2H, m, $NCH_2CH_2NCO$), 4.39 (1H, s, 3-H), 5.11-5.29 (6H, m, 2 × $OCH_2Ph$ and $CO_2CH_2Ph$), 5.59 (1H, s, 5-H), 5.69 (1H, d, J 7Hz, PhCHCO), [6.80 (1H, dd, J 8 and 2Hz), 6.98 (1H, d, J 8Hz), 7.07 (1H, d, J 2Hz), $C_6H_3$], 7.24-7.62 (20H, m, 4 × $C_6H_5$), 8.18 (1H, d, J 1Hz, CHO), 8.25 (1H, brs, exchangeable, NHCHO), 8.80 (1H, brs, exchangeable, 6β-CONH), 10.05 (1H, d, J 7Hz, exchangeable, NHCHO), m/z (F.A.B.) 961 ($MNa^+$, 65%) and 531 (100).

e) Sodium 6β-[D-2-[4-[2-(3,4-Dihydroxyphenyl)ethyl-2,3-dioxopiperazin-1-ylcarbonylamino]-2-phenylacetamido]-6α-formamidopenicillinate

Benzyl 6β-[D-2-[4-[2-(3,4-dibenzyloxyphenyl)ethyl]-2,3-dioxopiperazin-1-ylcarbonylamino]-2-phenylacetamido]- 6α-formamidopenicillanate (446mg) was hydrogenated over a mixture of 10% palladium on carbon (225mg) and palladium black (225mg) in redistilled tetrahydrofuran (20ml) until complete deprotection had been achieved. In this case a change of catalyst was found to be necessary for the reaction to go to completion. When deprotection was complete, the catalyst was removed by filtration through 'Celite'. The filter bed was washed with redistilled tetrahydrofuran and the filtrate evaporated to dryness under reduced pressure. Trituration under diethyl ether gave a crude free acid (238mg; 75%), which was collected by filtration, washed with diethyl ether and dried under reduced pressure. The free acid (150mg) was dissolved in water (10ml) by addition of saturated, aqueous sodium hydrogencarbonate to pH 6.5 and subjected to HP20 chromatography. Elution with mixtures of tetrahydrofuran in water gave a number of column fractions containing the title compound. These were combined and evaporated to dryness under reduced pressure. The last traces of water were removed by azeotroping with ethanol (10ml) then toluene (10ml). The residual, white solid was triturated under diethyl ether and the title compound collected by filtration, washed with diethyl ether and dried under reduced pressure (61mg; 19%); $\nu_{max}$ (KBr) 1773, 1710, 1679 and 1610cm$^{-1}$; $\delta_H$ (250MHz; $D_2O$) 0.83 and 1.23 (each 3H, s, 2-$(CH_3)_2$), 2.79 (2H, t, J 6Hz, $ArCH_2$), 3.30-3.45 (2H, m, $NCH_2CH_2NCO$), 3.59-3.86 (4H, m, $CH_2NCH_2CH_2NCO$), 4.14 (1H, s, 3-H), 5.40 (1H, s, 5-H), 5.56 (1H, s, PhCHCO), 6.62-6.86 (3H, m, $C_6H_3$), 7.35-7.54 (5H, m, $C_6H_5$), 8.08 (1H, s, CHO).

Example 19

Sodium 7$\beta$-[DL-2-[4-(3,4-Dihydroxyphenyl)-2,3-dioxopiperazin-1-ylcarbonylamino]-2-phenylacetamido]-7$\alpha$-formamido-3-[(1,3,4-thiadiazol-2-yl)thiomethyl]ceph-3-em-4-carboxylate

a) 1-(3,4-Diacetoxyphenyl)-2,3-dioxopiperazine

1-(3,4-Dibenzyloxyphenyl)-2,3-dioxopiperazine (797mg) was dissolved in methanol and tetrahydrofuran (1:1; 200ml) and hydrogenated over 10% palladium on carbon (797mg) until hydrogen uptake had ceased. The catalyst was removed by filtration through 'Celite', the filter bed washed with methanol/tetrahydrofuran (1:1, 3 × 20ml) and the filtrate evaporated to dryness under reduced pressure to give 1-(3,4-dihydroxyphenyl)-2,3-dioxopiperazine (406mg; 92%) as a grey solid.

The above piperazinedione was suspended in dry dichloromethane (20ml) under argon and treated with pyridine (0.1ml), 4-dimethylaminopyridine (10mg) and then acetic anhydride (410mg). After 18h at room temperature, the insoluble solid (356mg; 64%) was collected by filtration, washed with a small volume of ethanol and then diethyl ether. The filtrate was evaporated to dryness under reduced pressure and the residue triturated under a small volune of ethanol. The resulting solid (165mg; 29%) was collected by filtration and washed with diethyl ether. The two crops were combined and recrystallised from ethanol (334mg; 60%), m.p. 197-198 °C; $\nu_{max}$ (KBr) 1772, 1709, 1674 and 1200cm$^{-1}$; $\delta_H$ [60MHz; $(CD_3)_2CO$ + $(CD_3)_2SO$] 2.30 (6H, s, 2 × $CH_3CO_2$), 3.42-3.69 and 3.83-4.15 (each 2H, m, $N(CH_2)_2N$), 7.33 (3H, br s, $C_6H_3$), 8.16-8.99 (1H, diffuse, exchangeable, NH).

b) D-2-[4-(3,4-Diacetoxyphenyl)-2,3-dioxopiperazin-1-ylcarbonylamino]-2-phenylacetic acid

1-(3,4-Diacetoxyphenyl)-2,3-dioxopiperazine (294mg) was suspended in dry dichloromethane (20ml) under argon and treated with chlorotrimethylsilane (104mg) followed by triethylamine (97mg). Examination of the reaction mixture after 1h by infra-red spectoscopy ($\nu_{max}$ ($CH_2Cl_2$) 1770, 1685 and 1665cm$^{-1}$) showed complete conversion to the O-silyliminoether. Trichloromethyl chloroformate (190mg) was added and the resulting mixture stirred at room temperature for a further 1h. Evaporation to dryness under reduced pressure gave the carbonyl chloride ($\nu_{max}$ ($CH_2Cl_2$) 1800, 1770, 1720 and 1700cm$^{-1}$) which was redissolved in dry dichloromethane (20ml). This solution was added to a solution of persilylated D-phenylglycine in dry dichloromethane (20ml) (prepared by heating D-phenylglycine (145mg) in hexamethyldisilazane (10ml) containing chlorotrimethylsilane (1ml) under argon at reflux for 2h) and the resulting mixture stirred at room temperature under argon for 0.5h. The volatiles were removed under reduced pressure and the residue treated with ethyl acetate (50ml) and 1M hydrochloric acid (20ml). After stirring vigorously for 15 min., the phases were separated and the organic phase washed with 1M hydrochloric acid (20ml), water (20ml), saturated brine (20ml), dried ($MgSO_4$) and evaporated to dryness under reduced pressure to give the title compound (420mg; 90%) as a white foam; $\nu_{max}$ ($CH_2Cl_2$) 1770, 1715, 1700 and 1205cm$^{-1}$; $\delta_H$ [60MHz; $(CD_3)_2CO$] 2.25 (6H, 2 × $CH_3CO_2$), 4.02 (4H, br s, $N(CH_2)_2N$), 5.49 (1H, d, J 7Hz, PhCHCO), 7.10-7.53 (8H, m, aromatics), 8.35-9.20 (1H, diffuse, exchangeable, $CO_2H$), 9.84 (1H, d, J 7Hz, exchangeable, NH).

c) Benzhydryl 7$\beta$-[DL-2-[4-(3,4-Diacetoxyphenyl)-2,3-dioxopiperazin-1-ylcarbonylamino]-2-phenylacetamido]-7$\alpha$-formamido-3-[(1,3,4-thiadiazol-2-yl)thiomethyl]ceph-3-em-4-carboxylate

Benzhydryl 7$\beta$-amino-7$\alpha$-formamido-3-[(1,3,4-thiadiazol-2-yl)thiomethyl]ceph-3-em-4-carboxylate (468mg) and N,N dicyclohexylcarbodiimide (196mg) were dissolved in the minimum volume of acetone and the solution cooled to 0-5 °C. A solution of D-2-[4-(3,4-diacetoxyphenyl)-2,3-dioxopiperazin-1-ylcarbonylamino]-2-phenylacetic acid (420mg) in the minimum volume of acetone was added slowly and the mixture stirred at room temperature for 18h. The insolubles were removed by filtration, washed with acetone (20ml) and the filtrate evaporated to dryness under reduced pressure. Chromatography through silica (<230 mesh ASTM), eluting with ethyl acetate/cyclohexane mixtures in a gradient of 80-100% ethyl acetate, gave the title compound (305mg; 35%) as a white foam; $\nu_{max}$ ($CH_2Cl_2$).1770, 1700 br, 1205 and 1180cm$^{-1}$; $\delta_H$ -

[250MHz; $(CD_3)_2CO$] 2.28 (6H, s, 2 × $CH_3CO_2$), 3.18 and 3.30, and 3.49 and 3.62 (each 2H, ABq, J 16Hz, 2-$H_2$, isomers), 4.11-4.28 (4H, m, $N(CH_2)_2N$), 4.38 and 4.68, and 4.39 and 4.71 (each 2H, ABq, J 16Hz, 3-$CH_2S$, isomers), 5.30 and 5.31 each 1H, s, 6-H, isomers), 5.76 and 5.81 (each 1H, d, J 7Hz, PhCHCO; isomers), 6.93 and 6.94 (each 1H, s, $CHPh_2$, isomers), 7.21-7.68 (18H, m, aromatics), 8.24 and 8.30 (each 1H, s, CHO, isomers), 8.55 (1H, br s, exchangeable, NHCHO), 8.62 and 8.74 (each 1H, br s, exchangeable, 7β-CONH, isomers), 9.30 (1H, s, thiadiazole 5-H), 10.05 (1H, d, J 7Hz, exchangeable, NHCHCO)

d) Sodium 7β-[DL-2-[4-(3,4-Dihydroxyphenyl)-2,3-dioxopiperazin-1-ylcarbonylamino]-2-phenylacetamido]-7α-formamido-3-[(1,3,4-thiadiazol-2-yl)thiomethyl]ceph-3-em-4-carboxylate

Benzhydryl 7β-[DL-2-[4-(3,4-diacetoxyphenyl)-2,3-dioxopiperazin-1-ylcarbonylamino]-2-phenylacetamido]-7α-formamido-3-[(1,3,4-thiadiazol-2-yl)thiomethyl]ceph-3-em-4-carboxylate (305mg) was dissolved in dry dichloromethane (10ml) under argon and cooled to 0-5°C. Anisole (984mg) and then trifluoroacetic acid (1.04g) were added and the mixture allowed to regain room temperature. After 0.5h at room temperature, the volatiles were removed under reduced pressure and the residue triturated under diethyl ether. The resulting solid was collected by filtration, washed with diethyl ether and dried in air.

The above free acid was suspended in water (5ml) and dissolved by the addition of saturated, aqueous sodium hydrogen carbonate to pH 7.2. A suspension of acetyl esterase in 2.5M aqueous ammonium sulphate solution (1.0ml, ex. Sigma) was added and the mixture gently agitated. A further portion (1.0ml) of enzyme was added at 0.75h and again (1.0ml) at 1.5h. When analysis by high performance liquid chromatography showed that reaction was complete, the reaction mixture was subjected to chromatography through HP20. Elution with mixtures of tetrahydrofuran in water in a gradient 0-30% tetrahydrofuran, followed by freeze-drying of the relevant column fractions, gave the title compound (34mg; 14%) as a white powder; $\nu_{max}$ (KBr) 1780, 1705, 1675, 1516, 1390, 1362 and 1186cm$^{-1}$; $\delta_H$ [250MHz; $D_2O$ + $(CD_3)_2CO$] 2.98 and 3.36, and 3.00 and 3.50 (each 2H, ABq, J 17Hz, 2-$H_2$, isomers), 3.93-4.51 (6H, m, $N(CH_2)_2N$ and 3-$CH_2S$), 5.18 and 5.23 (each 1H, s, 6-H, isomers), 5.56 and 5.52 (each 1H, s, PhCHCO, isomers), [6.78 (1H, dd, J 2 and 8Hz), 6.89 (1H, d, J 2Hz) and 6.95 (1H, d, J 8Hz), $C_6H_3$], 7.37-7.59 (5H, m, $C_6H_5$), 8.06 and 8.11 (each 1H, s, CHO), 9.38 and 9.39 (each 1H, s, thiadiazole 5-H, isomers).

## Example 20

Sodium 3-Acetoxymethyl-7β-[D-2-[4-(3,4-dihydroxybenzyl) -2,3-dioxopiperazin-1-ylcarbonylamino]-2-phenylacetamido]ceph-3-em-4-carboxylate

1-(3,4-Dihydroxybenzyl)-2,3-dioxopiperazine (236mg) was suspended in dry dichloromethane (20ml) under argon and treated with chlorotrimethylsilane (326mg) then triethylamine (303mg). After 1h at room temperature, $\nu_{max}$ ($CH_2Cl_2$) 1670cm$^{-1}$, the mixture was treated with trichloromethyl chloroformate (198mg) and stirred at room temperature for a further 1.5h. The volatiles were removed by evaporation under reduced pressure to yield the carbonyl chloride intimately mixed with triethylamonium chloride, $\nu_{max}$ ($CH_2Cl_2$) 1800, 1725 and 1690cm$^{-1}$. This mixture was redissolved in dry dichloromethane (20ml) and added to a solution of 3-acetoxymethyl-7β-(D-2-amino-2-phenylacetamido)ceph-3-em-4-carboxylic acid (0.5g) and triethylamine (0.4ml) in dry dichloromethane (20ml). After 2h at room temperature, the mixture was evaporated to dryness under reduced pressure and the residue treated with water (30ml) and ethyl acetate (30ml). The phases were separated, the aqueous phase washed with ethyl acetate (30ml) then 'layered' with ethyl acetate (30ml). This two-phase mixture was treated with 5M hydrochloric acid to pH 2.0 and the phases separated. The aqueous phase was further extracted with ethyl acetate (25ml), the extracts combined, washed with 1M hydrochloric acid (20ml), water (20ml), saturated brine (20ml), dried ($MgSO_4$) and evaporated to dryness under reduced pressure. The resulting solid was redissolved in dry acetone (5ml) and treated with 1.93M sodium 2-ethylhexanoate in 4-methylpentan-2-one (0.35ml). Diethyl ether (50ml) was added and the title compound, 419mg (61%), collected by filtration, washed with diethyl ether (50ml) and dried under reduced pressure; $\nu_{max}$ (KBr) 1765, 1715, 1685, 1609, 1517, 1395, 1364 and

1232cm$^{-1}$; $\delta_H$ (250MHz; $D_2O$) 2.04 (3H, s, $CH_3CO_2$), 3.07-3.54 (4H, m, 2-$H_2$ and $ArCH_2NC\underline{H}_2$), 3.65-3.98 (2H, m, $CONHC\underline{H}_2CH_2N$), 4.49 (2H, brs, $C\underline{H}_2Ar$), 4.61 and 4.81 (2H, ABq, J 12Hz, 3-$CH_2S$), 4.96 (1H, d, J 5Hz, 6-H), 5.41 (1H, s, $PhC\underline{H}CO$), 5.62 (1H, d, J 5Hz, 7-H), 6.68-6.89 (3H, m, $C_6H_3$), 7.34-7.53 (5H, m, $C_6H_5$).

Example 21

Sodium 3-Acetoxymethyl-7β-[D-2-[4-(1,4-dihydro-5-hydroxy-4-oxopyridin-2-yl)methyl-2,3-dioxopiperazin-1-ylcarbonylamino]-2-phenylacetamido]ceph-3-em-4-carboxylate

(a) 1-(1,4-Dihydro-5-hydroxy-4-oxopyridin-2-yl)methyl-2,3-dioxopiperazine

1-(4,5-Dibenzyloxypyridin-2-yl)methyl-2,3-dioxopiperazine (1.0g) was dissolved in a mixture of methanol and tetrahydrofuran (1:1; 150ml) and hydrogenated at STP over 10% palladium on carbon (1.0g). When hydrogen uptake had ceased, the catalyst was removed by filtration through "Celite", the filter bed washed with a mixture of methanol and tetrahydrofuran (1:1; 3 × 20ml) and the filtrate evaporated to dryness under reduced pressure to give the title compound as a white powder (465mg; 82%), m.p. 177°C (dec.); $\nu_{max}$ (Nujol) 1700, 1660, 1650 and 1635cm$^{-1}$; $\delta_H$ [60MHz; $(CD_3)_2SO$] 3.27-3.54 (4H, m, $N(CH_2)_2N$), 4.48 (2H, s, $NC\underline{H}_2pyr$), 6.25 and 7.34 (each 1H, s, 2 × pyridone-CH), 8.32-8.76 (1H, brs, exchangeable, piperazine-NH).

(b) Sodium 3-acetoxymethyl-7β-[D-2-[4-(1,4-dihydro-5-hydroxy-4-oxopyridin-2-yl)methyl-2,3-dioxopiperazin-1-ylcarbonylamino]-2-phenylacetamido]ceph-3-em-4-carboxylate

1-(1,4-Dihydro-5-hydroxy-4-oxopyridin-2-yl)methyl-2,3-dioxopiperazine (237mg) was suspended in dry dichloromethane (20ml) under argon. Chlorotrimethylsilane (0.38ml) followed by triethylamine (0.42ml) were added and the mixture stirred at room temperature for 1h. The resulting solution, [$\nu_{max}$ ($CH_2Cl_2$) 1675 and 1665cm$^{-1}$], was treated with trichloromethyl chloroformate (0.12ml) and the resulting mixture stirred at room temperature for a further 0.75h. The volatiles were removed under reduced pressure and the residue [$\nu_{max}$ ($CH_2Cl_2$) 1865, 1855, 1795, 1725 and 1690cm$^{-1}$] suspended in dry dichloromethane (20ml). This suspension was added to a mixture of 3-acetoxymethyl-7β-(D-2-amino-2-phenylacetamido)ceph-3-em-4-carboxylic acid dihydrate (0.5g), triethylamine (0.4ml) and 3A molecular sieves (5g) in dry dichloromethane (20ml) under argon at room temperature and the resulting mixture stirred for 1h. The insoluble material was removed by filtration and the filtrate evaporated to dryness under reduced pressure. The residue was treated with water (25ml) and ethyl acetate (25ml) and the phases separated. The aqueous phase was 'layered' with ethyl acetate (25ml) and acidified to pH 2 with 5M hydrochloric acid. The insoluble material was collected by filtration, washed with water (10ml) and ethyl acetate (10ml). It was then suspended in water (20ml) and dissolved by addition of saturated aqueous sodium hydrogen carbonate solution to pH 8.0. The resulting mixture was subjected to chromatography through HP20, eluting with mixtures of tetrahydrofuran in water in a gradient of 0-50% tetrahydrofuran. The product-containing fractions were combined, concentrated under reduced pressure and freeze-dried to give the title compound (80mg; 12%); $\nu_{max}$ (KBr) 1772, 1717, 1508, 1394, 1363 and 1229cm$^{-1}$; $\delta_H$ [250MHz; $(CD_3)_2SO$] 2.02 (3H, s, $CH_3CO_2$) 3.37 and 3.51 (2H, ABq, J 18Hz, 2-$H_2$), 3.48-3.64 (2H, m, $CH_2NC\underline{H}_2CH_2N$), 3.87-4.05 (2H, m, $NCH_2C\underline{H}_2NCO$), 4.50 (2H, 2 brs, $C\underline{H}_2pyr$), 4.67 and 4.97 (2H, ABq, J 13Hz, 3-$C\underline{H}_2S$), 5.01 (1H, d, J 5Hz, 6-H), 5.66 (1H, d, J 7Hz, $PhC\underline{H}CO$), 5.74 (1H, dd, J 5 and 8Hz, 7-H), 6.35 (1H, brs, pyridone 3-H), 7.28-7.56 (6H, m, $C_6H_5$ and pyridone 6-H), 9.48 (1H, d, J 8Hz, exchangeable, 7β-CONH), 9.88 (1H, d, J 7Hz, exchangeable, $N\underline{H}CHCO$).

Example 22

Sodium [6R,7R] 3-(1,3,4-Thiadiazol-2-yl)thiomethyl-7[[R]-2[2,3-dioxo-4(3,4-dihydroxybenzylideneamino)-piperazin-1-yl-carbonylamino]-2-phenylacetamido]-7-formamido-ceph-3-em-4-carboxylate

(a) 1-t-Butoxycarbonyl-2(2-hydroxyethyl)hydrazine

Glycolaldehyde (2g) and t-butyl carbazate (4.4g) were each dissolved into warm ethanol (30ml). The two solutions were combined and evaporated. The residual gum was redissolved into ethanol (20ml) and toluene (50ml) and reevaporated. This process was repeated three times before the residual gum was taken up into ethanol (100ml) and hydrogenated over 10% palladium on carbon (5g) until hydrogen uptake had ceased. The reaction was filtered, evaporated and chromatographed on silica eluting with ethyl acetate and n-hexane (2:1). The product was isolated as an oil that slowly crystallized (3g; 51%), m.p. 53°C; $\nu_{max}$ (KBr) 3308 and 1706cm$^{-1}$; $\delta$ ($CDCl_3$) 1.5 (9H, s), 2.86-3.16 (2H, m), 3.56-3.86 (2H, m), 4.20 (2H, br s), 6.96 (1H, br s); m/z 117 (MH$^+$, 2%) and 121 (100).

(b) Ethyl N-(t-Butoxycarbonylamino)-N(2-hydroxyethyl)oxamate

The product from part (a) (2.53g) was dissolved into dry tetrahydrofuran (50ml) and cooled to 0°C under argon. Propylene oxide (4ml) was added followed by dropwise addition of ethyl oxalylchloride (1.76ml) in dry tetrahydrofuran (10ml). After 15 minutes the reaction was evaporated causing the product to crystallize. The solid was washed with hot n-hexane (3 × 30ml) followed by ether (2 × 20ml) before being dried under vacuum (3g; 75.6%); m.p. 130-131°C; $\nu_{max}$ (KBr) 3437, 3198, 1741 and 1675cm$^{-1}$; $\delta$ ($CDCl_3$) 1.11-1.66 (12H, m), 3.23 (1H, br s), 3.49-3.87 (4H, m), 4.03-4.5 (2H, m) and 7.34 (1H, br s); m/z 553 (2M + H$^+$, 4%), 277 (MH$^+$, 23), 221 (93) and 203 (100).

(c) Ethyl N-(t-Butoxycarbonylamino)-N-(2-methanesulphonyloxyethyl)oxamate

The product from part (b) (2.92g) was dissolved into dichloromethane (50ml) and cooled to -20°C under argon. Triethylamine (1.5ml) was added followed by the dropwise addition of methanesulphonyl chloride (0.9ml) dissolved into dichloromethane (10ml). After 20 minutes the reaction was diluted with chloroform and washed with water (4 × 80ml), dried ($MgSO_4$) and evaporated to a colourless oil. The product crysallized on standing and was triturated with n-hexane and filtered (3.31g; 88.4%); m.p. 74-75°C; $\nu_{max}$ (KBr) 3288, 1743 and 1700cm$^{-1}$; $\delta$ ($CDCl_3$) 1.2-1.69 (12H, m), 3.09 (3H, s), 3.69-4.62 (6H, m), 7.23 (1H, br s); m/z 299 (100).

(d) Ethyl N-(t-Butoxycarbonylamino)-N-(2-azidoethyl)oxamate

The product from part (c) (3.31g) was dissolved into N,N-dimethylformamide (35ml) and tetramethylguanidium azide (1.68g) was added. The reaction was heated to 55°C for 2.5 hours and then allowed to cool. After dilution with ethyl acetate (70ml) the solution was washed with brine (3 × 50ml), dried ($MgSO_4$) and evaporated to an oil. Chromatography on silica eluting with n-hexane/ethyl acetate (3:1 graduating to 1:1) afforded the product as a colourless oil (1.73g; 61%); $\nu_{max}$ (film) 3300, 2098, 1739 and 1688cm$^{-1}$; $\delta$ ($CDCl_3$) 1.14-1.64 (12H, m), 3.4-3.93 (4H, m), 4.1-4.53 (2H, m), 7.34 (1H, br s).

(e) 1(t-Butoxycarbonylamino)-2,3-dioxopiperazine

The product from part (d) (1.6g) was dissolved into tetrahydrofuran and methanol (1:1, 80ml) to which was then added Lindlar catalyst (1.5g) as a suspension in ethanol (10ml). The reaction was then hydrogenated and monitored by I.R. for the disappearance of the stretching frequency occurring at 2098cm$^{-1}$. After 0.5 hour the reaction was filtered and evaporated. The crystalline residue was triturated with ethanol (30ml), and ether (30ml) then added. The product was filtered and dried under vacuum (1.195g; 98%); m.p. 189°C; $\nu_{max}$ (KBr) 3233, 3134 sh, 1736, 1700 and 1680 sh cm$^{-1}$; $\delta$ (DMSO-D6) 1.43 (9H, s), 3.0-3.74 (4H, m, + $H_2O$), 8.6 (1H, br s) and 9.30 (1H, br s); m/z 403 (2M + H$^+$, 1%), 230 (MH$^+$, 8), 174 (100) and 156 (7).

(f) 1-(3,4-Dihydroxybenzylideneamino)-2,3-dioxopiperazine

The product from part (e) (0.249g) and 3,4-dihydroxybenzaldehyde (0.15g) were dissolved into glacial acetic acid (25ml). After stirring at 90°C for 1.5 hours the reaction was cooled and the product filtered. After washing with glacial acetic acid (20ml) and ethyl acetate (50ml), the product was dried under vacuum to give a buff coloured solid (0.213g; 78.6%); m.p. 238-242°C decomp; $\nu_{max}$ (KBr) 3299, 1675 and 1646cm$^{-1}$; m/z 249 ($M^+$, 33%), 136 (100), 135 (80) and 115 (74).

(g) [R] N-[[4(3-4-Dihydroxbenzylideneamino)-2,3-dioxo piperazin-1-yl]-carbonyl]-2-phenylglycine

The product from part (f) (0.2g) as a suspension in dichloromethane (50ml) was cooled under argon to O°C. The reaction was treated sequentially with trimethylsilylchloride (0.32ml) and triethylamine (0.35ml). After 5 minutes the cooling bath was removed for 1 hour, when the reaction was recooled to 0°C and treated with trichloromethyl chloroformate (0.1ml). After a further 1 hour the reaction was evaporated from toluene (20ml) and the product ($\nu_{max}$ 1803, 1725 and 1684cm$^{-1}$) redissolved into dichloromethane (10ml). This solution was added dropwise to persilylated D-phenylglycine (0,.134g), prepared as for Example 7(a), dissolved into dichloromethane (10ml). After 1.5 hours the reaction was diluted with ethyl acetate (50ml) and stirred vigorously with dilute hydrochloric acid. The ethyl acetate was separated and washed with water (3 × 30ml) before being dried ($MgSO_4$) and evaporated to a glass. This glass was taken up into ether and precipitated with n-hexane to give a buff coloured solid (0.291g; 85%); m.p. 104°C dec; $\nu_{max}$ (KBr) 3299, 3064, 1716 and 1680cm$^{-1}$.

(h) Sodium [6R,7R] 3-(1,3,4-Thiadiazol-2-yl)thio methyl-7[[R]-2[2,3-dioxo-4(3,4-dihydroxybenzylideneamino)piperazin-1-yl-carbonylamino]-2-phenylacetamido]-7-formamido-ceph-3-em-4-carboxylate

[6R,7R] 3-(1,3,4-Thiadiazol-2-yl)thiomethyl-7-amino-7-formamido-ceph-3-em-4-carboxylic acid (0.2g) was suspended in dichloromethane (10ml) and treated with N,O-bistrimethylsilylacetamide (0.16ml) and trimethylsilylchloride (2 drops). After stirring under argon at 40°C for 0.75 hours the reaction was cooled and evaporated from toluene.

Meanwhile, the product from part (g) (0.23g) was suspended in dichloromethane (10ml) and treated with trimethylsilylchloride (0.55ml) and hexamethyldisilazane (0.57ml). The reaction was gently refluxed for 1 hour and then evaporated from toluene. The residue was taken up into dichloromethane (10ml) and treated with trichloromethyl chloroformate (0.065ml). After 1.5 hours the reaction was evaporated from toluene and redissolved into dichloromethane (10ml). This was added in 1ml aliquots to the persilyalated cephem nucleus, prepared above, in dichloromethane (20ml). After 1 hour the reaction was evaporated, taken up into ethyl acetate (30ml) and washed vigorously with dilute hydrochloric acid. After final washings with distilled water the solvent was evaporated to give a yellow/buff coloured solid. This residue was dissolved into tetrahydrofuran and ethyl acetate (4:1, 25ml). The pH was adjusted to 7.4 with sodium hydrogen carbonate solution prior to dilution with distilled water (50ml) and ethyl acetate (50ml). The aqueous portion was concentrated and chromatographed upon HP20SS eluting with water graduating to 10% acetone in water. After combining and freeze drying fractions containing the product the title compound was obtained as a yellow/green solid (40.3mg; 9.3%); $\nu_{max}$ (KBr) 3302, 1771, 1711 sh, 1684 and 1602cm$^{-1}$; $\delta$ ($D_2O$) (major rotamer) 2.87 and 3.26 (2H, ABq, J 17Hz), 3.72-4.4 (6H, m), 5.12 (1H, s), 5.43 (1H, s), 6.80 (1H, d, J 8.1Hz), 7.08 (1H, d, J 8.1Hz), 7.22-7.60 (6H, m), 7.99 (1H, s), 8.08 (1H, s), and 9.30 (1H, s).

Example 23

Minimum Inhibitory Concentration (MIC) values of compounds of the invention against a variety of organisms were determined by serial dilution in Iso-sensitest agar (from Oxoid Ltd. Basingstoke, England). The plates were inoculated with $10^4$ colony-forming units and incubated overnight at 37°C. The MIC values recorded in Table I were the lowest concentration of antibiotic to inhibit growth. Comparative data for Piperacillin (6β-[D-2-[(4-ethyl-2, 3-dioxopiperazin-1-yl)carbonylamino]-2-phenylacetamido] -penicillanic acid, sodium salt) and for the compound 6α-formamido-6β-[D-2-[(4-ethyl-2, 3-dioxopiperazin-1-yl)carbonylamino]-2- phenyl acetamido]-penicillanic acid, sodium salt (Compound A) disclosed in U.K. Patent GB-2107307B, are also given.

## Table 1

## MIC data

| Organism | MIC | | | ($\mu$g/ml) | | |
|---|---|---|---|---|---|---|
| | Example 1 | Example 2 | Example 3 | Example 6 | Piperacillin | Compound A |
| *E.Coli* JT 425 | 1.0 | <0.03 | 16 | 0.25 | 32 | 0.25 |
| *E.Coli* DC RTEM | 4.0 | <0.03 | 2.0 | 0.06 | >128 | 0.12 |
| *K.pneumoniae* T767 | 0.5 | <0.03 | 0.25 | 0.06 | 8.0 | 0.12 |
| *P.aerginosa* 10662 | 0.12 | 0.12 | 4.0 | 1.0 | 8.0 | 1.0 |
| *P.aeruginosa* K79961 | <0.06 | 0.12 | 1.0 | 0.3 | 0.25 | 0.12 |
| *S agalactiae* 2798 | 0.25 | 16 | >128 | >32 | 0.5 | 64 |

## Claims

1. A compound of formula (I) or a pharmaceutically acceptable salt or in vivo hydrolysable ester thereof:

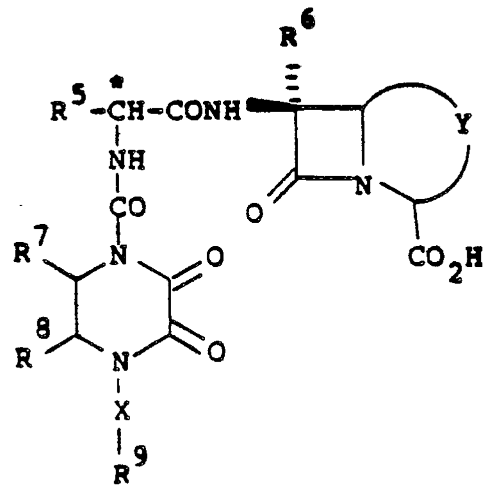

(I)

wherein Y is

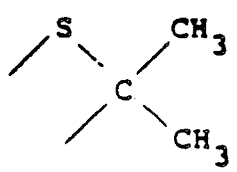

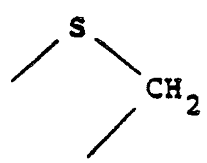

or

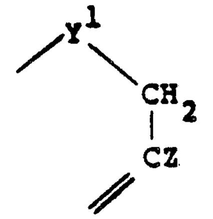

in which $Y^1$ is oxygen, sulphur or $-CH_2-$ and Z represents hydrogen, halogen, $C_{1-4}$ alkoxy, $-CH_2-Q$ or $-CH=CH-Q$ where Q represents hydrogen, halogen, hydroxy, mercapto, cyano, carboxy, carboxylic ester, $C_{1-4}$ alkyloxy, acyloxy, aryl, a heterocyclyl group bonded via carbon, a heterocyclylthio group or a nitrogen containing heterocyclic group bonded via nitrogen;
$R^5$ represents phenyl, substituted phenyl, cyclohexenyl, cyclohexadienyl or a 5 or 6-membered heterocyclic ring containing up to three hetero atoms selected from oxygen, sulphur or nitrogen optionally substituted with hydroxy, amino, substituted amino, halogen or $C_{1-6}$ alkoxy, $R^6$ is hydrogen, hydroxymethyl, formamido, or methoxy, $R^7$ and $R^8$ are the same or different and represent hydrogen, $C_{1-6}$ alkyl, substituted $C_{1-6}$ alkyl, halogen, amino, phenyl, substituted phenyl, hydroxy or $C_{1-6}$ alkoxy or $R^7$ and $R^8$ forn the residue of a 5 or 6-membered carbocyclic ring or a 5 or 6-membered heterocyclic ring containing up to three hetero atoms selected from oxygen, sulphur or nitrogen optionally substituted with hydroxy, amino, substituted amino, halogen or $C_{1-6}$ alkoxy; $R^9$ is

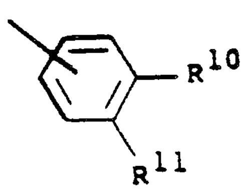

or

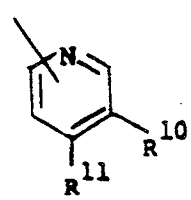

wherein $R^{10}$ and $R^{11}$ are the same or different and each represents hydroxy, or protected hydroxy and $XR^9$ is $-(CH_2)_nR^9$, $-NHCOR^9$, $-N=CHR^9$, $-NHCH_2R^9$, or $-COR^9$, where n is from 0 to 2; with the proviso that $XR^9$ does not represent $-N=CHR^9$ when $R^6$ represents hydrogen.

2. A compound as claimed in Claim 1 in which the group $R^5$ is phenyl, 4-hydroxyphenyl or 2-thienyl.

3. A compound as claimed in any one of the preceding claims in which Y is a group

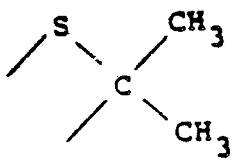

or

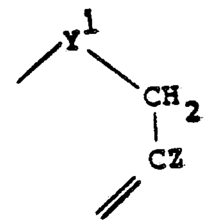

wherein $Y^1$ is sulphur.

4. A compound as claimed in any one of the preceding claims in which $R^7$ and $R^8$ are hydrogen.

5. A compound as claimed in any one of the preceding claims in which X is a methylene group.

6. A compound as claimed in any one of the preceding claims in which $R^9$ is a group:

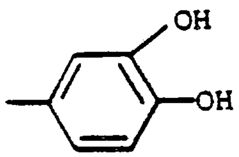

,

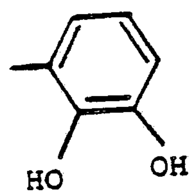

or

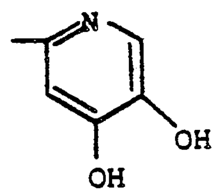

in which one or both hydroxy groups are optionally protected.

7. A compound as claimed in any one of the preceding claims in which $R^6$ is formamido.

8. A compound as claimed in any one of the preceding claims selected from the following or a pharmaceutically acceptable salt or pharmaceutically acceptable in vivo hydrolysable ester thereof:

6β-[D-2[[4-(3,4-Dihydroxybenzyl)-2,3-dioxopiperazin-1-yl]carbonylamino]-2-phenylacetamido]penicillanic acid;

6β-[D-2[[4-(3,4-Dihydroxybenzyl)-2,3-dioxopiperazin-1-yl]carbonylamino]-2-phenylacetamido]-6α-formamidopenicillanic acid;

6β-[D-2-[[4-(3,4-Dihydroxybenzyl)-2,3-dioxopiperazin-1-yl]carbonylamino]-2-phenylacetamido]-6α-methoxypenicillanic acid;

6β-[D-2-[[4-(3,4-Dihydroxybenzyl)-2,3-dioxopiperazin-1-yl]carbonylamino]-2-(4-hydroxyphenyl)acetamido]-6α-formamidopenicillanic acid;

6β-[D-2-(3,4-Diacetoxyphenyl-2-[[4-(3,4-dihydroxybenzyl)-2,3-dioxopiperazin-1-yl]carbonylamino] acetamido]-6α-formamidopenicillanic acid;

6β-[D-2-[[4-(4,5-Dihydroxypyridin-2-ylmethyl)2,3-dioxopiperazin-1-yl]carbonylamino]-2-phenylacetamido]-6α-formamidopenicillanic acid;

6β-[D-2-[[4-(3,4-dihydroxybenzyl)-2,3-dioxopiperazin-1-yl]carbonylamino]-2-phenylacetamido]-6α-(hydroxymethyl) penicillanic acid;

6β-[D-2-[[4-(3,4-Dihydroxybenzyl)-2,3-dioxopiperazin-1-yl]carbonylamino]-2-[4-aminophenyl)acetamido]-6α-formamidopenicillanic acid;

7β-[D-2-[[4-(3,4-Diacetoxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;

7β-[D-2-[[4-(3,4-Diacetoxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylic acid;

7β-[D-2-[[4-(3,4-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;

6β-[D-2-(3,4-Diacetoxyphenyl)-2-[(4-(3,4-dihydroxybenzyl)-2,3-dioxopiperazin-1-yl)carbonylamino] acetamido]-6α-(hydroxymethyl)penicillanic acid;

7β-[2-[[4-(3,4-Dihydroxybenzyl)-2,3-dioxopiperazin-1-yl]carbonylamino]-2-(2-thienyl)acetamido]7α-formamido-3-[(1,3,4-thiadiazol-2-yl)thiomethyl]ceph-3-em-4-carboxylic acid;

7β-[D-2-[[4-(3,4-Dihydroxybenzyl)-2,3-dioxopiperazin-1-yl]carbonylamino]-2-phenylacetamido]-7α-formamido-3-[(1-methyl-1H-tetrazol-5-yl)thiomethyl]ceph-3-em-4-carboxylic acid;

6β-[2-[[4-(2,3-dihydroxybenzyl)-2,3-dioxopiperazin-1-yl]carbonylamino]-2-phenylacetamido]-6α-formamidopenicillanic acid;

7β-[D-2-[[4-(2,3-Dihydroxybenzyl)-2,3-dioxopiperazin-1-yl]carbonylamino]-2-phenylacetamido]-7α-formamido-3-[(1,3,4-thiadiazol-2-yl)thiomethyl]ceph-3-em-4-carboxylic acid;
6β-[D-2-[4-(3,4-Dihydroxyphenyl)-2,3-dioxopiperazin-1-ylcarbonylamino]-2-phenylacetamido]-6α-formamidopenicillanic acid;
6β-[D-2-[4-[2-(3,4-Dihydroxophenyl)ethyl]-2,3-dioxopiperazin-1-ylcarbonylamino]-2-phenylacetamido]-6α-formamidopenicillanic acid;
7β-[DL-2-[4-(3,4-Dihydroxyphenyl)-2,3-dioxopiperazin-1-ylcarbonylamino]-2-phenylacetamido]-7α-formamido-3-[(1,3,4-thiadiazol-2-yl)thiomethyl]ceph-3-em-4-carboxylic acid;
3-Acetoxymethyl-7β-[D-2-[4-(3,4-dihydroxybenzyl)2,3-dioxopiperazin-1-ylcarbonylamino]-2-phenylacetamido]ceph-3-em-4-carboxylic acid; and
3-Acetoxymethyl-7β-[D-2-[4-(1,4-dihydro-5-hydroxy-4-oxopyridin-2-yl)methyl-2,3-dioxopiperazin-1-ylcarbonylamino]-2-phenylacetamido]ceph-3-em-4-carboxylic acid.

9. A pharmaceutical composition comprising an antibacterially effective amount of a pharmaceutically acceptable compound as claimed in any one of Claims 1 to 8 together with a pharmaceutically acceptable carrier or excipient.

10. A pharmaceutical composition as claimed in Claim 9 which further comprises a β-lactamase inhibitor.

11. The use of a compound of formula (I) as defined in Claim 1 for the manufacture of a medicament for the treatment of bacterial infections in humans and animals.

European Patent Office

# EUROPEAN SEARCH REPORT

Application Number

EP 87 30 9151

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 092 722 (BAYER)<br>* Pages 22,24,25; claims * | 1-4,9-11 | C 07 D 499/68<br>C 07 D 501/36<br>A 61 K 31/43<br>A 61 K 31/545//<br>C 07 D 241/08<br>C 07 D 401/06 |
| Y | EP-A-0 003 992 (BAYER)<br>* Claims * | 1,9-11 | |
| Y | EP-A-0 000 392 (BAYER)<br>* Claims * | 1,9-11 | |
| A | US-A-4 410 522 (I. SAIKAWA) | | |
| A | US-A-4 189 482 (U.D. TRUENER) | | |
| A | CHEMICAL ABSTRACTS, vol. 98, no. 9, 28th February 1983, page 602, abstract no. 71807c, Columbus, Ohio, US; & JP-A-57 118 587 (TOYAMA CHEMICAL CO., LTD) 23-07-1982 | | |
| | | | **TECHNICAL FIELDS SEARCHED (Int. Cl.4)** |
| | | | C 07 D 499/00<br>C 07 D 501/00<br>C 07 D 498/00<br>C 07 D 471/00<br>A 61 K 31/00 |

The present search report has been drawn up for all claims

| Place of search | Date of completion of the search | Examiner |
|---|---|---|
| THE HAGUE | 01-09-1988 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)